# FASCICULE DE BREVET EUROPEEN

(11) **EP 0 117 777 B1**
(45) Date de publication et mention de la délivrance du brevet: **23.12.1987**
(21) Numéro de dépôt: 84400143.8
(22) Date de dépôt: 23.01.1984
(51) Int. Cl.: C07H 21/00, G01N 33/53, G01N 33/58

(54) **Nouveaux composés comportant une séquence d'oligonucléotide liée à un agent d'intercalation, leur procédé de synthèse et leur application**
Verbindungen,welche eine oligonucleotide Sequenz enthalten, die an einen Interkalationsstoff gebunden ist, Verfahren zu deren Synthese und deren Verwendung
Compounds containing an oligonucleotide bonded to an intercallating agent, a process for their preparation and their application

(30) Priorité: 27.01.1983 FR 8301223
(43) Date de publication de la demande: 05.09.1984
(73) Titulaire: Etablissement Public dit: CENTRE NATIONAL DE LA RECHERCHE SCIENTIFIQUE (CNRS), 75007 Paris (FR)
(72) Inventeur: Nguyen, Thanh Thuong, F-45510 Tigy (FR); Helene, Claude, F-45590 Saint Cyr en Val (FR); Asseline, Ulysse, F-45100 La Source (FR)
(74) Mandataire: Warcoin, Jacques

## Description

La présente invention concerne des composés chimiques nouveaux ainsi que leur application notamment à titre de sonde permettant la détection d'une séquence définie d'ADN ou'd'ARN.

Il a déjà été proposé d'insérer des dérivés du psoralène dans la double hélice des acides nucléiques puis de les fixer par liaison covalente à l'aide d'une radiation électromagnétique dans le brevet US-A-4196281.

Les composés chimiques selon la présente invention sont constitués par un oligonucléotide ou un oligodésoxynucléotide comportant un enchaînement de nucléotides naturels ou modifiés sur lequel se trouve fixé par une liaison covalente au moins un groupe intercalant.

Il s'agit de composés de formule : 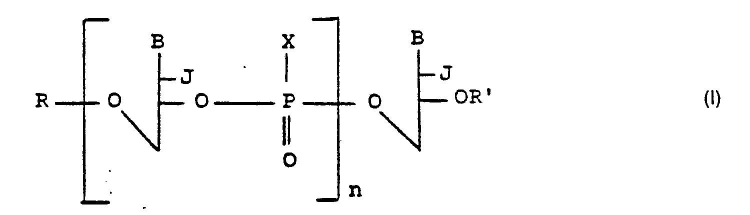 dans laquelle les radicaux B peuvent être identiques ou différents et représentent chacun une base d'un acide nucléique naturelle ou modifiée ;

Les radicaux X, qui peuvent être identiques ou différents, représentent chacun un oxoanion 0⁶, un thioanion S⁸, un groupe alkyle, un groupe alcoxy, aryloxy, un groupe aminoalkyle, un groupe aminoalcoxy, un groupe thioalkyle ou un groupement -Y-Z ;
R et R', qui peuvent être identiques ou différents, représentent chacun un atome d'hydrogène ou un groupement ―Y―Z ;
Y représente un radical alcoylène droit ou ramifié -alk- ou un radical 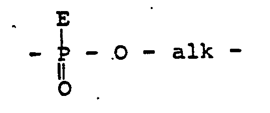 ou bien un radical ―Y"―O―Y'― où Y" ou Y' peuvent avoir les significations données pour Y ;
E peut avoir les mêmes significations que X ;
J représente un atome d'hydrogène ou un groupe hydroxy ;
Z est un radical correspondant à un agent d'intercalation et l'un au moins des groupes X, R, R' ou E est un groupement ―Y―Z ;
n est un nombre entier y compris 0.

Il convient de remarquer que la formule I représente un enchaînement de nucléotides qui peuvent être identiques ou différents, n indiquant simplement le nombre de nucléotides compris dans la molécule ; n est de préférence un nombre compris entre 1 et 50 et de préférence de 1 à 25.

Les agents d'intercalation Z sont des composés connus dans les techniques touchant aux acides nucléiques, il s'agit de composés capables de « s'intercaler dans la structure des ADN ou des ARN.

Ces agents d'intercalation sont, en général, constitués par des composés polycycliques ayant une configuration plane tels que l'acridine, la furocoumarine ou l'ellipticine et leurs dérivés par exemple.

Parmi les significations de Z, deux seront utilisées plus particulièrement :

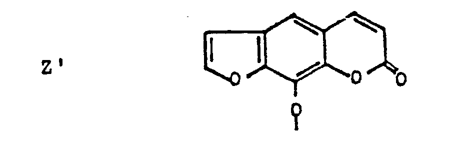

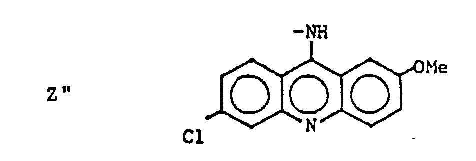

Le radical B est, de préférence, constitué par une base naturelle d'un acide nucléique, par exemple la thymine, l'adénine, la cytosine, la guanine ou l'uracile ; mais il est également possible d'utiliser des bases d'acides nucléiques modifiées, notamment halogénées ou azidées. par exemple la 5-bromo-uracile ou la 8-azido-adénine. En outre, il est possible d'utiliser des dérivés photoactivables des bases dont on verra ci-après les applications.

Le radical X, bien qu'il représente de préférence un oxoanion, peut avoir d'autres significations ; lorsque le radical X représente un groupement alkyle, il s'agit de préférence d'un groupement alkyle inférieur en Ci à C₇ et, par exemple, les groupements éthyle, méthyle ou propyle ; lorsque le radical X représente un groupe alcoxy, il s'agit de préférence d'un groupement alcoxy inférieur en Ci à C₇, par exemple le groupement méthoxy ou éthoxy ; lorsque X représente un groupement amino-alkyle ou amino-alcoxy, il peut s'agir d'un groupement amino-alkyle monosubstitué, disubstitué ou bien d'un radical amino sous forme de sel d'ammonium quaternaire, dans ces conditions les substituants sont de préférence des radicaux alkyles inférieurs comme définis précédemment ; quant à la chaîne alkyle ou alcoxy reliant le radical amino au phosphore, il s'agit de préférence d'une chaîne droite ou ramifiée comportant de 1 à 10 atomes de carbone ; enfin, lorsque le radical X est un radical thioalkyle, il s'agit de préférence d'un radical thioalkyle inférieur, c'est-à-dire qui comporte entre 1 et 7 atomes de carbone.

R et R', outre la signification hydrogène, peuvent représenter notamment :

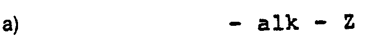

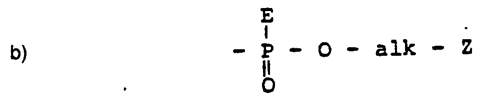

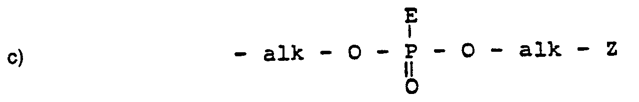

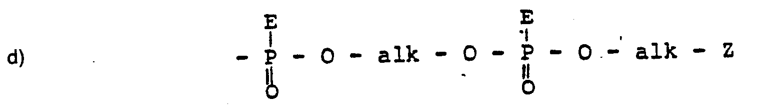

Le radical -alk- est de préférence un radical alcoylène droit ou ramifié ayant de 1 à 10 atomes de carbone.

La présente invention concerne également les composés précédents sous forme de sel avec des bases ou des acides, et les composés sous forme racémique, ou sous forme d'isomères R ou S optiques purifiés ou en mélange.

Parmi les composés plus particulièrement intéressants, on peut citer, par exemple, les composés de formule : 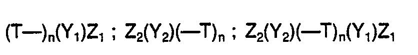 où T est le radical thymidine, n est un nombre entier de 1 à 25 et Y₁, et Y₂ représentent un radical alcoylène en C₁ à C₇ ou un radical : 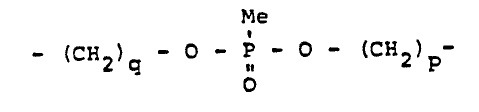 dans laquelle p et q sont des nombres entiers de 1 à 10 et Z₁ et Z₂ ont les significations de Z, et de formule : 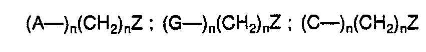 et les composés séquencés tels que les oligonucléotides :

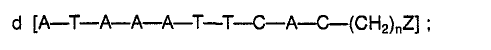

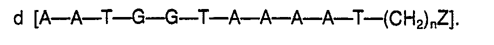

Ces composés peuvent être préparés par des procédés qui sont déjà connus, en particulier par le procédé de synthèse dit « au phosphotriester » ; dans ce procédé on prépare tout d'abord la chaîne de nucléotides, les différents groupements non mis en oeuvre étant protégés, puis on élimine enfin les groupements protecteurs pour obtenir les produits recherchés.

Dans un premier mode de réalisation, les composés selon la présente invention comportant un groupement d'intercalation en 3', par exemple les composés de formule 1a, peuvent être préparés par couplage du nucléotide de 3'-phosphodiester de formule 2 avec le dérivé hydroxylé de l'agent intercalant de formule 3 ou avec le dérivé hydroxylé en 5' d'un oligonucléotide possédant déjà l'agent d'intercalation en 3' tel que 4 (schéma 1).

Les composés 1 b possédant les groupes d'intercalation en 5' peuvent être obtenus, soit par couplage du dérivé hydroxylé de l'agent d'intercalation 3 avec un nucléotide 5'-phosphodiester tel que 5, soit par condensation d'un diester de l'agent d'intercalation 6 avec l'hydroxyle-5' du nucléotide tel que 7 (schéma 2).

Afin d'obtenir des composés possédant des groupes d'intercalation à la fois en 3' et en 5' tels que 1c. il est possible, par exemple, de coupler un diester tel que 6 avec un triester de formule 4 ou à partir d'un nucléotide 3',5'-bis(phosphodiester) tel que 8, avec le dérivé hydroxylé d'agent d'intercalation 3 (schéma 3).

Enfin, les composés possédant un agent intercalant relié à un phosphate internucléotidique tel que 1d peuvent être préparés, soit par couplage du dérivé hydroxylé de l'agent intercalant avec le groupe phosphodiester internucléotidique 9, soit par couplage d'un diester 2 avec le dérivé hydroxyle-5' d'un oligonucléotide 10 possédant déjà l'agent d'intercalation (schéma 4).

La préparation des dimères tels que 22 et 23 et des oligomères intermédiaires peut être réalisée selon l'un des schémas ci-annexés, 5, 6 et 7 ; il s'agit là de techniques déjà connues dans la synthèse dite au phosphotriester.

Selon ces schémas, on peut, à partir de différents nucléosides convenablement protégés de formule 11, accéder aux différents monomères 16 et 17, puis aux oligomères de séquence déterminée.

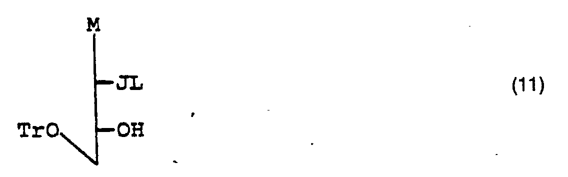

Dans la formule 11, M représente l'une des bases des acides nucléiques naturelles ou modifiées dont la fonction NH₂ est protégée par un groupe acyle ; JL représente un atome d'hydrogène ou un groupement trialkylsilyloxy ou autre groupement de protection ; Tr représente un groupement trityle ou un groupement monométhoxytrityle ou un groupement diméthoxytrityle.

Les composés dans lesquels X est un radical de type éthérifié peuvent être préparés à partir des nucléosides convenablement protégés tels que 11 en utilisant des réactifs de phosphorylation tels que 12, 13 et 14 :

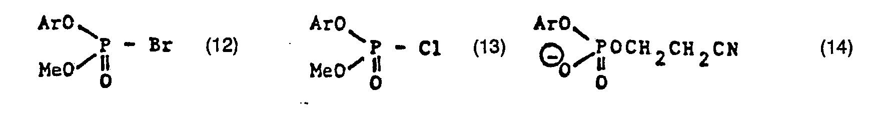

Les conditions réactionnelles de ces différentes réactions sont connues et sont rappelées en abrégé sur les schémas ci-annexés.

Les dinucléosides-monophosphates tels que 20 et 21 dont le phosphate porte trois substituants différents peuvent être obtenus sous forme de diastéréoisomères (R ou S), soit sous forme d'isomère 20R ou 20S et 21 R ou 21 S.

L'obtention des isomères 21 R ou 21 S peut être réalisée, soit en séparant les deux composants du diastéréoisomère, soit en traitant chacun des isomères 20R et 20S par un alcoolate.

La phosphorylation du diastéréoisomère 21 R, S et des isomères 21 R et 21 S par les réactifs 13 et 14 conduit respectivement aux dimères 22 et 23 dont le phosphate porteur du groupement X = OR"' peut être sous forme de diastéréoisomère (R, S) ou sous l'une des deux formes d'isomères R ou S.

Les dimères 22 et 23 dérivés d'acide phosphonique (X = R"') peuvent être obtenus par l'intermédiaire du métaphosphonate 24 selon le schéma 6.

A partir des dimères 22 et 23 on peut accéder aux oligomères 2, 4, 5. 7 et 8 (schéma 7).

La préparation d'oligomère renfermant un groupe phosphodiester internucléotique 9 est donnée par le schéma 8.

Enfin, le passage des différents oiigomères totalement protégés aux composés de formule 1 se fait par élimination sélective des différents groupements protecteurs. La déprotection des phosphates internucléotidiques (déarylation) a été réalisée en milieu anhydre à l'aide de l'ion benzohydroxamate. Selon ce nouveau procédé, on peut débloquer les phosphoesters arylés tout en respectant à la fois les phosphoesters alkylés et les groupements intercalants utilisés.

Enfin, les composés selon l'invention peuvent être préparés par des méthodes semi-synthétiques en particulier en utilisant des fragments d'ADN ou d'ARN préparés par des méthodes de génie génétique sur lesquels sont ensuite fixés les agents intercalants.

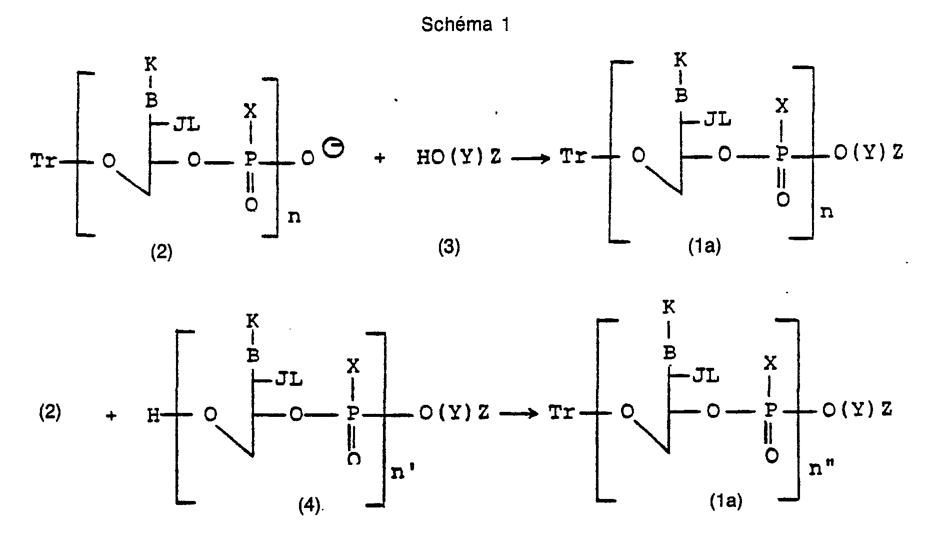

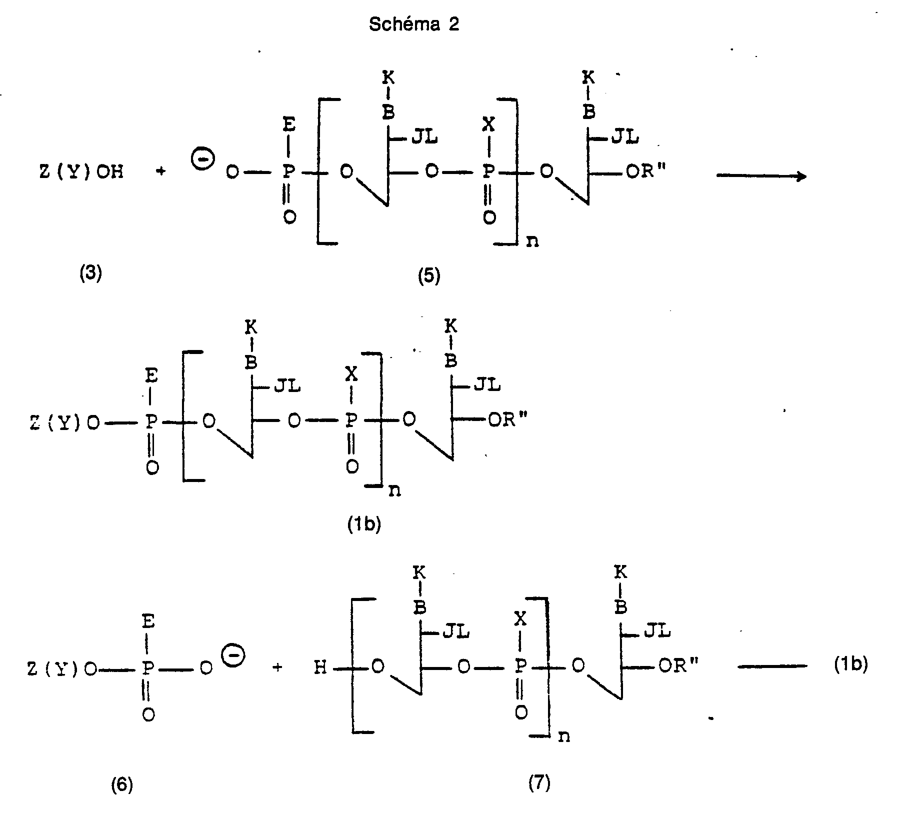

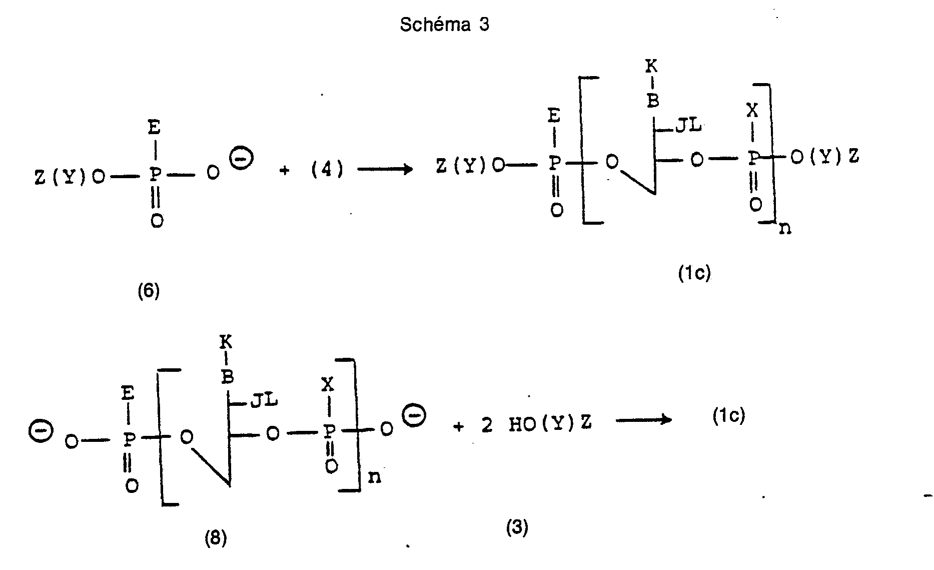

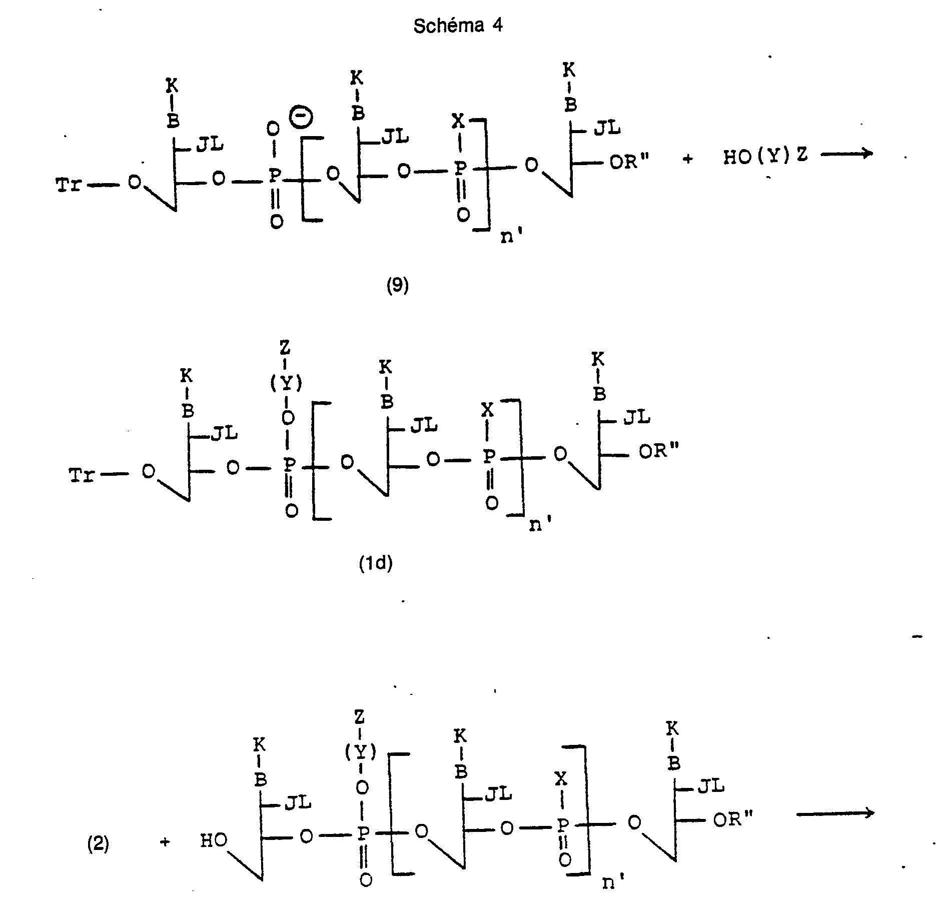

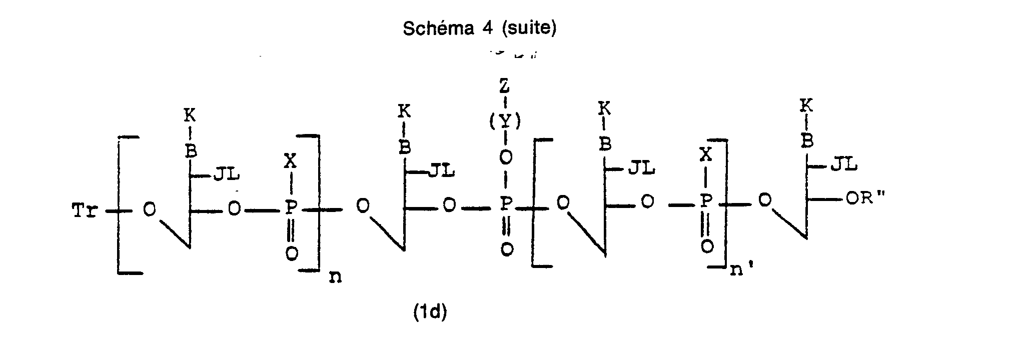

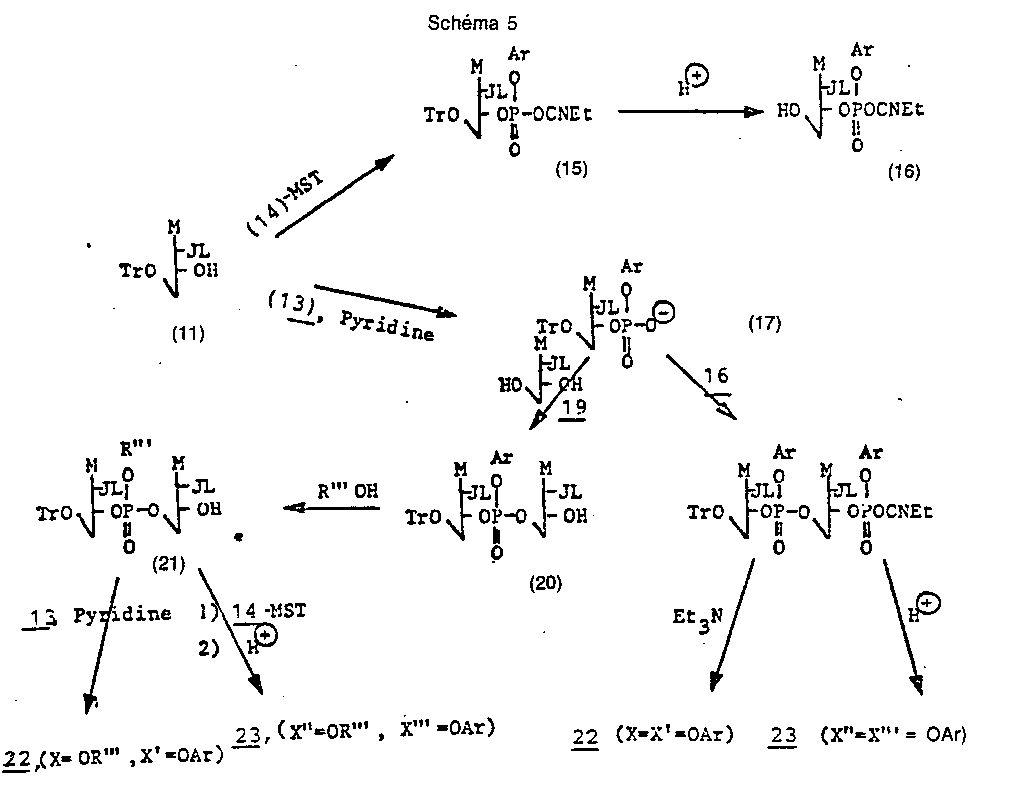

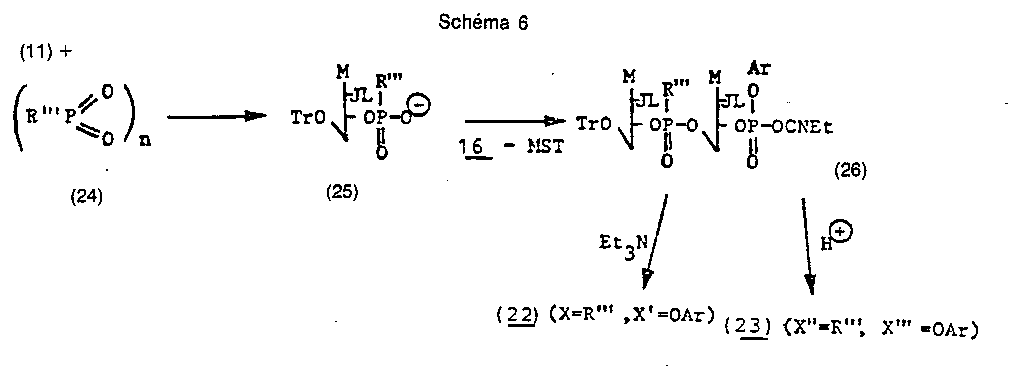

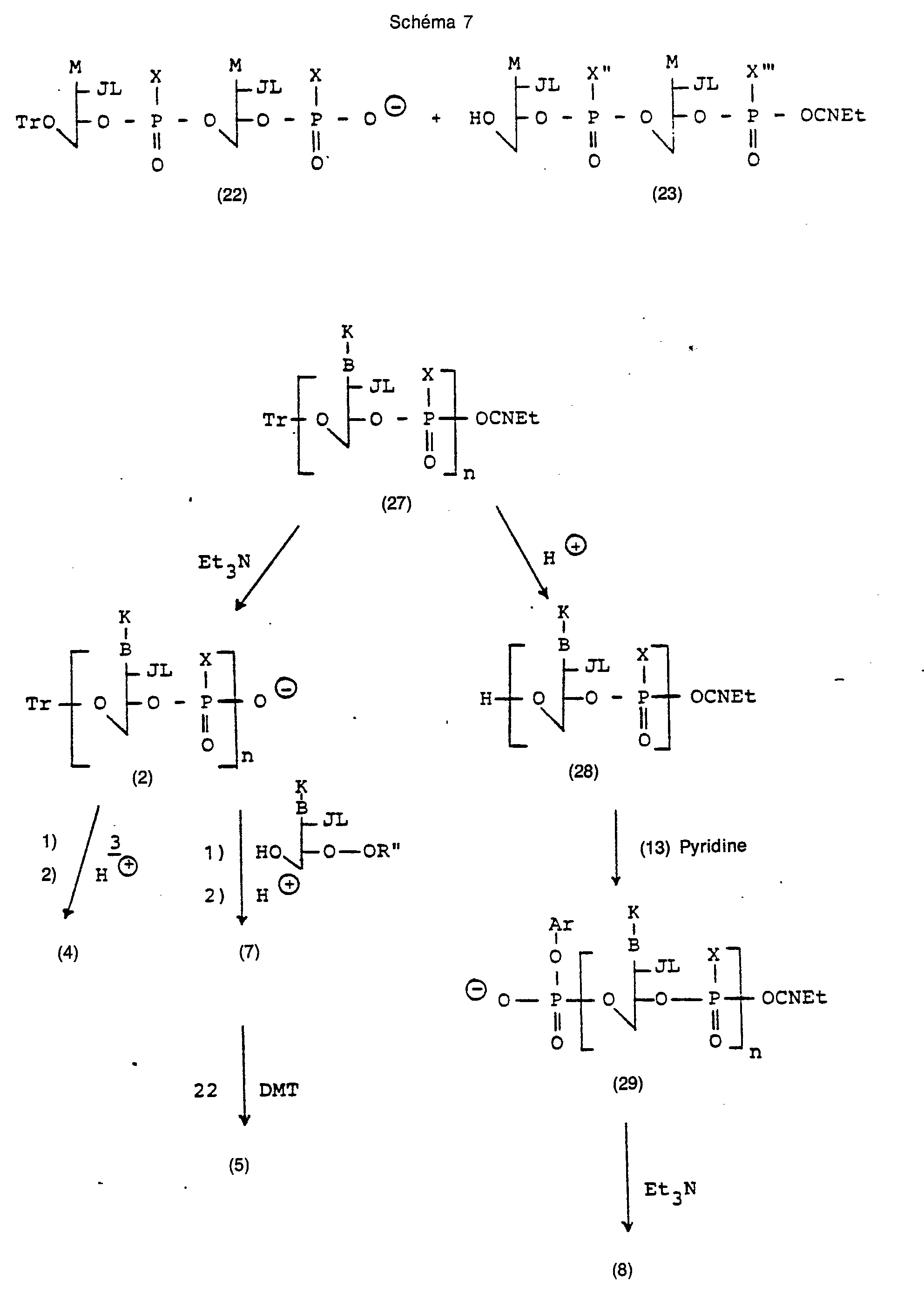

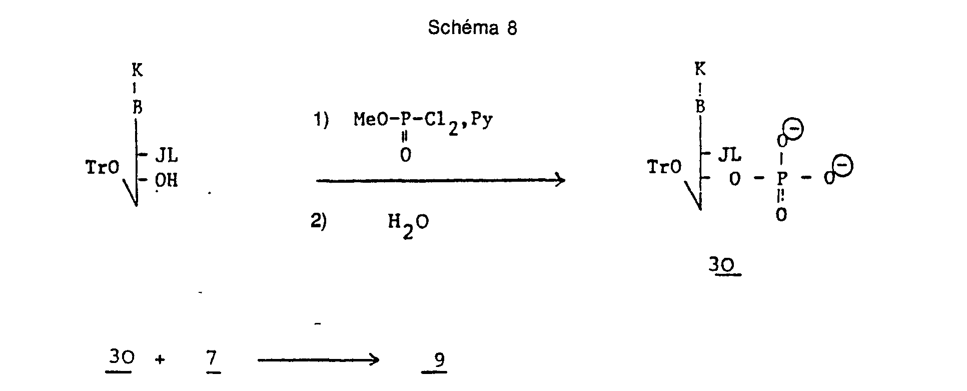

Les composés selon la présente invention constitués d'une chaîne d'oligonucléotides et d'un agent intercalant se lient de façon sélective à toute séquence complémentaire d'oligonucléotides. La présence d'un agent intercalant dans les composés de l'invention permet d'augmenter l'affinité du composé pour l'oligonucléotide complémentaire par rapport à l'oligonucléotide seul.

La présence d'agents intercalants. dotés de propriétés distinctes des nucléotides permet leur détection et par là même la détection de la présence d'une chaîne de nucléotides complémentaire de la chaîne dont ils font partie.

Les composés selon la présente invention sont donc utilisables à titre de sonde d'hybridation mais également comme composant de purification pour des séquences déterminées d'ADN ou d'ARN.

La présence d'agents intercalants permet donc la détection in situ des composés fixés, par exemple par fluorescence avant le pontage photochimique pour les dérivés de l'acridine ou après pontage photochimique dans le cas des dérivés de furocoumarine.

Ceci permet d'utiliser les composés selon la présente invention pour détecter, par hybridation sur gel par exemple, une séquence donnée dans un fragment d'ADN ou d'ARN.

Les composés peuvent être également utilisés pour détecter des mutations au niveau d'un ADN ou d'un ARN. Si une base (G par exemple) dans un gène normal a été remplacé par une autre base (T par exemple) dans le gène ayant subi une mutation, il est possible de construire un oligodésoxynucléotide se terminant du côté 3' par A, l'intercalant étant attaché au groupement phosphate en 3'. L'appariement sera complet avec le gène muté alors que la dernière paire de bases ne pourra pas se former avec le gène normal. Cela entraînera une perturbation de l'interaction de l'intercalant qui amplifiera, du point de vue de la stabilité du complexe formé, l'absence d'appariement. Le même effet peut être obtenu en choisissant la base mis-appariée du côté 5' de l'oligodésoxynucléotide, le groupement intercalant étant lié au groupe phosphate en 5'.

Par exemple les deux oligodésoxynucléotides (Tp)ₑ(CH₂)₅Z" et (Tp)₇Ap(CH₂)gZ' se fixent sur le poly(rA). Les températures de demi-dissociation des complexes formés sont respectivement de 41 et 27 °C. Le premier forme 8 paires de bases A.T., le deuxième en forme 7 mais la perturbation de l'intercalation due à l'absence de la dernière partie de base déstabilise considérablement le complexe. A titre de comparaison le composé (T_{P})₄(CH₂)₅Z" qui ne peut former que 4 paires de bases mais qui donne lieu. à une intercalation analogue à celle de (Tp)g(CH₂)₅Z" a une température de demi-dissociation de 31 °C.

Il est également possible de détecter, grâce aux composés de l'invention, des séquences accessibles dans un complexe protéine-acide nucléique tel que ceux que l'on trouve dans les ribosomes par exemple.

Les composés selon la présente invention permettent de purifier un fragment d'ADN ou d'ARN contenant une séquence donnée par chromatographie d'affinité.

Au niveau cellulaire, les composés selon la présente invention permettent de séparer des cellules possédant une séquence donnée (telles que des virus) par cytofluorimétrie de flux ou bien peuvent permettre de diagnostiquer la présence d'une séquence donnée d'ADN ou d'ARN (d'un virus par exemple) dans une cellule (cytopathologie).

De façon générale, les composés selon la présente invention peuvent être utilisés comme des sondes fluorescentes et remplacer les sondes radioactives utilisées jusqu'à maintenant. Il est évident qu'un tel type de sonde présente énormément d'avantages par rapport aux sondes radioactives. En particulier comme cela a été dit, l'oligonucléotide présente une affinité plus grande pour sa séquence complémentaire grâce à la présence de l'intercalant ; en outre, l'agent fluorescent peut être détecté beaucoup plus rapidement que dans le cas des sondes radioactives et il présente l'avantage de pouvoir effectuer une résolution spatiale très aisée.

Il est assez facile avec ce type de sonde de mesurer quantitativement la fluorescence et donc d'effectuer des déterminations quantitatives.

Enfin, un avantage non négligeable des sondes selon la présente invention par rapport à celles de la technique antérieure est qu'il n'est pas nécessaire de manipuler de produits radioactifs.

L'association des composés selon la présente invention avec des acides nucléiques peut être suivie par différentes méthodes : absorption, fluorescence, dichroïsme circulaire, résonance magnétique nucléaire.

Des modifications spectrales importantes sont observées dans les bandes d'absorption de l'intercalant (hypochromisme important pouvant atteindre 50 %, déplacement du spectre vers les grandes longueurs d'ondes).

Lorsque l'oligonucléotide contient une séquence de thymine (T4, T8, T12), ces modifications spectrales ne s'observent que par fixation sur la séquence complémentaire (polyA). Aucune interaction n'est observée avec polyU et polyC.

Quand la séquence d'oligonucléotide contient l'adénine (A5), seul le polyU ou le polydT donne lieu à des modifications spectrales caractéristiques. Le polyA est sans effet.

Les complexes formés avec les composés selon la présente invention ont été étudiés afin de mettre en évidence l'influence des différents paramètres sur la stabilité.

La stabilité peut être mesurée par la température de demi-transition des complexes (T_{1/2}) lors d'une élévation de température. Le tableau ci-dessous compare ces températures pour les oligonucléotides substitués soit par un groupement éthyle, soit par un bras (CH₂)₅ lié à l'intercalant dérivé de l'acridine. Les valeurs de Tᵢ₁₂ ont été déterminées pour un rapport 1 : 1 (T : A) à la concentration de 5 x 10-⁵ M en intercalant et dans un tampon à pH 7 contenant 10-² M cacodylate de sodium et 0,1 M NaCi.

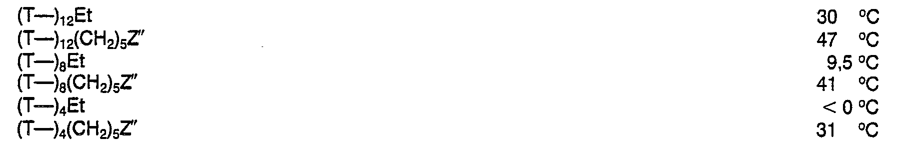

La stabilisation des complexes due à la présence de l'intercalant est évidente. La différence de température est d'autant plus grande que l'oligonucléotide est plus court.

La stabilisation dépend de la longueur du bras utilisé pour fixer l'intercalant sur l'oligonucléotide comme le montrent les T_{1/2} suivants :

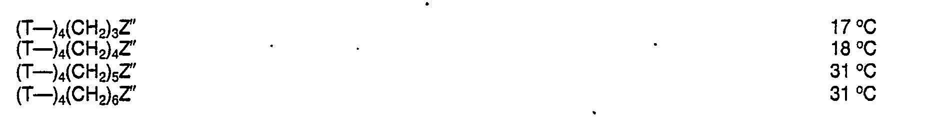

Un bras de Gₛ est donc suffisant pour assurer une bonne stabilisation du complexe.

La stabilité des complexes peut être également déterminée par l'enthalpie libre de réaction (kilocalories par mole) mesurée à partir de la variation du T_{1/2} avec la concentration en oligonucléotide.

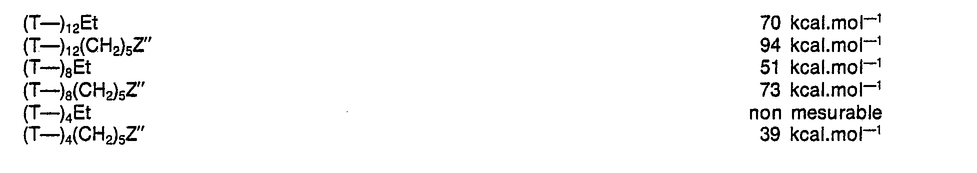

La présence de l'intercalant permet donc de gagner 20 à 30 kilocalories par mole d'oligonucléotide fixé.

Les exemples suivants sont destinés à illustrer d'autres caractéristiques et avantages de la présente invention mais ne la limitent évidemment nullement.

Dans la description, on utilise la représentation condensée des nucléotides suivante : 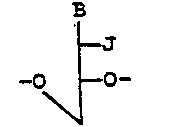 qui correspond à la formule développée : 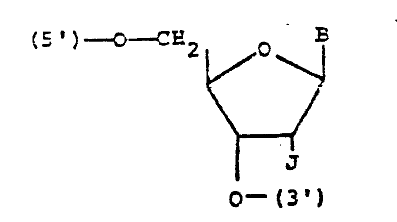 sur laquelle ont été mentionnées les extrémités (3') et (5').

Dans la présente description les abréviations ci-après sont utilisées :
ODN : oligodésoxyribonucléotide
Ar : parachlorophényle
CNet : (β-cyanoéthyle
Me : méthyle
Et : éthyle
MMTr : monométhoxytrityle
DMTr : diméthoxytrityle
Tr : trityle
MST mésithylènesulfonyltétrazolide
DMT : diméthyl-1;5-tétrazole
T : thymidine 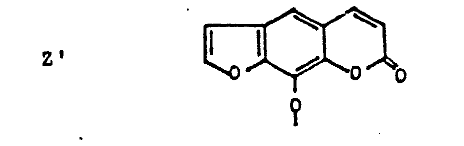 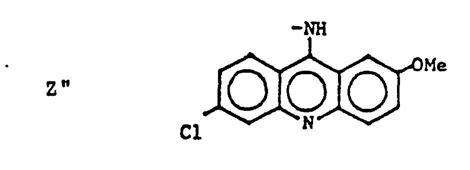
TEA : triéthylamine
CCM : chromatographie sur couche mince

Dans les formules (DMTr) T∓T∓T∓T∓Ar, la liaison phosphodiester est représentée par un trait et la liaison phosphotriester est représentée par le symbole (∓), chaque phosphate est protégé par le groupe p-chlorophényle (Ar).

### Exemple l

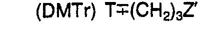

1) 5'-O(diméthoxytrityl)-thymidine 3'-(p-chlorophényl phosphate) de pyridinium
a) On ajoute sous agitation et à ―20 °C 3 équivalents de méthÿl p-chlorophényl-chlorophosphate (préparé par action du chlore sur le diméthyl p-chlorophénylphosphite) à 1 solution de 9 équivalents de diméthyl-1,5-tétrazole (DMTe) dans la pyridine anhydre ; l'addition terminée, on continue l'agitation pendant une heure à la température ambiante, puis ajoute à-10 °C une solution de 1 équivalent de 5'-O-(diméthoxytrityl)-thymidine (J. Amer. Chem. Soc., 1963, 85, 3821) dans la pyridine. Après quelques heures de réaction à la température ambiante, on ajoute ensuite de l'eau glacée et extrait le diester avec du chloroforme ; la solution chloroformique est ensuite séchée puis concentrée sous vide. le diester obtenu sous forme de solution dans la pyridine est pratiquement pur pour être utilisé dans les étapes suivantes. Le rendement de cette réaction est pratiquement quantitatif par rapport au nucléoside.
   CCM gel de silice ; isoPrOH, TEA, H₂0 (85 : 5 : 10, v/v), Rf - 0,5.
b) On obtient le même résultat en remplaçant le méthyl p-chlorophényl-chlorophosphate par le méthyl p=chlorophényl-bromophosphate (Téthrahedron Letters, 1980, 21, 2063).

2) On fait réagir sous agitation pendant 2 heures à la température ambiante une solution de 0,25 mmol de diester préparé précédemment, de 0.2 mmol de (w-hydroxy-propyloxy) 8-psoralène et de 0,4 mmol de mésitylènesulfonyltétrazole (MST) (Nucleic Acids Res., 1977, 4, 353) dans 1,5 ml de pyridine anhydre ; on ajoute ensuite 0,5 ml d'eau glacée puis continue l'agitation pendant 20 minutes à la même température. On concentre la solution sous vide et reprend le résidu avec du chloroforme ; on lave la phase organique avec 2 x 1 ml d'une solution aqueuse à 5 % de NaHC0₃ puis la sèche sur Na₂S0₄. Après avoir chassé le solvant sous vide, le produit obtenu est purifié sur une colonne de gel de silice (éluant : CH₂Cl₂―MeOH).
Le rendement de la préparation est de l'ordre de 80 % par rapport au dérivé du psoralène.
CCM : gel de silice 60F254 ; solvant : CH₂Cl₂, MeOH (9 : 1, v/v), Rf = 0,73.

### Exemples Il et III

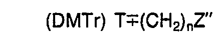

En opérant comme dans l'exemple l, en remplaçant le (w-hydroxypropyloxy)-8-psoralène par le méthoxy-2 chloro-6 (w-hydroxypropylamino)-9 acridine et par le méthoxy-2 chloro-6 (w-hydroxypentyla- mino)-9 acridine, on a préparé les composés suivants :

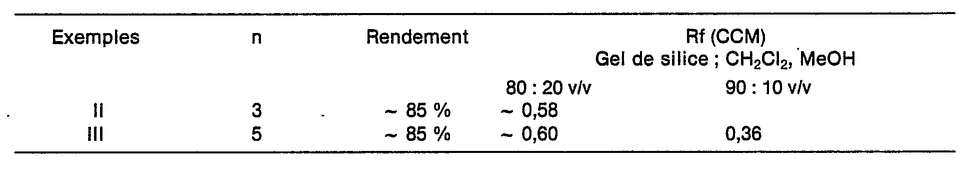

### Exemple IV

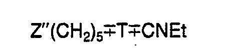

### 1)Ar―T∓CNEt

On opère comme dans l'exemple 1-1 en remplaçant le 5'-0(diméthoxytrityl)-thymidine par la thymidine-3'(p-chlorophényl (β-cyanoéthyl phosphate) (Biochimie, 1981, 63, 775) ; après avoir extrait le produit avec du chloroforme, on chasse le solvant et la pyridine sous vide, reprend le résidu avec de l'éther puis lave le solide blanc obtenu plusieurs fois avec le même solvant.
Rendement : 90-95 %
CCM : gel de silice ; CH₂CI₂, MeOH (85 : 25, v/v), Rf - 0,32.

### 2) Couplage

On fait réagir pendant 2 heures à la température ambiante un mélange de 1 équivalent de nucléoside diphosphate préparé précédemment (exemple IV-1) de 1,5 équivalent de méthoxy-2 chloro-6 (ω-hydroxy- pentylamino)-9 acridine de 2 équivalents de mésitylène sulfonyl chlorure et de 6 équivalents de tétrazole dans la pyridine. Après avoir détruit l'excès du réactif de couplage par addition de l'eau glacée, le produit est ensuite extrait avec du chloroforme puis purifié sur gel de silice en utilisant les mélanges CH₂CI₂, MeOH (99 : 1 à 90 10, v/v). On obtient le produit sous forme de solide jaune ; le rendement de la préparation est de 82 %.
CCM : gel de silice ; CH₂Cl₂, MeOH (9 : 1, v/v), Rf - 0,21.

### Exemple V

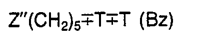

### 1) Z"(CH2)₅ ∓T―Ar

Le composé de l'exemple IV-2 est traité pendant quelques heures à la température ambiante avec une solution de EtₛN-pyridine (1 : 2 v/v) (1 ml) par mmole de triester (Nucleic Acids Res., 1976, 3, 3397). Après avoir chassé les solvants sous vide, le solide jaune obtenu est ensuite lavé avec de l'éther.
Rendement - 88 %
CCM : gel de silice ; iso-PrOH, Et₃N, H₂0 (85 : 5 : 10, v/v), Rf - 0,63.

2) On opère comme dans l'exemple IV-2 et en utilisant un équivalent de Z"(CH₂)₅∓T-Ar, 3 équivalents de 3'-benzoylthymidine et 10 équivalents de MST, on obtient le produit sous forme de solide jaune avec un rendement de 75 %.

CCM : gel de silice ; CH₂CI₂, MeOH (9 : 1, v/v), Rf - 0,25.

### Exemple VI

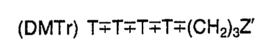

### 1) (DMTr) T∓T―Ar

0,3 mmol de dinucléotide (DMTr) dT∓T∓CNEt (Biochimie, 1981, 775) est traité pendant quelques heures à la température ambiante avec 5 ml d'une solution de pyridine-triéthylamine (2:1. v/v). On chasse le solvant sous vide puis lave le solide obtenu avec de l'éther. Rendement - 90 %.

### 2) (DMTr) T∓T∓T∓T∓CNEt

Le dinucléotide (DMTr) T∓T∓CNEt (0,2 mmol) est traité avec l'acide benzène sulfonique (2 %) en solution dans le chloroforme, méthanol (7 : 3, v/v) (3 ml) à 0 °C pendant 15 minutes (Nucleic Acids Res.. 1977, 4, 353). Le mélange est repris avec 40 ml de chloroforme et lavé avec une solution aqueuse de NaHC0₃ à 5 % puis séché sur Na₂S0₄ et concentré sous vide. A ce résidu on ajoute 0,25 mmol de dinucléotide préparé d'après VI-1 ; après avoir séché le mélange par évaporation avec la pyridine, on ajoute au résidu 3 ml de pyridine anhydre, puis 0.6 mmol de MST et laisse le mélange réactionnel à la température ambiante pendant 2 heures sous agitation. On détruit ensuite l'excès de réactif de couplage par addition de l'eau glacée puis termine la préparation comme dans l'exemple 1-2 en remplaçant le mélange CH₂Cl₂, MeOH par le système CH₂CI₂, eau, acétone (43: 2: 55, v/v). Le rendement de la préparation est de 75 %.
CCM : gel de silice ; CH₂CI₂, MeOH (9 : 1, v/v), Rf - 0,50.

### 3) (DMTr) T∓T∓T∓T-Ar

On opère comme dans l'exemple VI-1 en utilisant le tétranucléotide préparé selon VI-2, on obtient le produit sous forme de solide avec un rendement de l'ordre de 80 %.

### 4) (DMTr) T∓T∓T∓T∓(CH₂)₃Z'

On fait réagir pendant 2 heures à la température ambiante, en solution dans la pyridine anhydre, 1 équivalent de (DMTr) T∓T∓T∓T―Ar, 1,3 équivalent de (w-hydroxypropyloxy)-8-psoralène et 3 équivalents de MST puis termine la préparation comme dans l'exemple VI-2. Rendement = 72 %.
CCM : gel de silice ; CH₂Cl₂, MeOH (90 : 10, v/v), Rf - 0,37.

### Exemple VII

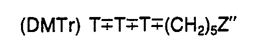

### 1) T∓(CH₂)₅Z"

Le ,composé (DMTr) T∓(CH₂)₅Z" de l'exemple III est traité par l'acide acétique à 80% pendant 1 heure 30 à la température ambiante ; après avoir chassé l'acide acétique par coévaporation avec l'éthanol, le produit est ensuite purifié sur gel de silice. Rendement : 86 %.
CCM : gel de silice ; CH₂C1₂, MeOH (90 : 10, v/v), Rf = 0,14.,

### 2) (DMTr) T∓T∓T∓(CH₂)₅Z"

On fait réagir pendant 2 heures sous agitation et à la température ambiante dans la pyridine anhydre un mélange de (DMTr) T∓T―Ar (1,2 équivalent) (exemple VI-1) de T∓(CH₂)₅Z" (1 équivalent) (préparé précédemment) de MST (2 équivalents) ; on termine ensuite la préparation comme dans l'exemple VI-2. Rendement = 78 %.
CCM : gel de silice ; CH₂Cl₂, MeOH (90 : 10, v/v), Rf = 0,41.

### Exemple VIII

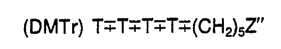

En opérant comme dans l'exemple VI-4, et en utilisant le méthoxy-2 chloro-6 (w-hydroxypentylamino)-9 acridine (1,5 équivalent) à la place du dérivé du psoralène, on obtient le produit cherché avec un rendement de 87 %.
CCM : gel de silice ; CH₂Cl₂, MeOH (90 : 10, v/v, Rf = 0.37.

### Exemple IX

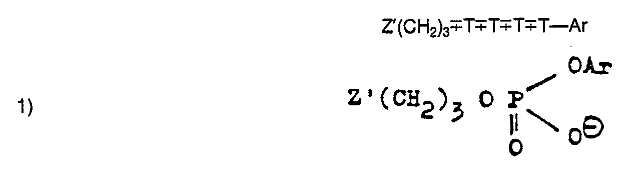

On ajoute sous agitation à ―20 °C le méthyl p-chlorophényl bromophosphate (3 équivalents) à un excès de pyridine anhydre (- 50 équivalents), on continue l'agitation pendant 15 minutes à la température ambiante puis ajoute le (w-hydroxypropyloxy)-8-psoralène (1 équivalent) : on abandonne la réaction pendant 2 heures à la température ambiante puis après un traitement habituel, le diester est purifié sur une colonne de silice et en utilisant successivement le mélange CH₂Cl₂, MeOH (90 :10. 85 : 15, puis 75^{.}: 25, v/v). Rendement - 75 %.
CCM : gel de silice ; CH₂CI₂, MeOH (85 : 15, v/v), Rf = 0,24.

### 2) T∓T∓T∓T∓CNEt

Le tétranucléotide totalement protégé (1 équivalent) (exemple VI-2) est traité avec une solution à 2 % d'acide benzènesulfonique dans CH₂CI₂, MeOH (7 : 3, v/v) (- 4 équivalents). Le mélange est dilué avec du chloroforme, lavé plusieurs fois avec une solution aqueuse à 5 % de NaHC0₃, puis séché sur Na₂S0₄ et concentré sous vide ; le solide obtenu est ensuite lavé plusieurs fois avec de l'éther. Le rendement de la préparation est de 82 %.

### 3) Z' (CH₂)₃∓T∓T∓T∓T∓CNEt

Le couplage. a été réalisé dans la pyridine anhydre en présence de diester IX-1 (1,3 équivalent) de T∓T∓T∓T∓CNEt (1 équivalent) et de MST (2,5 équivalents). On termine ensuite la préparation comme dans l'exemple VI-2 en purifiant le produit sur silice et en utilisant successivement les mélanges de solvants CH₂CI₂, H₂0, acétone (43 : 2 : 55 ; 32 : 3 : 65, v/v). Rendement - 55 %.
CCM : gel de silice ; CH₂Cl₂, MeOH (90 : 10, v/v), Rf - 0,31.
4) Décyanoéthylation
Le composé totalement protégé (exemple IX-3) est traité selon l'exemple V-1. Rendement - 80 %.
CCM : gel de silice ; CH₂Cl₂, MeOH (85 : 15, v/v), Rf - 0,64.

### Exemple X

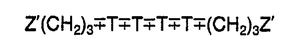

### 1) Ar―T∓T∓T∓T∓CNEt

On opère comme dans l'exemple IV-1 en remplaçant-le mononucléotide T∓CNEt par le tétranucléotide T∓T∓T∓T∓CNEt, on obtient le sel de pyridinium sous forme de solide blanc. Rendement ~ 92 %.
CCM : gel de silice ; CH₂Cl₂, MeOH (75 : 25, v/v), Rf - 0,47.

### 2) Ar―T∓T∓T∓T―Ar

En partant du tétranucléotide Ar―T∓T∓T∓T∓CNEt et en opérant comme dans l'exemple VI-1, on obtient le produit sous forme de solide blanc avec un rendement de 90 %.
CCM : gel de silice ; isoPrOH, TEA, H₂0 (85 : 5 : 10, v/v), Rf - 0,12.

3) On réalise le couplage dans la pyridine anhydre en utilisant 1 équivalent de tétranucléotide-3',5' bis-(arylphosphate) Ar―T∓T∓T∓T―Ar, 4 équivalents de (ω-hydroxypropyloxy)-8-psoralène et de 6 équivalents de MST et en opérant comme dans l'exemple IX-3, on obtient le produit sous forme de solide blanc.

CCM : gel de silice ; CH₂C1₂, MeOH (9 : 1, v/v), Rf - 0,5.

### Exemple XI

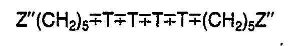

En utilisant le méthoxy-2 chloro-6 (ω-hydroxypentylamino)-9 acridine et en opérant selon l'exemple X-3, on a obtenu le produit sous forme de solide jaune.
CCM : gel de silice ; CH₂C1₂, MeOH (88 : 12, v/v), Rf - 0,27.

### Exemple XII

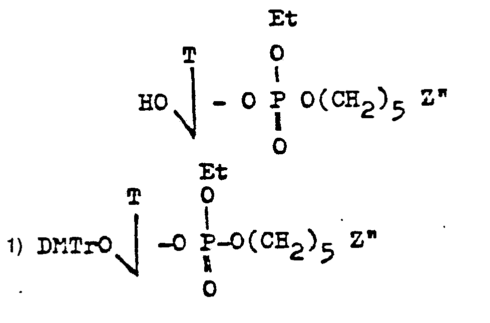

A une solution de fluorure de césium (5 équivalents) de concentration 6.10⁻⁴ molaire dans le mélange EtOH, CH₃CN (50 : 50, v/v) on ajoute sous agitation et à la température ambiante 1 équivalent de (DMTr)T∓(CH₂)₅Z" (exemple III) ; on" continue ensuite l'agitation pendant 2 heures à la même température puis purifie le produit sur gel de silice en utilisant le système de solvant CH₂Cl₂, EtOH (99 : 1 à 93 : 7, v/v). Le produit est obtenu sous forme de solide jaune. Rendement = 85 %.
CCM : gel de silice ; CH₂CI₂, MeOH (90 : 10, v/v), Rf - 0,22.

2) La détritylation a été réalisée par l'acide acétique selon l'exemple VII-1. Rendement = 78 %.
CCM : gel de silice ; CH₂Cl₂. MeOH (90 : 10, v/v), Rf - 0.1.

### Exemple XIII

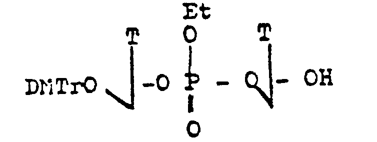

### 1) (DMTr) T∓T

a) On fait réagir pendant 1 heure 30 sous agitation et à la température ambiante un mélange de 5'-0-(diméthoxytrityl)-thymidine 3'-(p-chlorophényl phosphate) de pyridinium (exemple 1-1) (1 équivalent) de thymidine (1,5 équivalent), de tétrazole (6 équivalents) et de mésitylène sulfonyl chlorure (1,5 équivalent) dans la pyridine anhydre. On termine la préparation comme dans l'exemple l-2 puis purifie le produit sur gel de silice en utilisant le système CH₂CI₂, EtOH (99 : 1 à 94 : 6, v/v). On obtient le produit sous forme de stéréoisomère ; le rendement de la préparation est de 78 %.
   CCM : gel de silice ; CH₂CI₂, MeOH (90 : 10, v/v),
   1 tache : Rf - 0,36
   CH₂CI₂, THF (5 : 4, v/v),
   2 taches : Rf = 0,48 - 0,42.
b) La séparation des isomères a été réalisée par chromatographie préparative sur plaques (gel de silice 60 F 254), solvant CH₂Cl₂, THF (5 : 4, v/v).
   CCM : gel de silice ; CH2CI2, THF (5 : 4, v/v)
   isomère a Rf = 0,48
   isomère (β Rf = 0,42

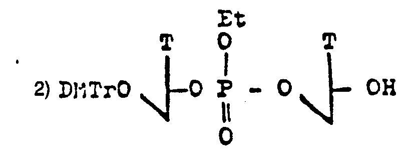

a) On opère comme dans l'exemple XII-1 et en remplaçant le composé (DMTr) T∓(CH₂)₅Z" par le stéréoisomère (DMTr) T∓T (ou par son isomère a ou par son isomère β), on obtient l'ester éthylé sous forme de stéréoisomère avec un rendement de 83 %.
   CCM : gel de silice ; CH₂CI₂, MeOH (90 : 10, v/v),
   1 tache : Rf - 0,27
   CH₂Cl₂, THF (5 : 4, v/v),
   2 taches : Rf = 0,32 - 0,24.
b) La séparation des isomères a été réalisée comme précédemment par chromatographie préparative sur plaques.
   CCM : gel de silice; CH₂Cl₂, THF (5 : 4, v/v)
   isomère α Rf = 0,32
   isomère β Rf = 0,24.

### Exemple XIV

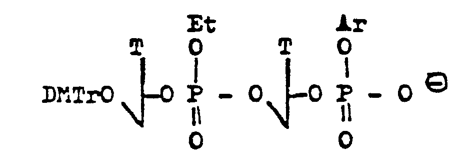

1) On opère comme dans l'exemple IV-1 en remplaçant le nucléotide T∓CNEt par le composé 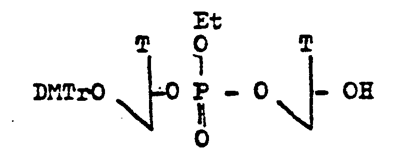 on obtient le produit sous forme de sel de pyridinium avec un rendement de 90 %.
CCM : gel de silice ; isoPrOH, TEA, H₂0 (85 : 5 : 10, v/v), Rf = 0,52.

### Exemple XV

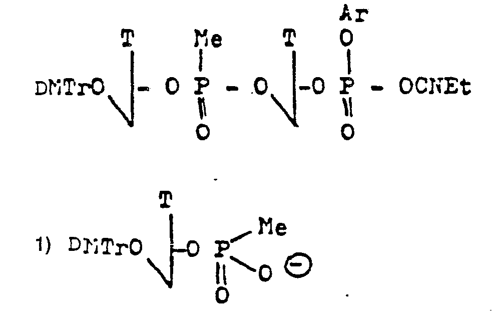

On fait réagir pendant une nuit à la température ambiante un mélange de 5'-0-(diméthoxytrityl)-thymidine (1 équivalent) et d'anhydride méthylphosphonique (6 équivalents) (brevet URSS N° 226606 du 9/1/67) dans la pyridine on termine ensuite-la préparation comme dans l'exemple I-1, puis purifie le produit sur silice. Rendement = 85 %
CCM : gel de silice ; isoPrOH, Et₃N, H₂0 (85 : 5 : 10, v/v), Rf - 0,4.

2) On réalise le couplage du nucléoside-3' méthylphosphonate XV-1 (1,5 équivalent) avec la thymidine-3'(p-chlorophényl p-cyanoéthylphosphate) (1 équivalent) en présence de MST (3 équivalents) puis termine la préparation comme dans l'exemple VI-2. Rendement = 60 %.
CCM : gel de silice ; CH₂CI₂, MeOH (87 : 13, v/v), Rf = 0,58 - 0,61.

### Exemple XVI

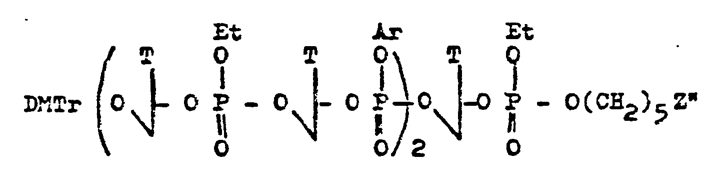

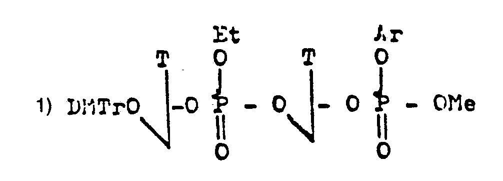

On ajoute sous agitation à - 20 °C du méthyl p-chlorophényl bromophosphate (3 équivalents) à une solution de 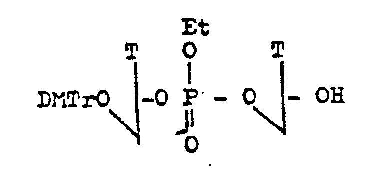 (1 équivalent) préparé selon l'exemple XIII-2a et de méthyl-1-imidazole (6 équivalents) dans l'acétonitrile puis continue l'agitation pendant 30 minutes à la température ambiante. Après avoir détruit l'excès de bromophosphate par addition d'eau, le produit est extrait avec du chloroforme puis purifié sur gel de silice. Rendement = 75 %.
CCM : gel de silice ; CH₂Cl₂, MEOH (9 : 1, v/v), qf ≃ 0,53.

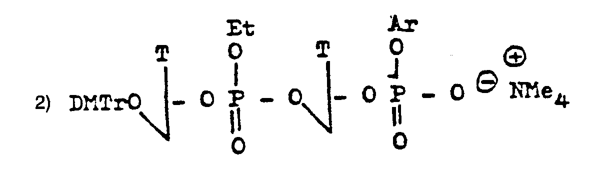

Le dinucléotide préparé selon l'exemple XVI-1 est traité pendant 1 à 2 heures à la température ambiante avec une solution de triméthylamine à 20 % dans l'acétonitrile ; on chasse le solvant sous vide et obtient le produit sous forme de sel de tétraméthylammonium.

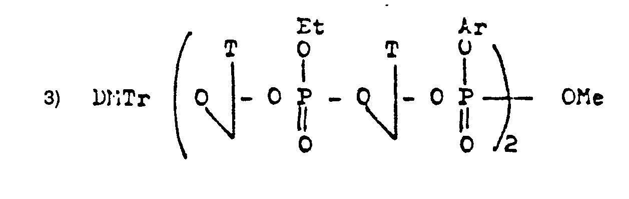

En opérant comme dans l'exemple VI-2 en remplaçant le composé (DMTr) T∓T∓CNEt par le composé préparé selon l'exemple XVI-1 et le dinucléotide VI-1 par le composé XVI-2 on a préparé le tétranucléotide.
CCM : gel de silice ; CH₂Cl₂, MeOH (9 : 1, v/v), Rf - 0,43.

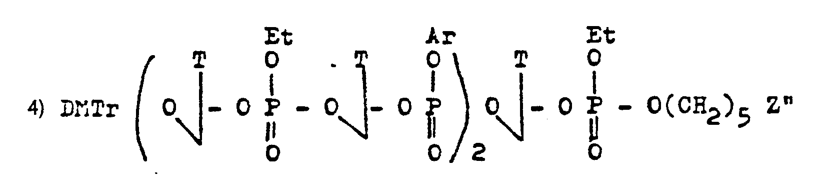

Le tétranucléotide XVI-3 (1 équivalent) déméthylé selon l'exemple XVI-2 est ensuite couplé avec le mononucléotide (1 équivalent) préparé selon l'exemple XII-2 en présence de MST (2 équjvalents) ; on termine la préparation comme dans l'exemple VI-2.
CCM : gel de silice ; CH₂Cl₂, MeOH (85 : 15, v/v), Rf = 0,39.

### Exemple XVII

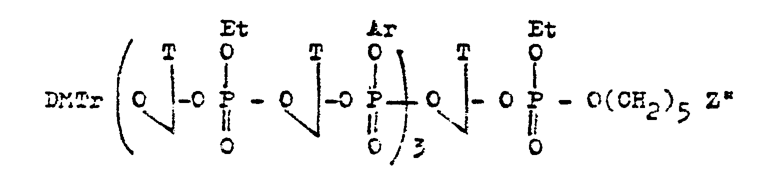

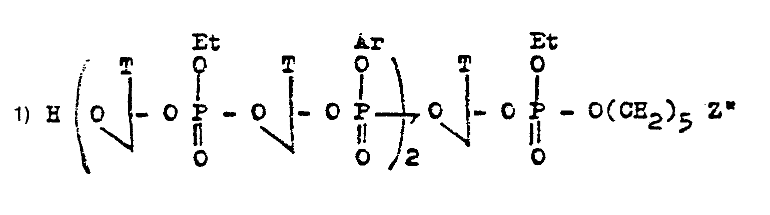

En partant du pentanucléotide XVI-4 et en opérant comme dans l'exemple IX-2, on a obtenu le produit sous forme de solide jaune.
CCM : gel de silice ; CH₂CI₂, MeOH (85 : 15, v/v), Rf - 0,26.

2) Le pentanucléotide XVII-1 (1 équivalent) est couplé avec le dinucléotide XVI-2 (1,5 équivalent) en présence de MST (3 équivalents) ; on termine ensuite la préparation comme dans l'exemple VI-2.
CCM : gel de silice ; CH₂Cl₂, MeOH (85 : 15, v/v); Rf - 0,35.

### Exemple XVIII

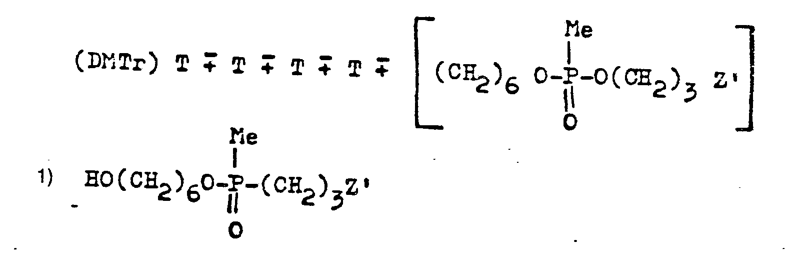

A une solution d'hydroxy-1 benzotriazole (2,2 équivalents), de pyridine (2,2 équivalents) dans le tétrahydrofuranne, on ajoute à 0 °C sous agitation le méthyl-dichlorophosphonate (1,1 équivalent) puis continue l'agitation 1 heure à 20 °C. A ce mélange on ajoute le (w-hydroxypropyloxy)-8-psoralène (1 équivalent) après 1 heure de réaction à la température ambiante, on ajoute ensuite l'hexanediol-1,6 (4 équivalents) et le N-méthylimidazole (4 équivalents) puis continue l'agitation pendant 3 heures à la température ambiante. Après extraction avec du chloroforme et lavage avec de l'eau le produit est ensuite purifié sur gel de silice. Le rendement de la préparation est de 62 %.
CCM : gel de silice ; CH₂CI₂, MeOH (9 : 1, v/v), Rf - 0,32.

2) En partant du composé préparé précédemment (exemple XVIII-1) et en opérant selon l'exemple Vl-4, on a préparé le produit protégé qui est purifié sur gel de silice avec le système CH₂CI₂, H₂0, acétone (46 : 1,5 : 52,5 ; 43 : 2 : 55, v/v). Rendement - 75 %.
CCM : gel de silice ; CH₂Cl₂, MeOH (9 : 1, v/v), Rf - 0,64.

### Exemple XIX

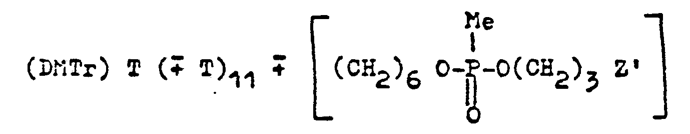

### 1) (DMTr) T(∓T)₇∓CNEt

On fait réagir pendant 2 heures à la température ambiante un mélange de diester (1,2 équivalent) préparé selon l'exemple VI-3, de triester (1 équivalent) préparé selon l'exemple IX-2 et de MST (3 équivalents) ; on termine ensuite la préparation comme dans l'exemple VI-2.
CCM : gel de silice ; CH₂Cl₂, MeOH (9 : 1, v/v), Rf - 0,61.

### 2) (DMTr) T(∓T)₆∓T―Ar

En partant du composé (DMTr) T(∓T)₇∓CNEt et en opérant selon l'exemple VI-1, on a obtenu le diester sous forme de solide blanc.

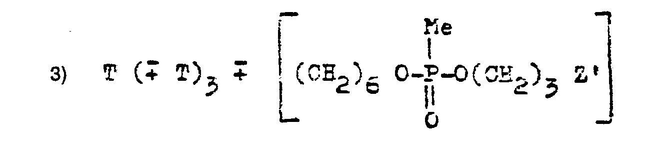

En partant du composé préparé selon l'exemple XVIII-2 et en opérant comme dans l'exemple IX-2, on a obtenu le produit sous forme de solide blanc.

### 4) Couplage

En partant du diester XIX-2 et du triester XIX-3 et en opérant selon XIX-1, on a préparé le dodécanucléotide protégé qui est purifié sur gel de silice avec le système CH₂CI₂, H₂0, acétone (20 : 5 : 75, v/v).
CCM : gel de silice ; CH₂CI₂, MeOH (9 : 1, v/v), Rf - 0,4.

### Exemple XX

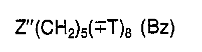

### 1) Z"(CH₂)₅ (∓T)₄∓CNEt

En opérant comme dans l'exemple VI-4 et en utilisant le diester préparé selon l'exemple X-1

(1 équivalent) et le méthoxy-2 chloro-6 (w-hydroxypentylamino)-9 acridine, on a obtenu le produit sous forme de solide jaune avec un rendement de 73 %.
CCM : gel de silice ; CH₂Cl₂, MeOH (85 : 15, v/v), Rf - 0,4.

### 2) Z"(CH₂)₅ (∓T)₄―Ar

Le tétranucléotide totalement protégé (XX-1) est traité selon l'exemple V-1. Rendement - 85 %.
CCM : gel de silice ; CH₂Cl₂, MeOH (85 : 15, v/v), Rf - 0,11.

3) On fait réagir pendant 2 heures sous agitation et à la température ambiante dans la pyridine anhydre un mélange de Z"(CH₂)₅ (∓T)₄―Ar (1 équivalent) de T(∓T)₃ (Bz) (1.2 équivalent) et de MST (2 équivalents), puis termine la préparation comme dans l'exemple VI-2.
CCM : gel de silice ; CH₂Cl₂, MeOH (85 : 15, v/v), Rf - 0,45.

### Exemple XXI

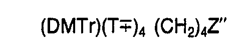

En opérant comme dans l'exemple VI-4 et en utilisant le méthoxy-2 chloro-6 (w-hydroxybutylamino)-9 acridine (1,5 équivalent) à la place du dérivé du psoralène on obtient le produit totalement protégé avec un rendement de 82 %.
CCM : gel de silice : CH₂Cl₂, MeOH (90 : 10, v/v), Rf = 0,18.

### Exemple XXII

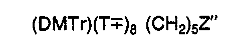

### 1) (T∓)4 (CH₂)₅Z"

Le produit est obtenu par détritylation du composé de l'exemple VIII.
CCM : gel de silice ; CH₂Cl₂, MeOH (85 : 15, v/v), Rf - 0,3.

2) On fait réagir pendant 1 heure à la température ambiante, en solution dans la pyridine, 1 équivalent de (T∓)₄(CH₂)₅Z", 1,2 équivalent de (DMTr)(T∓)₃T―Ar (Exemple VI-3) et 2,5 équivalents de MST puis termine la préparation comme dans l'exemple VI-2 en utilisant le système : CH₂Cl₂, H₂0, acétone (20 : 5 : 75, v/v). Rendement 73 %.
CCM : gel de silice ; CH₂Cl₂, MeOH (85 : 15, v/v), Rf ≃0,37.

### Exemple XXIII

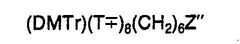

En partant du diester (DMTr)T(∓T)₆∓T―Ar de l'exemple XIX-2 (1 équivalent) et du méthoxy-2 chloro)6(w-hydroxyhexylamino)-9 acridine (2 équivalents) et du MST (2 équivalents) et en opérant selon XXII-2 on a obtenu le produit avec un rendement de 72 %.
CCM : gel de silice ; CH₂CI₂, MeOH (85 : 15, v/v), Rf - 0,38.

### Exemple XXIV

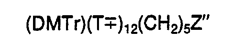

En utilisant le composé (DMTr)T(∓T)₆∓T―Ar (Exemple XIX-2) (1 équivalent) et le composé (T∓)₄(CH₂)₅Z" (Exemple XXII-1) (1,2 équivalent) et en opérant comme dans l'exemple XXII-2, on a obtenu le dodécanucléotide totalement protégé avec un rendement de 88 %.
CCM : gel de silice ; CH₂Cl₂, MeOH (85 : 15, v/v), Rf - 0,32.

### Exemple XXV

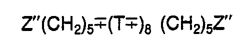

### 1) Ar―(T∓)₈CNEt

Le (T∓)₈CNEt préparé par détritylation de (DMTr)(T∓)₈CNEt (Exemple XIX-1) est phosphorylé comme dans l'exemple IV-1.
CCM : gel de silice ; CH₂CI₂, MeOH (85 : 15, v/v), Rf - 0,12
CH₂CI₂, MeOH (75 : 25, v/v), Rf - 0,6.

### 2) Ar―(T∓)₇T―Ar

En partant de Ar―(T)₈CNEt et en opérant comme dans l'exemple VI-1, on obtient le produit sous forme solide.
CCM, gel de silice ; CH₂Cl₂, MeOH (75 : 25, v/v), Rf - 0,15.

3) En utilisant Ar―(T∓T―Ar (1 équivalent) le méthoxy-2-chloro-6(ω-hydroxypentylamino)-9 acridine (3 équivalents) et MSTe (5 équivalents) et en opérant comme dans l'exemple XXII-2, on a obtenu l'octanucléotide.
CCM : gel de silice ; CH₂Cl₂, MeOH (85 : 15, v/v), Rf - 0,3.

### Exemple XXVI

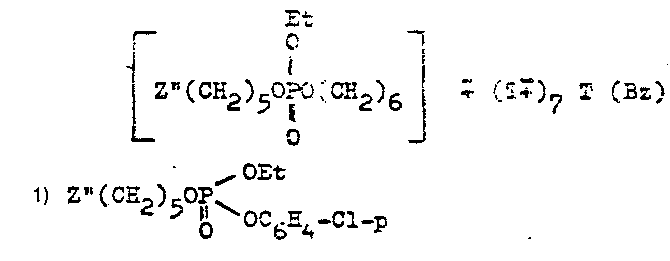

On ajoute à ―10 °C le p-chlorophényl-éthyl-bromophosphate (2 équivalents) à la solution de méthoxy-2 chloro-6(w-hydroxypentylamino)-9 acridine (1 équivalent) dans la pyridine ; après 20 minutes à la température ambiante on détruit l'excès de bromophosphate par addition d'eau puis extrait le produit avec du chloroforme et le purifie sur silice.
CCM : gel de silice ; CH₂Cl₂, MeOH (90 : 10, v/v), Rf - 0,42.

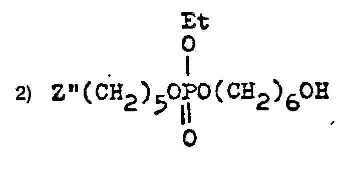

On fait réagir pendant 24 heures à la température ambiante un mélange de composés

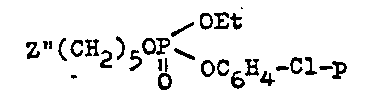

(1 équivalent), d'hexanediol-1,6 (6 équivalents) et de fluorure de césium (5 équivalents) dans l'acétonitrile puis purifie le produit sur gel de silice.
CCM : gel de silice ; CH₂Cl₂, MeOH (90 : 10, v/v), Rf - 0,23.

3) Ar―(T∓)₇T(Bz)

(T∓)₇T(bz) obtenu par détritylation de (DMTr)(T∓)₇T(bz) est phosphorylé selon l'exemple IV-1. CCM : gel de silice ; CH₂CI₂, MeOH (85 : 15, v/v), Rf - 0,14.

4) En partant de 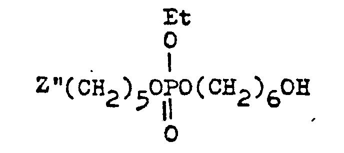 (1,5 équivalent) de Ar―(T∓)₇T(Bz) (1 équivalent) et de MST (2,5 équivalents) et en opérant comme dans l'exemple XXII-2, on a obtenu le produit.
CCM : gel de silice ; CH₂C1₂, MeOH (90 : 10, v/v), Rf - 0,2.

### Exemples XXVII et XXVIII

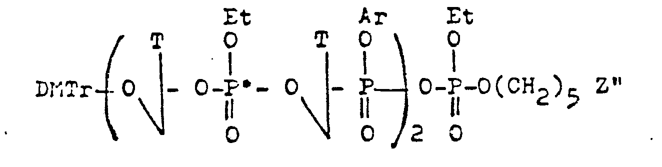

Isomère α : On opère comme dans l'exemple XVI en remplaçant le diastéréoisomère 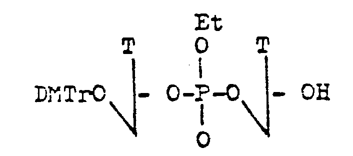 par l'isomère α (exemple XIII-2b) (CCM : gel de silice ; CH₂CI₂, THF (5 : 4, v/v), Rf - 0,32). On a obtenu le pentanucléotide dont les deux groupes phosphates d'éthyle internucléotidiques sont sous forme d'isomère a.
CCM : gel de silice : CH₂Cl₂, MeOH (85 : 15, v/v), Rf = 0,39.

Isomère β: En partant de l'isomère β du dinucléotide-monophosphate 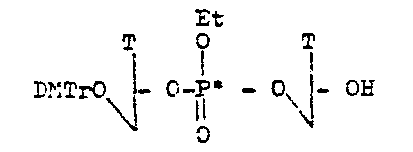 (exemple XIII-2b), (CCM : gel de silice ; CH₂Cl₂, THF (5:4, v/v), Rf = 0,24) et en opérant comme . précédemment, on a obtenu le pentanucléotide dont les deux groupes phosphate d'éthyle internucléotidiques sont sous forme d'isomère β.
CCM : gel de silice, CH₂Cl₂, MeOH (85 15, v/v), Rf - 0,39.

### Exemple XXIX

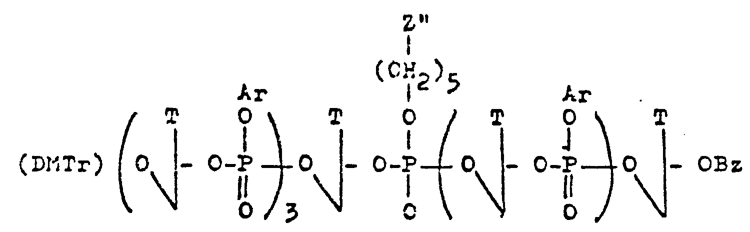

### 1) 5'-0-(diméthoxytrityl)-thymidine-3' phosphate d'ammonium

On ajoute sous agitation à -20 °C le méthyldichlorophosphate (4 équivalents) à la pyridine ~ 1 000 équivalents), après 15 minutes on ajoute à ce mélange le 5'-O-(diméthoxytrityl-thymidine) (1 équivalent) puis continue l'agitation pendant 30 minutes. Après avoir détruit l'excès de réactif de phosphorylation par addition d'eau, le produit est extrait avec du chloroforme puis purifié sur silice (Solvant : isoPrOH, NH₄0H, H₂0) (7 : 2 : 1, v/v). Rendement - 75 %.
CCM : gel de silice ; IsoPrOH, NH₄0H, H₂0 (7 : 2 : 1, v/v), Rf - 0,65.

### 2) (DMTr)T-(T∓)₃T(Bz)

On fait réagir pendant 3 heures à la température ambiante une solution de 5'-O-(diméthoxytrityl)-thymidine-3' phosphate de triéthylammonium (2 équivalents) (obtenu par coévaporation du sel d'ammonium avec une solution de triéthylamine dans la pyridine), de (T∓)₃T(Bz) (1 équivalent), de 2,4,6-triisopropylbenzènesulfonyl-chlorure (3 équivalents) dans la pyridine ; après 1 traitement habituel le produit est ensuite purifié sur silice (plaque préparative) en utilisant le système de solvant : CH₂CI₂, MeOH (85 : 15, v/v). Rendement - 78 %.
CCM : gel de silice ; CH₂CI₂, MeOH (80 : 20, v/v), Rf - 0,3.

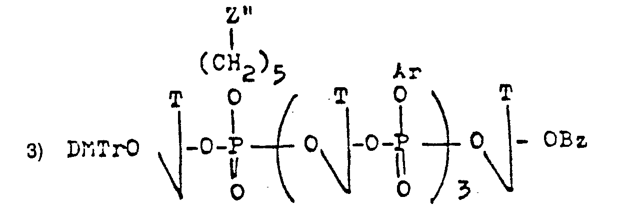

On fait réagir pendant 6 heures à la température ambiante une solution de (DMTr)T―(T∓)₃T(Bz) (1 équivalent) préparé précédemment, de méthoxy-2-chtoro-6(ω-hydroxypentylamino)-9 acridine (2 équivalents), de MST (5 équivalents) dans la pyridine puis termine la préparation comme dans l'exemple XXII-2. Rendement - 65 %.
CCM : gel de silice ; CH₂CI₂, MeOH (85 : 15, v/v), Rf - 0,33.

4) Le composé 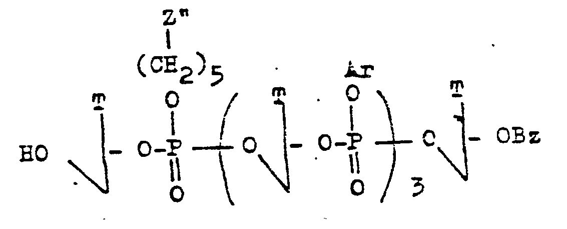 (1 équivalent) (CCM : gel de silice ; CH₂CI₂, MeOH (85 : 15, v/v), Rf - 0,2, préparé par détritylation du composé de l'exemple XXIX-3) est couplé avec le diester (DMTr)T∓T∓T―Ar (1,5 équivalent) en présence de MST (2,5 équivalents) dans la pyridine, on termine ensuite la préparation comme dans l'exemple XXII-2. Rendement - 63 %.
CCM : gel de silice ; CH₂Cl₂, MeOH (85 : 15, v/v), Rf - 0,4.

### Exemple XXX

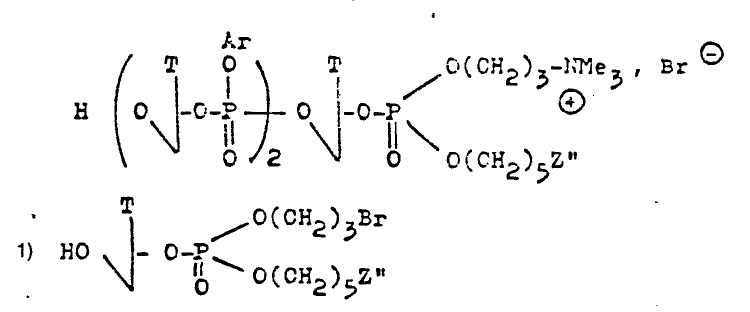

On opère comme dans l'exemple XII en remplaçant l'éthanol par le bromo-3-propanol-1.
CCM : gel de silice ; CH₂Cl₂, MeOH (90 :10, v/v), Rf = 0,18.

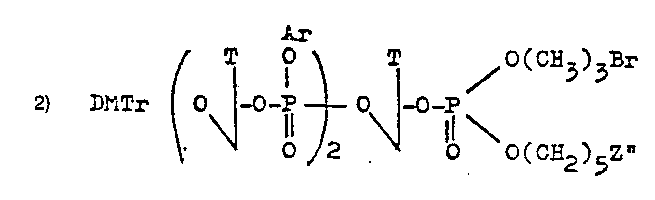

Le produit est obtenu par couplage du dinucléotide (DMTr)T∓T―Ar (2 équivalents) avec le mononucléotide (Exemple XXX-1) (1 équivalent) en présence de MST (3 équivalents).
CCM : gel de silice ; CH₂CI₂, MeOH (85 : 15, v/v), Rf = 0,21 et 0,27.

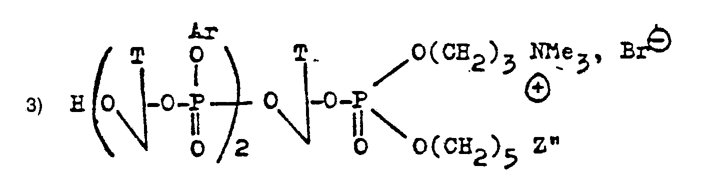

Le trinucléotide 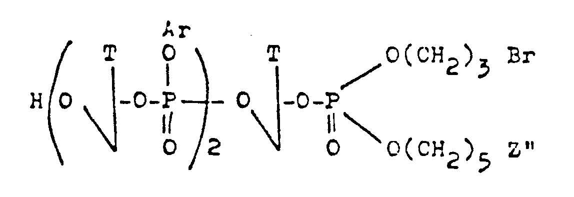 (CCM : gel de silice silanisée ; acétone, eau (16 : 6, v/v), Rf - 0,5) (préparé par détritylation du composé de l'exemple XXX-2) est traité pendant quelques heures à la température ambiante avec une solution de triméthylamine dans l'acétonitrile. Après évaporation du solvant, le produit est obtenu sous forme de solide jaune.
CCM : gel de silice silanisée ; acétone, eau (16 : 6, v/v), Rf = 0,05.

### Exemple XXXI

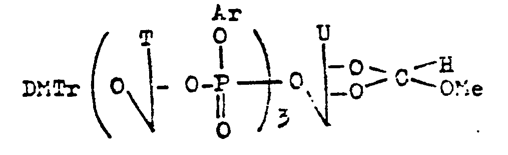

### 1) (DMTr)(T∓)₃CNEt

On opère comme dans l'exemple VI-2 en remplaçant le dinucléotide (DMTr)T∓T―Ar par le mononucléotide (DMTr)T-Ar, on a préparé le trinucléotide.

### 2) Ar―(T∓)₃CNEt

(T∓)₃CNEt préparé par détritylation de (DMTr(T∓)₃CNEt est phosphorylé selon l'exemple IV-1.
CCM : gel de silice ; CH₂CI₂, MeOH (90 10, v/v), Rf = 0
CH₂Cl₂, MeOH (80 : 20, v/v), Rf = 0,5.

### 3) Z"(CH₂)₅∓(T∓)₃CNEt

En couplant le trinucléotide Ar―(T∓)₃CNEt (1 équivalent) avec le méthoxy-2 chloro-6(w-hydroxypenty- lamino)-9 acridine (2 équivalents) en présence de MST (3 équivalents), on a obtenu le trinucléotide totalement protégé avec un rendement de 70 %.
CCM : gel de silice ; CH₂CI₂, MeOH (90 10, v/v), Rf = 0,1
CH₂Cl₂, MeOH (85 : 15; v/v), Rf = 0,35.

4) Le diester Z"(CH₂)₅∓(T∓)₂T―Ar (1 équivalent) préparé par décyanoéthylation de Z"(CH₂)₅∓(T∓)₃CNEt selon l'exemple VI-1 est couplé avec le 2',3'-0-méthoxyméthylidène-uridine (2 équivalents) en présence de MST (3 équivalents) ; après un traitement habituel, le produit est purifié sur gel de silice.
CCM : gel de silice ; CH₂Cl₂, MeOH (90 : 10, v/v), Rf = 0,36
CH₂CI₂, MeOH (85 : 15, v/v), Rf = 0,53.

### Exemple XXXII

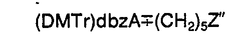

1) 5'-O-(diméthoxytrityl) N-benzoyldésoxyadénosine-3'-(p-chlorophényl phosphate) de pyridinium.

En partant du 5'-O-(diméthoxytrityl) N-benzoyldésoxyadénosine et en opérant comme dans l'exemple I-1a on a préparé le produit.
CCM : gel de silice ; isoPrOH, TEA, H₂0 (85 : 5 : 10, v/v), Rf - 0,5.

2) On opère comme dans l'exemple I-2 en remplaçant le (w-hydroxypropyloxy) 8-psoralène par le méthoxy-2-chloro-6(w-hydroxypentylamino)-9 acridine et le diester dérivé de la thymine par le diester dérivé de l'adénine. Rendement = 80 %.
CCM : gel de silice ; CH₂CI₂, MeOH (90 : 10, v/v), Rf = 0,42.

### Exemple XXXIII

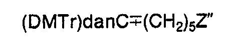

En partant de 5'-0-(diméthoxytrityl) N-anisoyldésoxycytidine et en opérant selon l'exemple XXXII. on a préparé le 5'-O-(diméthoxytrityl) N-anisoyl désoxycytidine-3'-(p-chlorophénylphosphate) de pyridinium (DMTr)danC-Ar [CCM, gel de silice ; isoPrOH, TEA, H₂0 (85 : 5 : 10, v/v), Rf ~ 0,47] puis le composé (DMTr)danC∓(CH₂)₅Z". Rendement = 80 %.
CCM : gel de silice ; CH₂CI₂, MeOH (90 : 10, v/v), Rf = 0,4.

### Exemple XXXIV

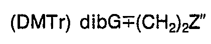

En partant du 5'-O-(diméthoxytrityl)-N-isobutyryl-désoxyguanosine et en opérant selon l'exemple XXXII, on a préparé le 5'-O-(diméthoxytrityl)-N-isobutyryl-désoxyguanosine-3'-(p-chlorophénylphosphate) de pyridinium (DMTr)dibG-Ar [CCM : gel de silice, isoPrOH, TEA, H₂0 (85 : 5 : 10, v/v), Rf - 0,45], puis le composé (DMTr)dibG∓(CH₂)₅Z". Rendement = 70 %.
CCM : gel de silice ; CH₂Cl₂, MeOH (90 : 10 ; v/v), Rf = 0,43.

### Exemple XXXV

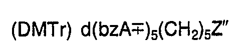

### 1) (DMTr) d(bzA∓)₃bz―A―Ar

En partant du dinucléotide (DMTr) dbzA∓bzA∓CNEt (biochimie, 1981, 775) et en opérant comme dans les exemples VI-1, VI-2 et VI-3, on a préparé le tétranucléotide.

2) Le composé dbzA∓(CH₂)₅Z" (1 équivalent) (obtenu par détritylation du composé (DMTr) dbzA∓(CH₂)₅Z", exemple XXXII) est couplé avec le tétranucléotide XXXV-1 (1,5 équivalent) en présence de MST (3 équivalents) ; après un traitement habituel, le produit est purifié sur silice en utilisant successivement les systèmes CH₂Cl₂, H₂0, acétone (46 : 2 : 52 et 40 : 2 : 58, v/v).
CCM : gel de silice ; CH₂Cl₂, MeOH (90 10, v/v), Rf = 0,16.

### Exemple XXXVI

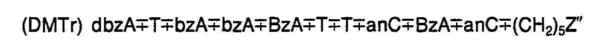

### 1) (DMTr) dbzA∓T∓bzA∓bzA∓CNEt

En partant de (DMTr) dbzA∓T―Ar (1,2 équivalent) de dbzA∓bzA∓CNEt (2 équivalents) et de MST (3 équivalents) on a préparé le tétranucléotide qui est purifié sur gel de silice en utilisant les systèmes CH₂Cl₂, H₂0, acétone (52 : 2 : 46, 43 : 2 : 55, v/v).
CCM : gel de silice ; CH₂Cl₂, MeOH (90 10, v/v), Rf - 0,35.

### 2) (DMTr) dbzA∓T∓bzA∓bzA∓bzA∓T∓CNEt .

Le tétranucléotide de l'exemple XXXVI-1 qui est décyanoéthylé par la triéthylamine selon l'exemple VI-1 est ensuite couplé avec le dinucléotide dbzA∓T∓CNEt en présence de MST. Après un traitement habituel le produit est purifié sur gel de silice en utilisant le système CH₂Cl₂, H₂0-acétone (43 : 2 : 55, v/v).

### 3) (DMTr) dbzA∓anC∓(CH₂)₅Z"

danC∓(CH₂)₅Z" (1 équivalent) (obtenu par détritylation du composé (DMTr) danC∓(CH₂)₅Z" selon l'exemple VII-1) est couplé avec le nucléotide préparé selon l'exemple XXXII-1 (1,2 équivalent) en présence de MST (3 équivalents), après un traitement habituel, le produit est purifié sur gel de silice en utilisant successivement les systèmes CH₂Cl₂, H₂0, acétone (59 : 1 : 40, 53 : 2 : 45, 46 : 2 : 52).

### 4) (DMTr) dT∓anC∓bzA∓anC∓(CH₂)Z"

Le couplage du dinucléotide (DMTr) dT∓anC―Ar (1,2 équivalent) avec le dinucléotide dbzA∓anC∓(CH₂)₅Z" (obtenu par détritylation du composé XXXVI-3) (1 équivalent) donne le tétranucléotide.
CCM : gel de silice ; CH₂CI₂, MeOH (90 : 10, v/v), Rf - 0,42.

5) Le couplage de l'hexanucléotide (DMTr) dbzA∓T∓bzA∓bzA∓bzA∓T―Ar (1,2 équivalent) (obtenu par décyanoéthylation du composé de l'exemple XXXVI-2) avec le tétranucléotide dT∓anC∓bzA∓anC∓(CH₂)₅Z" (1 équivalent) (préparé par détritylation du composé de l'exemple XXXVI-4) donne le décanucléotide totalement protégé qui est purifié sur gel de silice en utilisant successivement les systèmes CH₂Cl₂, H₂0, acétone (63 1 : 36, 32 : 3 : 65, v/v).
CCM : gel de silice ; CH₂CI₂, MeOH (90 : 10, v/v), Rf - 0,24.

### Exemple XXXVII

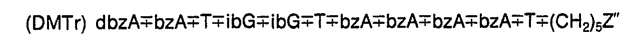

1) (DMTr) dbzA∓bzA∓bzA∓bzA∓T∓(CH₂)₅Z"

On fait réagir pendant 2 heures à la température ambiante, en solution dans la pyridine, le tétranucléotide (DMTr) dbzA∓bzA∓bzA∓bzA―Ar (1 équivalent) (Exemple XXXV-1) avec le mononucléotide T∓(CH₂)₅Z" (1 équivalent) (Exemple VII-1) (en présence de MST (3 équivalents) puis termine la préparation comme dans l'exemple XXXV-2. Rendement = 50 %.
CCM : gel de silice ; CH₂CI₂, MeOH (90 : 10, v/v), Rf - 0,18.

2) (DMTr) dT∓ibG∓ibG∓T∓CNEt

En partant de (DMTr) dT∓ibG―Ar, de dibG∓T∓CNEt et en opérant comme dans l'exemple XXXV-2 on a obtenu le tétranucléotide avec un rendement de 50 %.
CCM : gel de silice ; CH₂CI₂, MeOH (90 : 10, v/v), Rf = 0.48.

3) (DMTr) dbzA∓bzA∓T∓ibG+ibG∓T∓CNEt

Le tétranucléotide dT∓ibG∓ibG∓T∓CNEt (1 équivalent) (préparé par détritylation du composé XXXVII-2) est couplé avec le dinucléotide (DMTr) dbzA∓bzA―Ar (1,3 équivalent) en présence de MST (3 équivalents) ; après un traitement habituel, le produit est purifié sur silice en utilisant le système CH₂C1₂, H₂0, acétone (43 : 2 : 55, v/v). Rendement 52 %.
CCM : gel de silice ; CH₂CI₂, MeOH (90 : 10, v/v), Rf = 0,42.

4) L'hexanucléotide (DMTr) dbzA∓bzA∓T∓ibG∓ibG∓T―Ar (1,2 équivalent) (préparé par décyanoéthylation du composé XXXVII-3 selon l'exemple VI-1) est couplé avec le pentanucléotide dbzA∓bzA∓bzA∓bzA∓T∓(CH₂)₅Z" (1 équivalent) (obtenu par détritylation du composé XXXVII-1) en présence de MST (3 équivalents) ; après un traitement habituel le produit est purifié sur silice en utilisant le système CH₂Cl₂, H₂0, acétone (32 : 3 : 65, v/v).
CCM : gel de silice ; CH₂Cl₂, MeOH (90 : 10, v/v), Rf = 0,2.

### Exemple XXXVIII

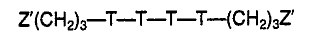

On fait réagir sous agitation pendant une nuit et à la température ambiante l'oligonucléotide totalement protégé préparé selon l'exemple X, avec une solution molaire en acide benzohydroxamique et en 1,8-diazabicyclo(5,4,0) undec-7-ene (DBU) dans la pyridine anhydre et en utilisant 10 équivalents de C₆H₅ÇNHOH-DBU par équivalent de phosphoester arylé à déprotéger. On neutralise le milieu réactionnel 0 avec du DOWEX _{§}0 (forme pyridinium), filtre et lave la résine avec 1 mélange H₂0, MeOH (1 : 1, v/v), puis chasse !e solvant sous vide. Le produit est ensuite purifié sur une colonne de DEAE Séphacel, en utilisant le gradient d'élution (10-3 M-0,5 M) en NH₄HC0₃ dans H₂0, MeOH (70 : 30, v/v).
CCM : gel de silice, isoPrOH, NH₄0H, H₂0 (65 : 9 : 15, v/v), Rf - 0,25.

### Exemple XXXIX

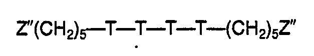

En partant du tétranucléotide préparé selon l'exemple XI et en opérant comme dans l'exemple XXXVIII, on a isolé le produit sous forme de solide jaune. CCM : gel de silice ; isoPrOH, NH₄0H, H₂0 (65 : 9 : 15, v/v), Rf~0.4.

### Exemple XXXX

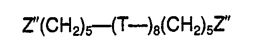

On déprotège l'octanucléotide Z"(CH₂)₅∓(T∓)₈(CH₂)₅Z" (exemple XXV) comme dans l'exemple XXXVIII. Le produit est purifié sur DEAE Séphacel en utilisant le gradient d'élution (5.10-² M―0,8 M) en NH₄HC0₃ dans H₂0. MeOH (50 : 50, v/v).
CCM : gel de silice, isoPrOH, NH₄0H, H₂0 (85 : 9 : 25, v/v), Rf = 0,42.

### Exemples XXXXI à LVI

On réalise la déarylation comme dans l'exemple XXXVIII ; le résidu obtenu après évaporation du solvant est traité avec l'acide acétique à 80 % pendant 1 à 2 heures à la température ambiante. On élimine l'acide acétique sous vide par évaporation plusieurs fois avec de l'éthanol ; on reprend le résidu avec de l'eau puis lave la phase aqueuse avec de l'éther. Les produits sont ensuite purifiés sur DEAE Séphacel en utilisant des gradients d'élution en NH₄HC0₃ en rapport avec le nombre de charges (10₋³ M―0.2 M à 10-³ M-1 M).

### (Voir Schémas pages 26 à 29)

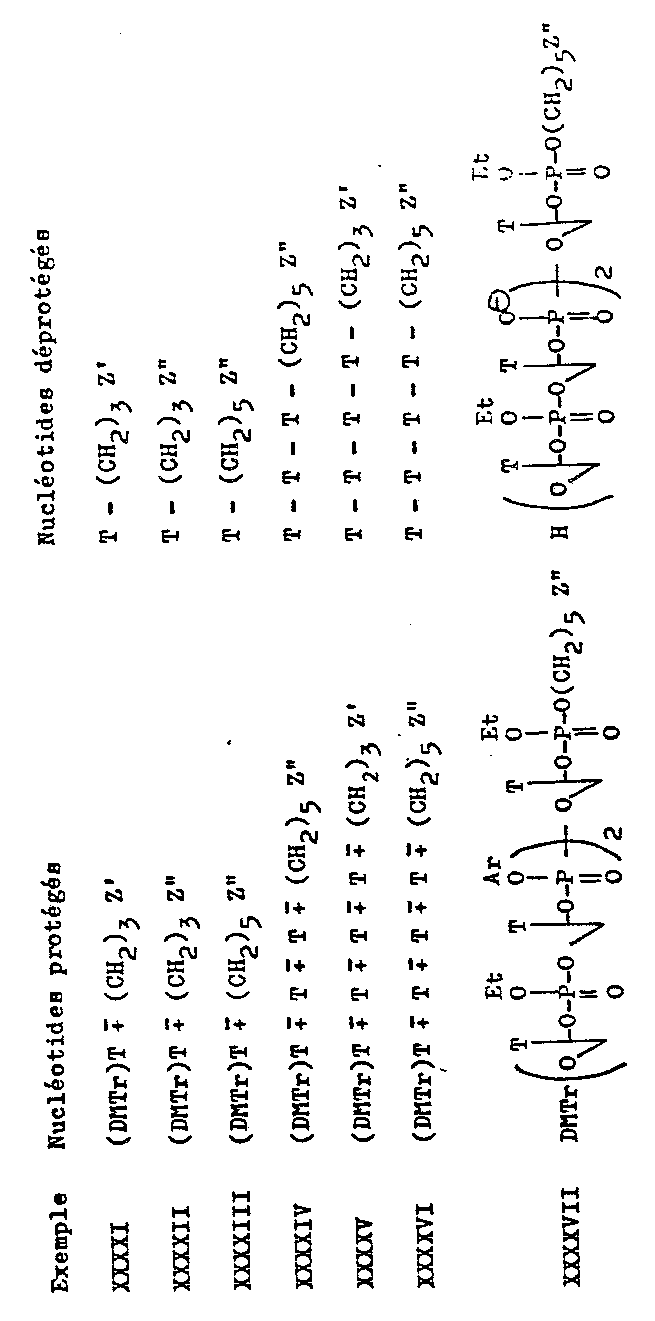

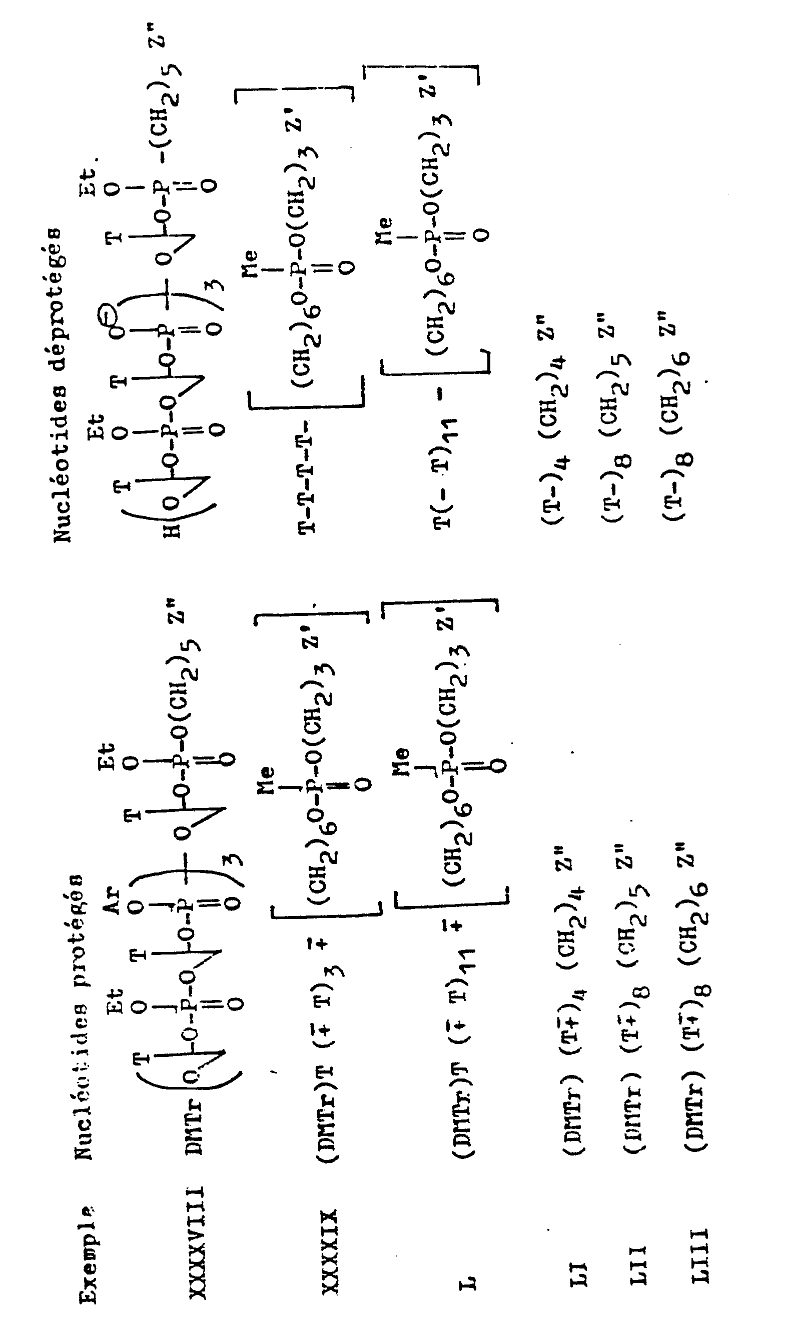

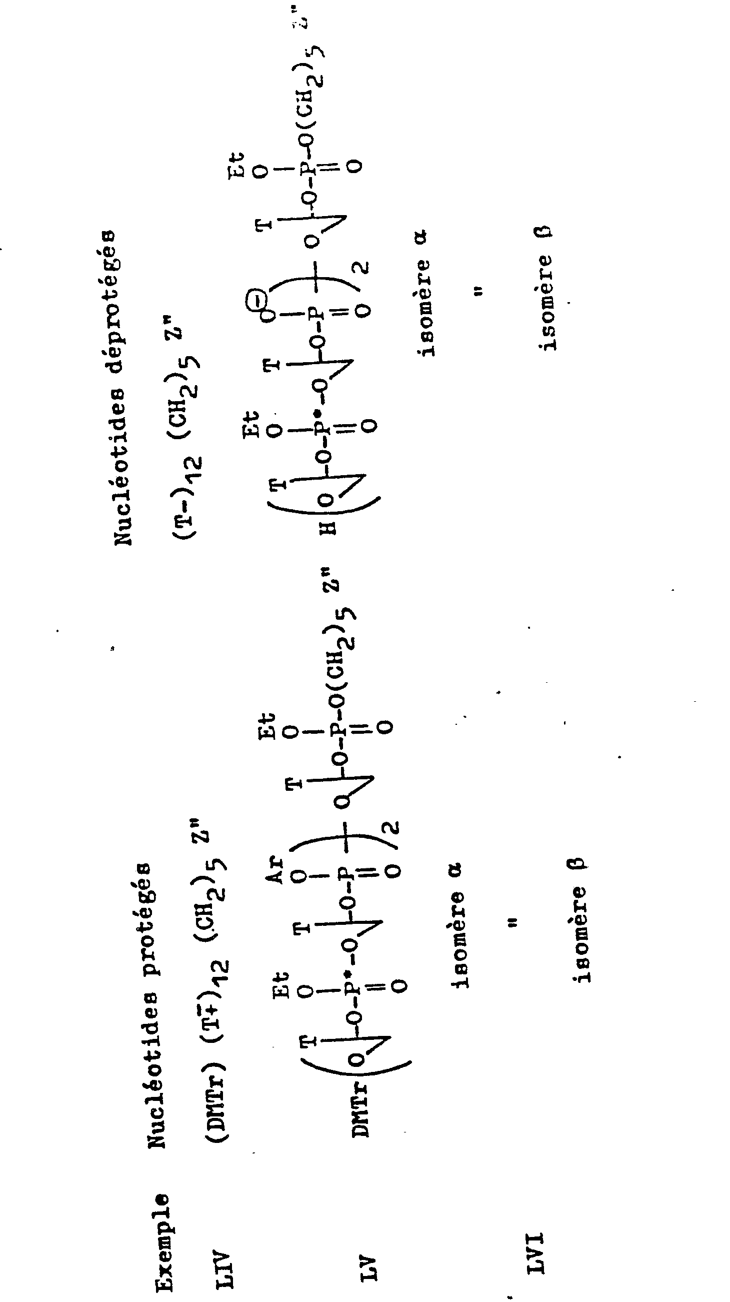

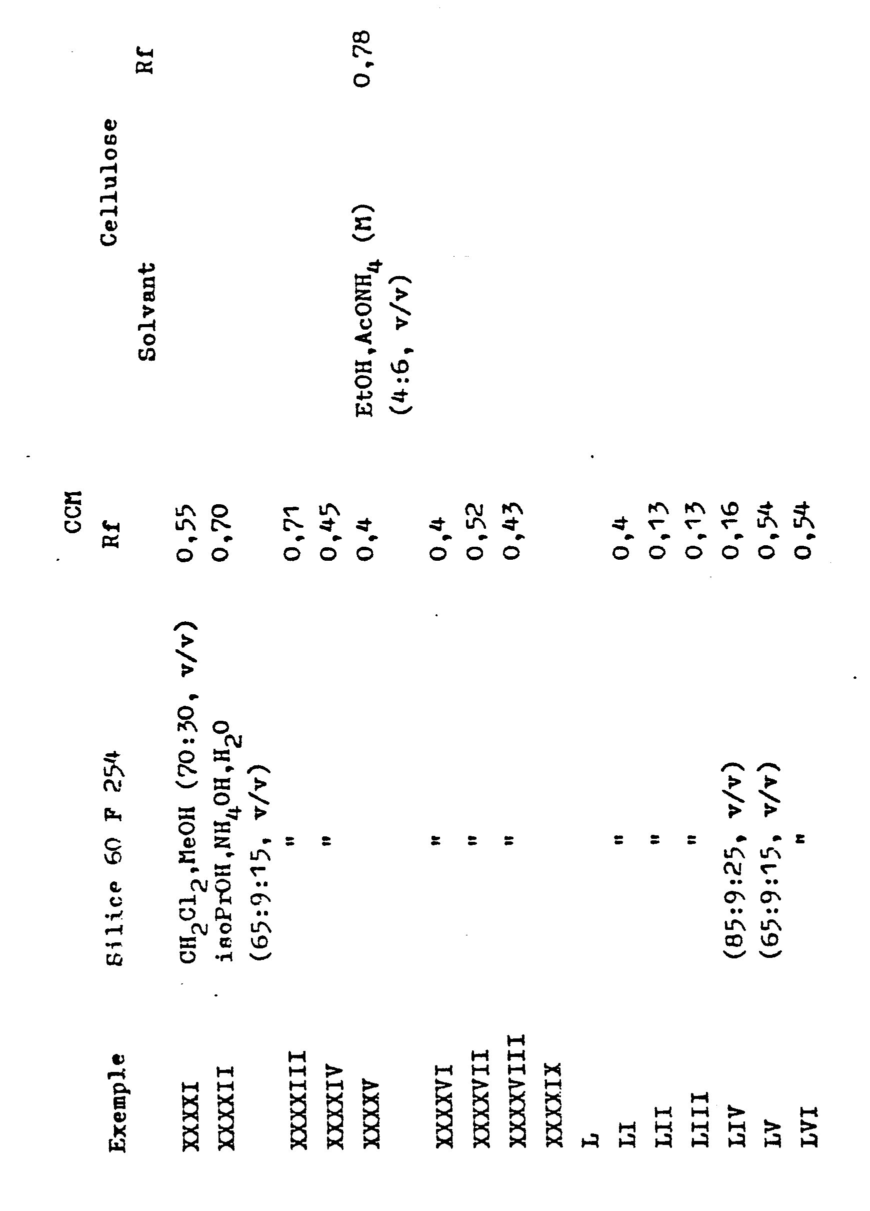

### Exemple LVII

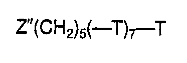

On réalise la déarylation du composé Z"(CH₂)₅(∓T)₇∓T(Bz) selon l'exemple XXXVIII ; le résidu obtenu après évaporation du solvant est traité pendant 45 minutes avec la soude aqueuse (0,25 M) à la température ambiante. Après avoir neutralisé le mélange réactionnel avec une résine anionique sous forme de pyridinium, on purifie le produit sur DEAE Séphacel [gradient d'élution 10-³ M―0,9 M en NH₄HC0₃ dans H₂0, MeOH (70 : 30, v/v)].
CCM : gel de silice ; isoPrOH, NH₄0H, H₂0 (65 : 9 : 15, v/v), Rf - 0,13.

### Exemple LVIII

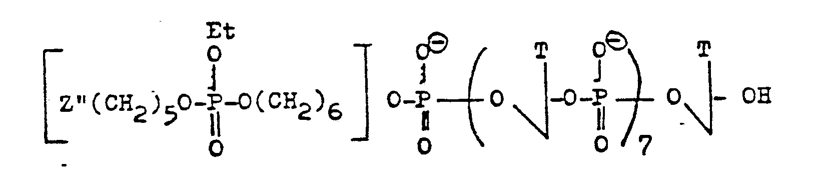

En traitant de l'octanucléotide préparé selon l'exemple XXVI et en opérant comme dans l'exemple LVII, on a obtenu le produit.
CCM : gel de silice ; isoPrOH, NH₄0H, H₂0 (65 : 9 :15, v/v), Rf - 0,13.

### Exemple LIX

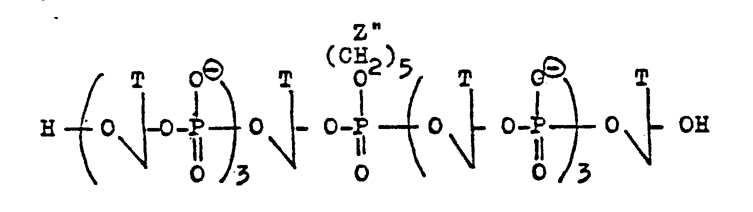

On réalise la déarylation du composé de l'exemple XXIX selon l'exemple XXXVIII, le résidu obtenu après évaporation du solvant est traité pendant 45 minutes avec la soude aqueuse (0,25 M) à la température ambiante. Après avoir neutralisé le mélange réactionnel avec une résine anionique (forme pyridinium) puis chassé le solvant sous vide, on traite ensuite le résidu avec l'acide acétique à 80 % puis termine la préparation comme dans les exemples XXXXI à LVI.
CCM : gel de silice ; isoPrOH, NH₄0H, H₂0 (65 : 9 : 15, v/v), Rf = 0,3.

### Exemple LX

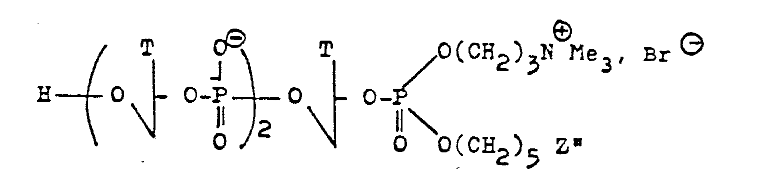

On réalise la déarylation du composé 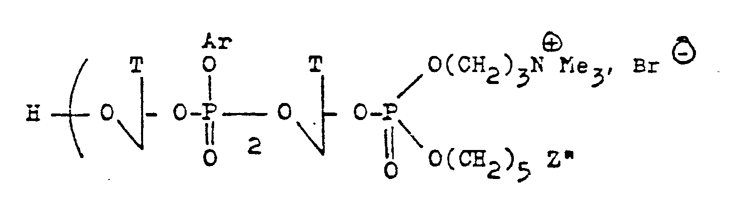 (exemple XXX) comme dans l'exemple XXXVIII. Le produit est ensuite purifié sur Séphadex G25 [solvant : H₂0, MeOH (85 : 15, v/v)].
CCM : gel de silice ; isoPrOH, NH₄0H, H₂0 (65 : 9 : 15, v/v), Rf = 0,27.
cellulose : EtOH, AcONH₄(M) (4 : 1, v/v), Rf = 0,51.

### Exemple LXI

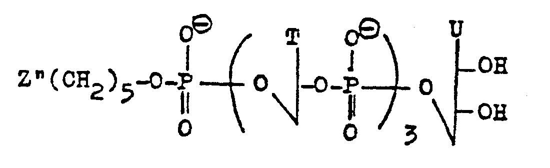

Le nucléotide totalement protégé (1 équivalent) (exemple XXXI) est traité pendant 50 minutes à 10 °C avec une solution à 2 % d'acide benzènesulfonique dans CH₂CI₂, MeOH, H₂0 (7 : 2,5 : 0,5, v/v). Le mélange est dilué avec du chloroforme, lavé avec une solution aqueuse de NH₄HC0₃ puis séché et concentre sous vide. Le résidu obtenu est traité avec une solution molaire de 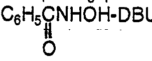 J dans la pyridine (30 équivalents), (2 jours à la température ambiante), puis, avec de la soude (concentration en soude de la solution = 0,1 N), (45 minutes à la température ambiante). Après neutralisation du milieu réactionnel avec du DOWEX 50 (forme pyridinium), puis évaporation du solvant, on purifie le produit sur DEAE Séphacel en utilisant le gradient d'élution (10⁻³ M-0,5 M) en NH₄HC0₃ dans H₂0, MeOH (70 : 30, v/v).
CCM : gel de silice ; isoPrOH, NH₄0H, H₂0 (65 : 9 15, v/v), Rf = 0,38.

### Exemple LXII

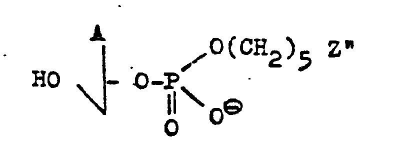

Le nucléotide (DMTr) d∓ bzA ∓ (CH₂)₅Z" (1 équivalent) est traité pendant une nuit à la température ambiante avec une solution molaire de 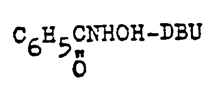 dans la pyridine (10 équivalents). A cette solution, on ajoute 2 volumes de solution de soude molaire MeOH, H₂0 (2 : 1, v/v) puis continue l'agitation pendant 1 jour à la température ambiante. Après avoir neutralisé le mélange réactionnel avec une résine anionique (forme pyridinium), on chasse le solvant sous vide puis traite le résidu avec l'acide acétique à 80 %. Le produit est ensuite purifié sur gel de silice (plaque préparative) solvant ; CH₂Cl₂, MeOH (50 : 50, v/v).
CCM : gel de silice ; CH₂Cl₂, MeOH (50 : 50, v/v), Rf = 0,33.

### Exemples LXIII et LXIV

On réalise la déprotection des composés XXXIII et XXXIV comme dans l'exemple LXII puis purifie les produits par chromatographie sur plaque. 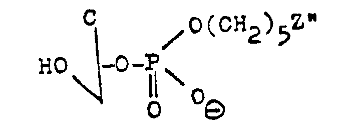 Gel de silice ; CH₂CI₂, MeOH (50 : 50, v/v
CCM : gel de silice ; CH₂Cl₂, MeOH (50 : 50. v/v), Rf = 0,43 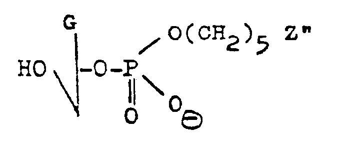
Gel de silice ; EtOH, AcONH₄(M) (8 : 2, v/v).

### Exemples LVI à LXVII

Les oligonucléotides (XXXV, XXXVI, XXXVII) sont déprotégés comme dans l'exemple LXII en utilisant 10 équivalents de

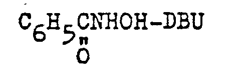

par équivalent de phosphoester arylé à déprôtéger ; les produits sont ensuite purifiés sur DEAE Séphacel® en utilisant des gradients d'élution en NH₄HC0₃ dans H₂0, MeOH (70 30, v/v) (10⁻³ M à 0,7 M pour LXV, 0,1 M à 0,9 M pour LXVI et 0,1 M à 1 M pour LXVII).

### (Voir Schéma page 33)

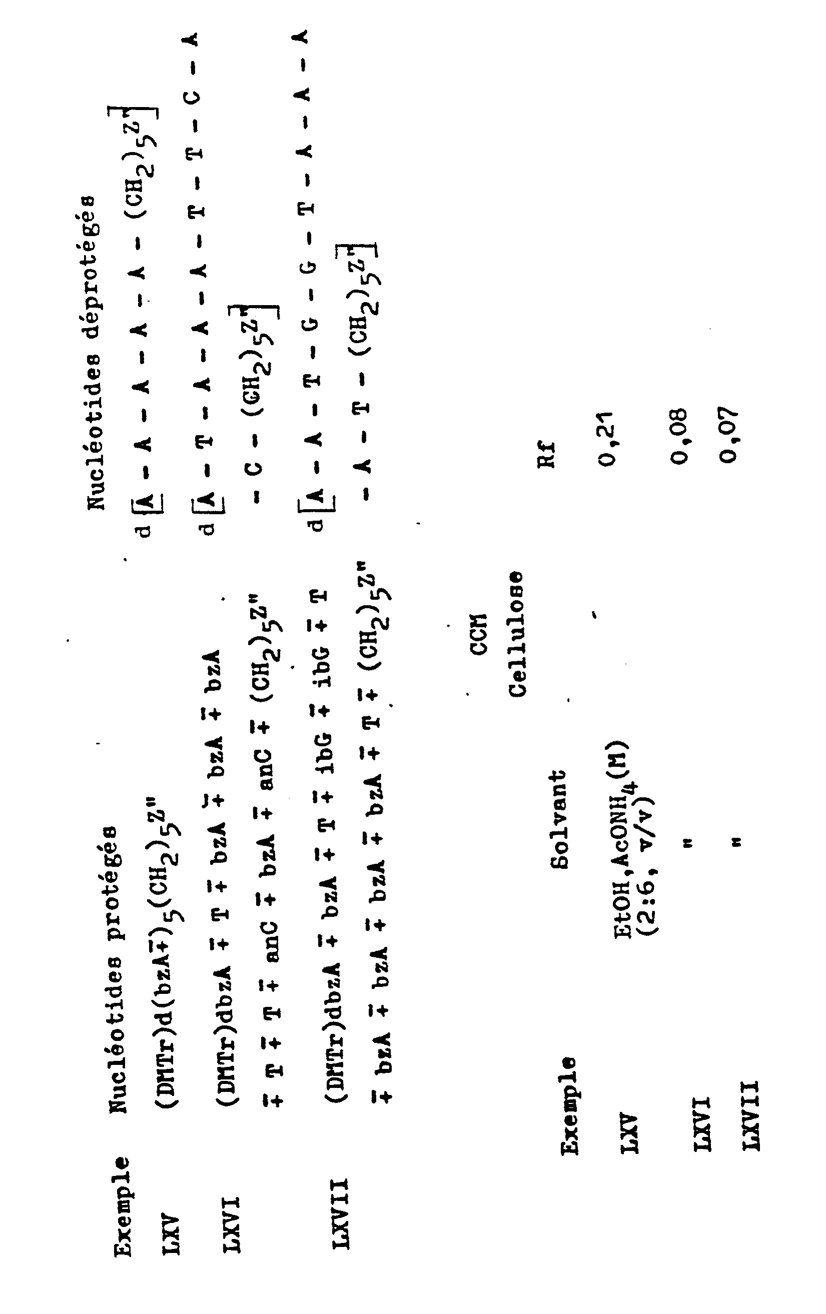

### Exemple LXVIII

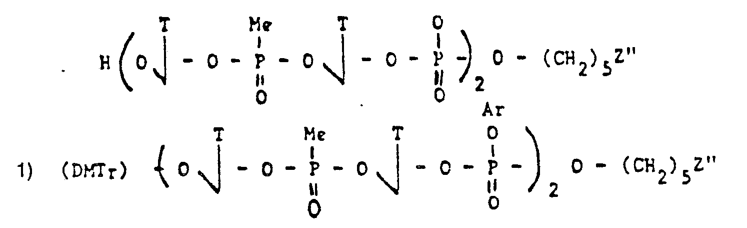

On opère comme dans l'exemple VI en remplaçant le dinucléotide (DMT) T±T±CNEt par le composé préparé selon l'exemple XV et le (w-hydroxypropyloxy)-8-psoralène par le méthoxy-2-chloro-6-(w-hydroxypentylamino)-9-acridine ; on obtient le produit protégé avec un rendement de 60 %

2) La déprototection a été réalisée comme dans les exemples XXXXI à LVI ; le produit est ensuite purifié par HPLC [colonne Lichrosorb" RP-18 (Merck) ; solvant : CH₃CN, solution aqueuse d'acétate de triéthylammonium à 10 % en poids (pH = 5,5), eau 216 : 79 : 713, v/v), débit 1,2 ml/mn], temps de rétention = 7', 40

### Exemples LXIX et LXX

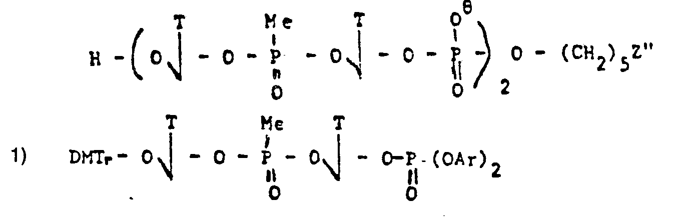

On réalise le couplage du nucléoside-3' méthyl-phosphate XV-1 (1,5 équivalents) avec la thymidine-3' phosphate de di(p-chlorophényle (1 équivalent) en présence de MST (3 équivalent) puis on-sépare les isomères α et p par chromatographie sur silice
CCM : gel,de silice ; CH₂CI₂, MeOH (9 : 1, v/v)
isomère α: Rf = 0,63
isomère β: Rf = 0,58

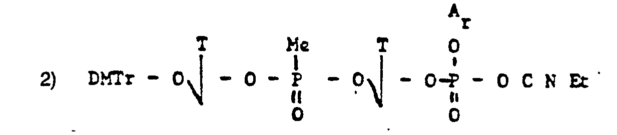

a) Isomère a

On fait réagir l'isomère a préparé selon les exemples LXIX et LXX-1 avec une solution de pyridine-triéthylamine-eau (3 : 1 : 1, v/v) pendant 6 h à 40 °C ; le diester formé est ensuite purifié par chromatographie sur silice en utilisant le système de solvant : CH₂Cl₂-MeOH-pyridine (85: 15 : 0,4, v/v). Le diester obtenu (1 équivalent) est couplé avec le β-cyanoéthanol (2 équivalents) en présence de MST (3 équivalents) ; on termine ensuite la préparation comme dans l'exemple l-2

isomère a : Rf = 0,55 et 0,52 [CCM : gel de silice ; CHC1₃, MeOH (9 : 1, v/v)]

b) Isomère β

A partir de l'isomère β préparé selon les exemples LXIX et LXX-1 et en opérant comme précédemment, on obtient l'isomère β
isomère β: Rf = 0,49 et 0,45 CCM : gel de silice ; CHCl₂, MeOH (9 : 1, v/v).

3) A partir de chacun des isomères α et β des exemples LXIX et LXX-2, on opère comme dans l'exemple LXVIII ; on obtient respectivement les tétranucléotides sous forme d'isomère a et d'isomère p.

HPLC [colonne Lichrosorb RP-18 (Merck) ; solvant : CH₃CN, solution aqueuse d'acétate de triéthylammonium à 10 % en poids (pH = 5,9), eau (216 : 79 : 713, v/v débit 1,2 ml/min].
LXIX isomère a temps de rétention = 7'30"
LXX isomère β temps de rétention = 7'46"

### Exemples LXXI à LXXXVII

En opérant comme dans les exemples XXXV à XXXVII, on prépare les oligodésoxynucléotides totalement protégés par couplage de blocs selon les schémas donnés ci-après puis on élimine les groupements protecteurs comme pour les exemples LXV à LXIII ; les oligonucléotides déprotégés LXXI à LXXVII sont ensuite purifiés par HPLC.

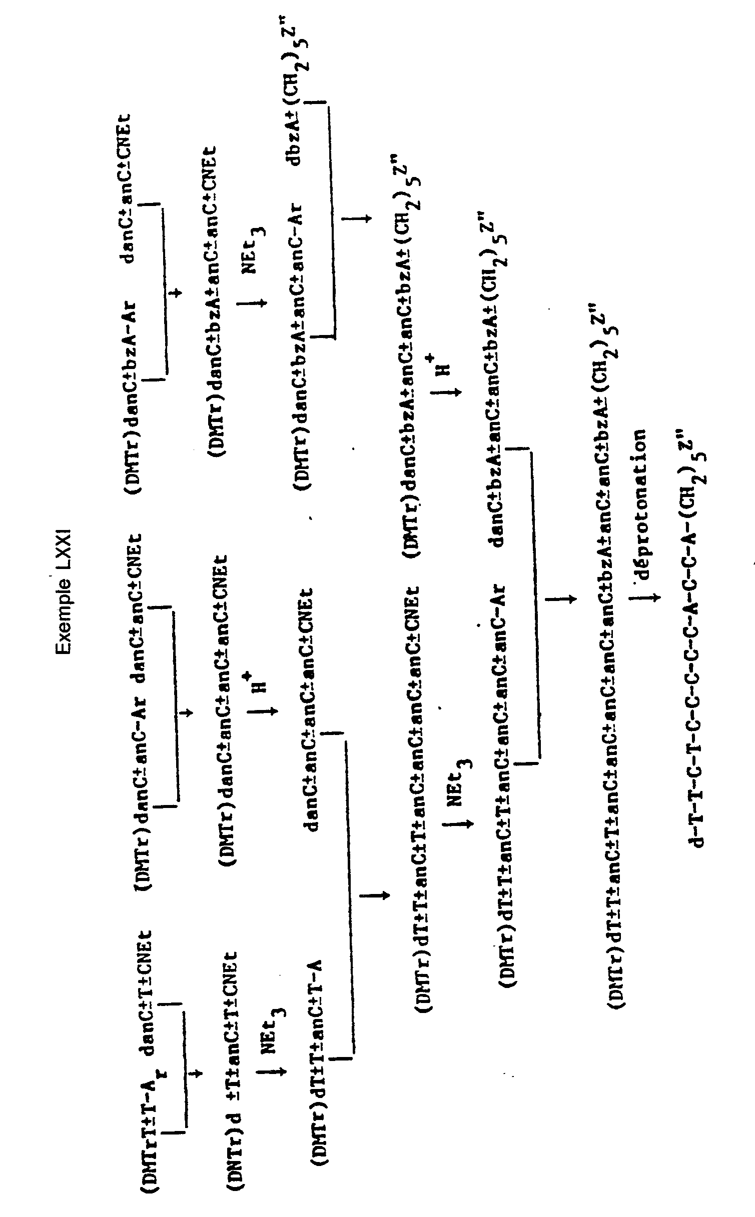

### Exemple LXXII

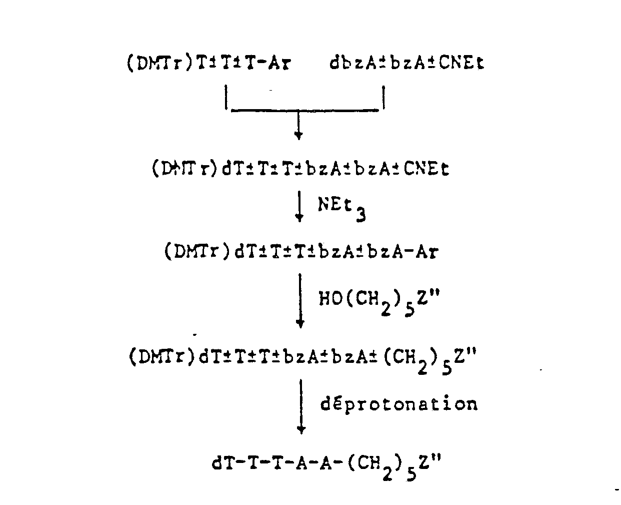

### Exemple LXXIII

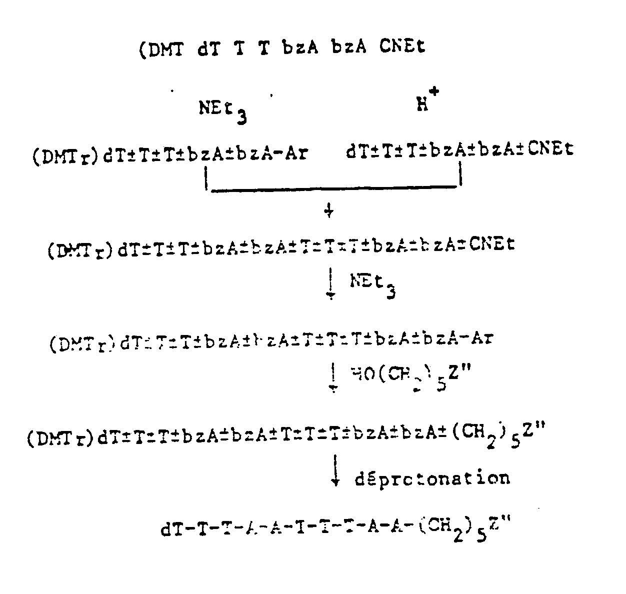

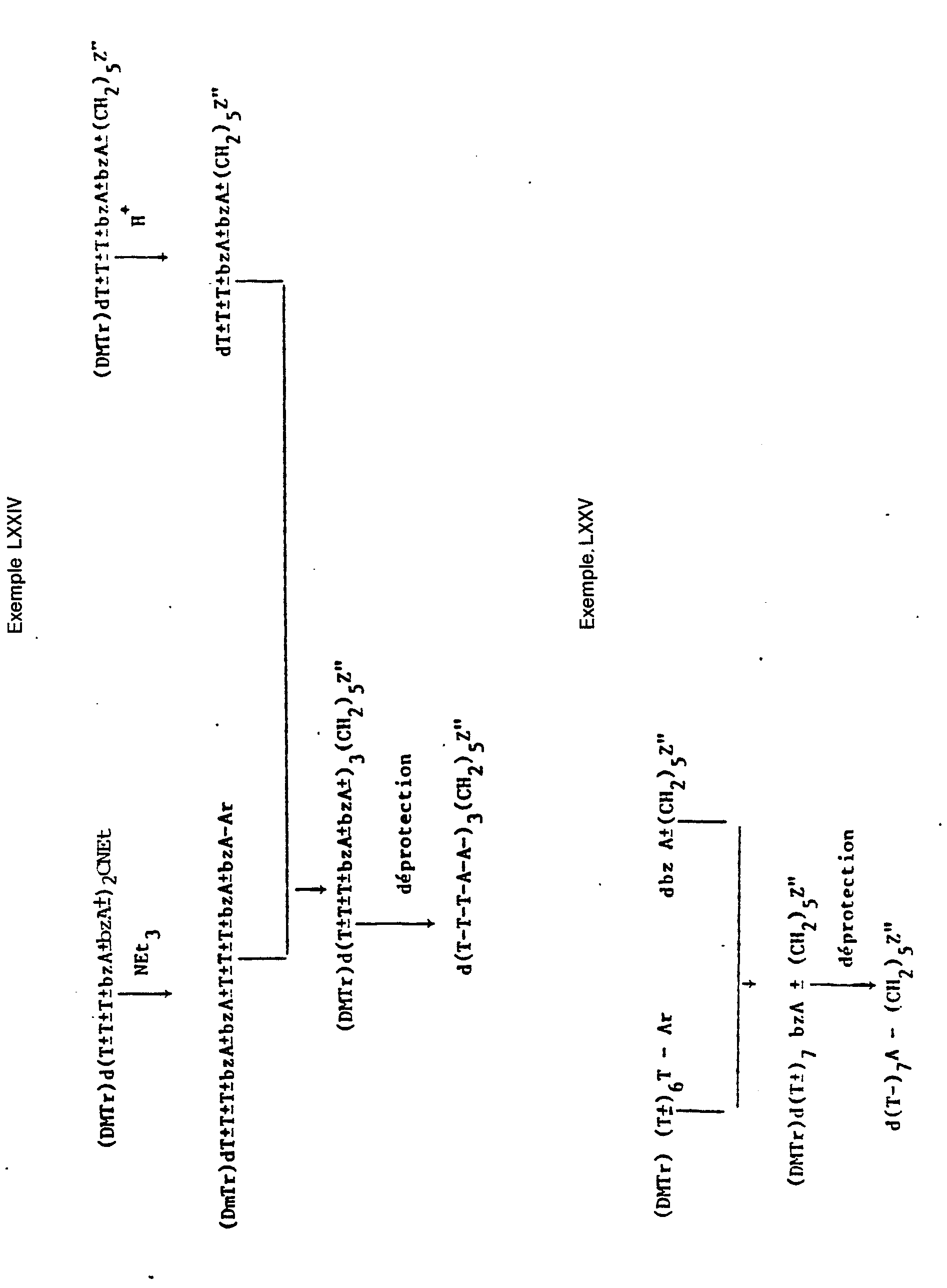

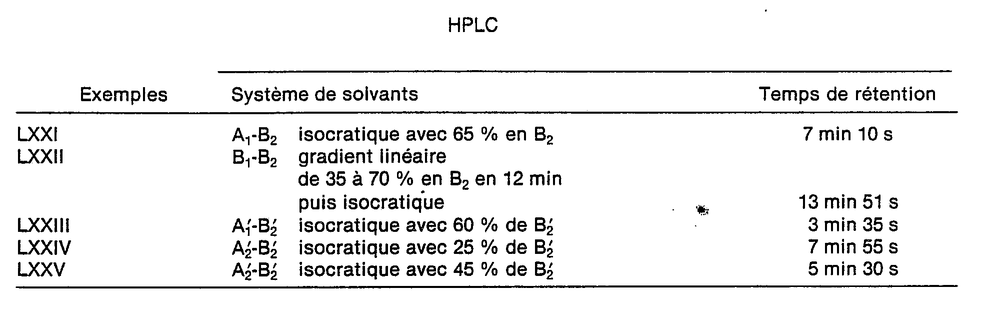

Colonne Lichrosorb RP-18 (Merck) ; débit : 1,2 ml/min
A₁: CH₃CN, TEAc, eau (18: 88: 794, v/v) ; A'₁: CH₃CN, AAc, eau (18: 88: 794, v/v)
B₁: CH₃CN, TEAc, eau (108 : 79 : 713, v/v) ; B₂: CH₃CN, TEAc, eau (216 : 79 : 713, v/v) B'₂ : CH₃CN, AAc, eau (216 79 : 713, v/v)
AAc : solution aqueuse d'acétate d'ammonium à 10 % en poids (pH = 5,9)
TEAc : solution aqueuse d'acétate de triéthylammonium à 10 % en poids (pH = 5,9)

## Revendications (Revendications pour l'(les) Etat(s) contractant(s) suivant(s) : BE, CH, DE, FR, GB, IT, LI, LU, NL, SE)

1. Composé de formule : 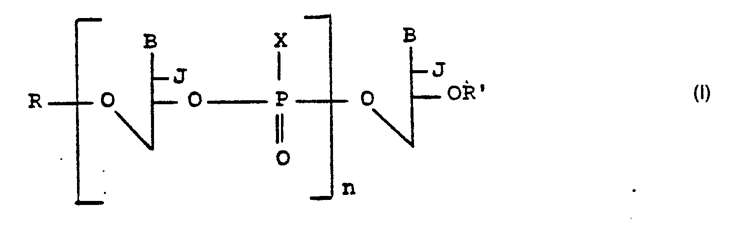 dans laquelle :
les radicaux B peuvent être identiques ou différents et représentent chacun une base d'un acide nucléique naturelle ou modifiée ;
les radicaux X, qui peuvent être identiques ou différents, représentent chacun un oxoanion 0⁼, un thioanion S^{e}, un groupe alkyle, un groupe alcoxy, un groupe aminoalkyle, un groupe aminoalcoxy, un groupe thioalkyle ou un groupement ―Y―Z;
R et R', qui peuvent être identiques ou différents, représentent chacun un atome d'hydrogène ou un groupement ―Y―Z;
Y représente un radical alcoylène droit ou ramifié -alk- ou un radical 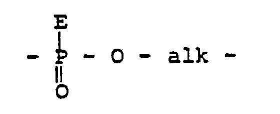 ou bien un radical ―Y"―0―Y' où Y" ou Y' peuvent avoir les significations données pour Y ;
E peut avoir les mêmes significations que X ;
J représente un atome d'hydrogène ou un groupe hydroxy ;
Z est un radical correspondant à un agent d'intercalation et l'un au moins des groupes X, R, R' ou E est un groupement -Y-Z ;
n est nombre entier y compris 0 ; ainsi que les sels et les mélanges d'isomères ou les isomères séparés.

2. Composé selon la revendication 1, caractérisé en ce que l'agent d'intercalation Z est choisi parmi les composés polycycliques ayant une configuration plane.

3. Composé selon la revendication 2, caractérisé en ce que Z est choisi parmi l'acridine, la furocoumarine ou l'ellipticine.

4. Composé selon l'une des revendications 1 à 3, caractérisé en ce que le radical B est un radical correspondant à la thymine, l'adénine, la cytosine, la guanine, l'uracile, la 5-bromo-uracile, la 8-azido-adenine 2,6 diaminopurine ou un dérivé photo-activable des bases.

5. Composé selon l'une des revendications 1 à 4, caractérisé en ce que le radical Z est choisi parmi :

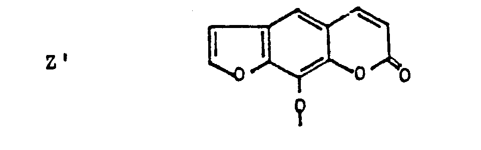

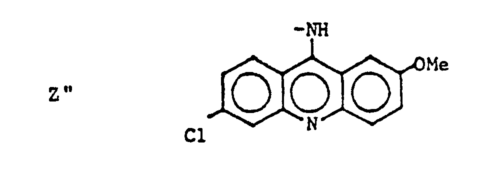

6. Composé selon l'une des revendications 1 à 5, caractérisé en ce que B est choisi parmi T, G, A, C et U ; X est-choisi parmi : O^{e}, méthyle, éthyle, propyle, méthoxy, éthoxy, propoxy et J = H.

7. Composé selon la revendication 1, de formule : 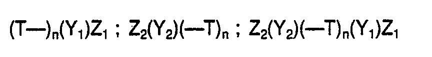 où T est le radical thymidine, n est un nombre entier de 1 à 25 et Y₁ et Y₂ représentent un radical alcoylène en Ci à C₇ ou un radical : 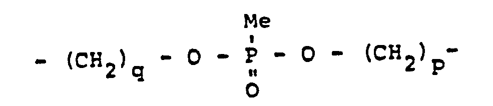 dans laquelle p et q sont des nombres entiers de 1 à 10 et Z₁ et Z₂ ont les significations de Z.

8. Composés selon la revendication 1, de formule :

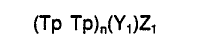

9. Composé selon la revendication 1, de formule : 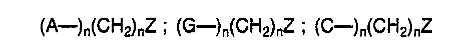 et les composés séquencés tels que les oligonucléotides :
d [A―T―A―A―A―T―T―C―A―C―(CH₂)ₙZ];
d [A―A―T―G―G―T―A―A―A―A―T―(CH₂)ₙZ]
d [T―T―C―T―C―C―C―C―C―A―C―C―A―(CH₂)ₙZ]
d [T―T―T―A―A―(CH₂)ₙZ]
d [T―T―T―A―A―T―T―T―A―A―(CH₂)ₙZ]
d [T―T―T―A―A―T―T―T―A―A―T―T―T―A―A―(CH₂)ₙZ]
d [T―T―T―T―T―T―T―A―(CH₂)ₙZ]

10. Composés selon la revendication 5 de formule :
Z(CH₂)₃∓T∓T∓T∓T∓(CH₂)₃Z'
Z"(CH₂)₅∓T∓T∓T∓T∓(CH₂)₅Z"
Z'(CH₂)₃―T―T―T―T―(CH₂)₃Z'
Z"(CH₂)₅―T―T―T―T―(CH₂)₅Z"
Z"(CH₂)₅―(T―)₈(CH₂)₅Z"
T―(CH₂)₃Z'
T―(CH₂)₃Z"
T―(CH₂)₅Z"
T―T―T―(CH₂)₅Z"
T―T―T―T―(CH₂)₃Z'
T―T―T―T―(CH₂)₅Z" 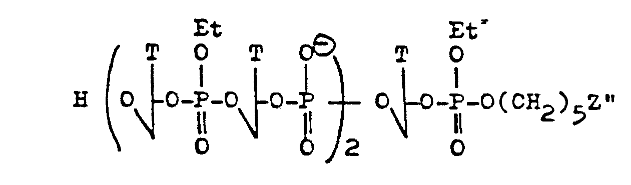 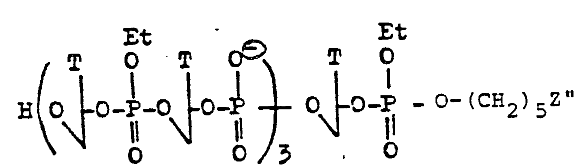 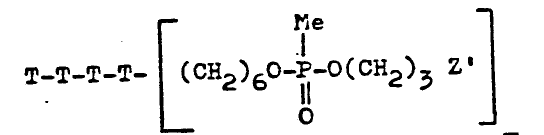 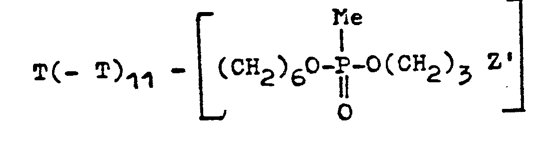
(T―)₄(CH₂)₄Z"
(T―)₈(CH₂)₅Z"
(T―)₈(CH₂)₆Z"
(T―)₁₂(CH₂)₅Z" 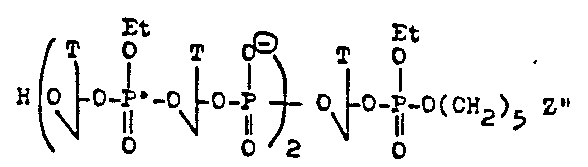 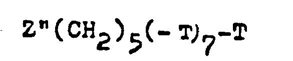 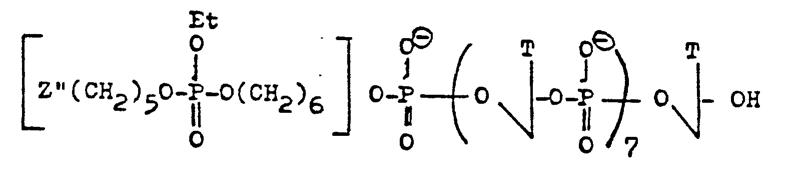 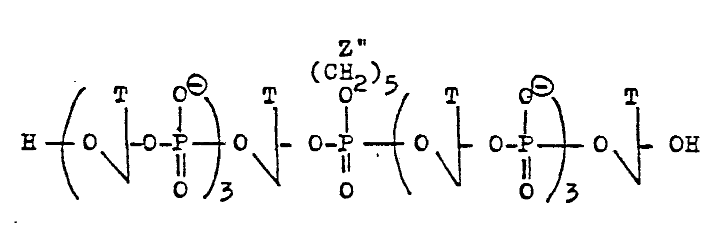 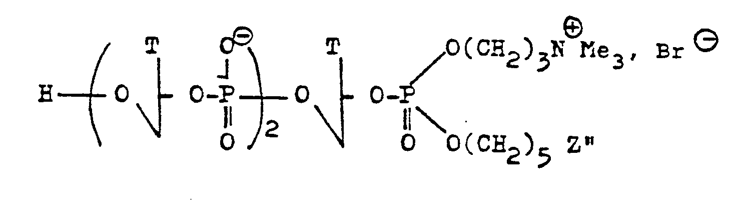 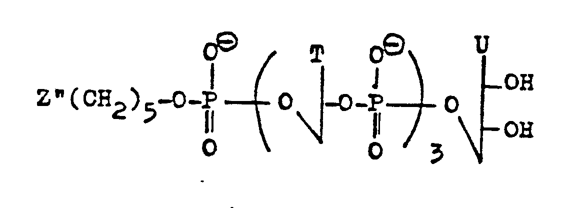 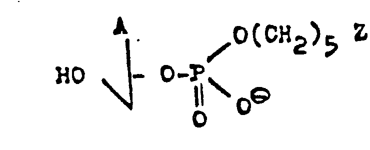 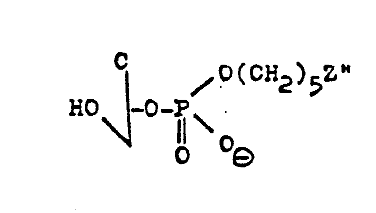 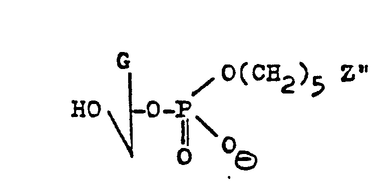
d [A―A―A―A―A―(CH₂)₅Z"]
d [A―T―A―A―A―T―T―C―A―C―(CH₂)₅Z"]
d [A-A T-G-G T-A-A-A-A-T-(CH_{z})₅Z"]
dans lesquels les symbole (+) représente la liaison phosphotriester, chaque phosphate étant protégé par le groupe p-chlorophényle.

11. Procédé de préparation de composés selon l'une des revendications 1 à 10, caractérisé en ce que l'on prépare les dérivés totalement protégés et que l'on élimine les groupements protecteurs.

12. Procédé selon la revendication 11, caractérisé en ce qu'on couple un oligonucléotodide 3'-phosphodiester protégé avec l'hydroxyle de l'agent intercalant ou avec l'hydroxyle-5' d'un oligonucléotide protégé et possédant déjà l'agent d'intercalation.

13. Procédé selon la revendication 11, caractérisé en ce qu'on couple un oligonucléotide 5'-phosphodiester protégé avec l'hydroxyle de l'agent intercalant ou un diester porteur du groupe intercalant avec l'hydroxyle-5' d'un oligonucléotide protégé.

14. Procédé selon la revendication 11, caractérisé en ce qu'on couple l'hydroxyle de l'agent intercalant avec un oligonucléotide-3',5'-bis-(phosphodiester) protégé.

15. Procédé selon la revendication 11, caractérisé en ce qu'on couple un diester porteur de l'agent intercalant avec l'hydroxy-5' d'un oligonucléotide protégé et possédant déjà l'agent d'intercalation.

16. Procédé selon la revendication 11, caractérisé en ce qu'on réalise la déprotection des groupes phosphoesters arylés en milieu anhydre à l'aide d'acide benzohydroxamique et en présence, de base tertiaire non nucléophile.

17. Application des composés selon l'une des revendications 1 à 10 à titre de sonde pour la détection d'une séquence déterminée d'ADN ou d'ARN.

18. Application des composés selon l'une des revendications 1 à 10 à la purification de séquence déterminée d'ADN ou d'ARN.

19. Application des composés selon l'une des revendications 1 à 10 au diagnostic de mutations dans les ADN et les ARN.

## Revendications (Revendications pour l'(les) Etat(s) contractant(s) suivant(s) : AT)

1. Procédé de préparation d'un composé de formule : 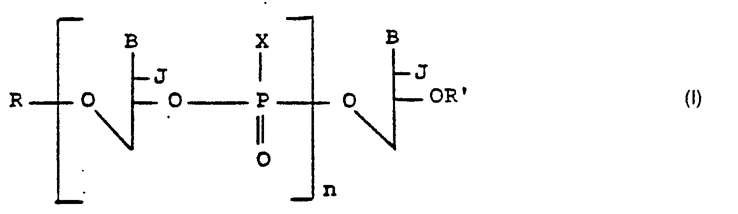 dans laquelle :
les radicaux B peuvent être identiques ou différents et représentent chacun une base d'un acide nucléique naturelle ou modifiée ;
les radicaux X, qui peuvent être identiques ou différents, représentent chacun un oxoanion 0³, un thioanion S^{b}, un groupe alkyle, un groupe alcoxy, un groupe aminoalkyle, un groupe aminoalcoxy, un groupe thioalkyle ou un groupement γ-Z ;
R et R', qui peuvent être identiques ou différents, représentent chacun un atome d'hydrogène ou un groupement -Y-Z;
Y représente un radical alcoylène droit ou ramifié ―atk― ou un radical E 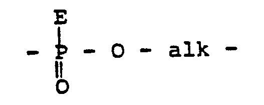 ou bien un radical ―Y"―0―Y'― où Y" ou Y' peuvent avoir les significations données pour Y ;
E peut avoir les mêmes significations que X ;
J représente un atome d'hydrogène ou un groupe hydroxy ;
Z est un radical correspondant à un agent d'intercalation et l'un au moins des groupes X, R, R' ou E est un groupement ―Y―Z;
n est un nombre entier y compris 0 ; ainsi que les sels et les mélanges d'isomères ou les isomères séparés, procédé dans lequel on prépare les dérivés totalement protégés et on élimine les groupements protecteurs.

2. Procédé selon la revendication 1, caractérisé en ce qu'on couple un oligonuclétodide 3'-phosphodiester protégé avec l'hydroxyle de l'agent intercalant ou avec l'hydroxyle-5' d'un oligonucléotide protégé et possédant déjà l'agent d'intercalation.

3. Procédé selon la revendication 1, caractérisé en ce qu'on couple un oligonucléotide 5'-phosphodiester protégé avec l'hydroxyle de l'agent intercalant ou un diester porteur du groupe intercalant avec l'hydroxyle-5' d'un oligonucléotide protégé.

4. Procédé selon la revendication 1, caractérisé en ce qu'on couple l'hydroxyle de l'agent intercalant avec un oligonucléotide-3',5'-bis-(phosphodiester) protégé.

5. Procédé selon la revendication 1, caractérisé en ce qu'on couple un diester porteur de l'agent intercalant avec l'hydroxy-5' d'un oligonucléotide protégé et possédant déjà l'agent d'intercalation.

6. Procédé selon la revendication 1, caractérisé en ce qu'on réalise la déprotection des groupes phosphoesters arylés en milieu anhydre à l'aide d'acide benzohydroxamique et en présence de base tertiaire non nucléophile.

7. Procédé selon l'une des revendications 1 à 6, caractérisé en ce que l'agent d'intercalation Z est choisi parmi les composés polycycliques ayant une configuration plane.

8. Procédé selon la revendication 7, caractérisé en ce que Z est choisi parmi l'acridine, la furocoumarine ou l'ellipticine.

9. Procédé selon l'une des revendications 1 à 8, caractérisé en ce que le radical B est un radical correspondant à la thymine, l'adénine, la cytosine, la guanine, l'uracile, la 5-bromo-uracile, la 8-azido-adenine 2,6 diaminopurine ou un dérivé photo-activable des bases.

10. Procédé selon l'une des revendications 1 à 9, caractérisé en ce que le radical Z est choisi parmi :

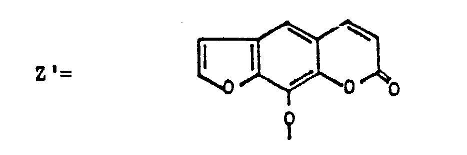

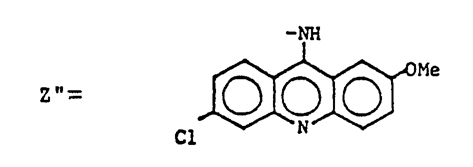

11. Procédé selon l'une des revendications 1 à 10, caractérisé en ce que B est choisi parmi T, G, A, C et U ; X est choisi parmi : O^{e}, méthyle, éthyle, propyle, méthoxy, éthoxy, propoxy et J = H.

12. Procédé de préparation d'un composé selon l'une des revendications 1 à 11 de formule : 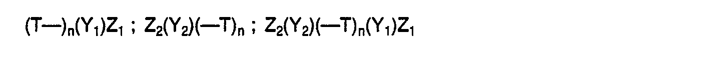 où T est le radical thymidine, n est un nombre entier de 1 à 25 et Y₁ et Y₂ représentent un radical alcoylène en Ci à C₇ ou un radical : 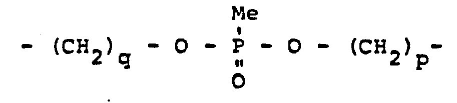 dans laquelle p et q sont des nombres entiers de 1 à 10 et Z₁ et Z₂ ont les significations de Z.

13. Procédé de préparation de composé selon l'une des revendications 1 à 12 de formule :

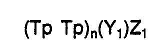

14. Procédé de préparation de composés selon l'une des revendications 1 à 13 de formules : 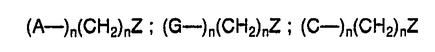 et. des composés séquencés tels que les oligonuclétotides :
d [A―T―A―A―A―T―T―C―A―C―(CH₂)ₙZ] ;
d [A―A―T―G―G―T―A―A―A―A―T―(CH₂)ₙZ]
d [T―T―C―T―C―C―C―C―C―A―C―C―A―(CH₂)ₙZ]
d [T―T―T―A―A―(CH₂)ₙZ]
d [T―T―T―A―A―T―T―T―A―A―(CH₂)ₙZ]
d [T―T―T―A―A―T―T―T―A―A―T―T―T―A―A―(CH₂)ₙZ]
d [T―T―T―T―T―T―T―A―(CH₂)ₙZ]

.15. Procédé de préparation de composés selon l'une des revendications 1 à 14 de formules :
Z'(CH₂)₃∓T∓T∓T∓T∓(CH₂)₃Z'
Z"(CH₂)₅∓T∓T∓T∓T∓(CH₂)₅Z"
Z'(CH₂)₃―T―T―T―T―(CH₂)₃Z'
Z"(CH₂)₅―T―T―T―T―(CH₂)₅Z"
Z"(CH₂)₅―(T―)₈(CH₂)₅Z"
T―(CH₂)₃Z'
T―(CH₂)₃Z"
T-(CH₂)₅Z"
T―T―T―(CH₂)₅Z"
T―T―T―T―(CH₂)₃Z'
T―T―T―T―(CH₂)₅Z" 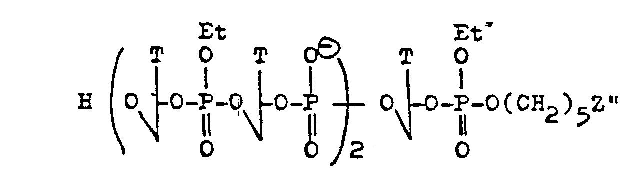 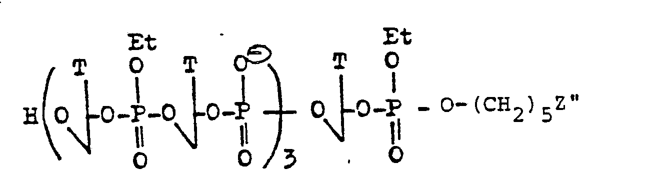 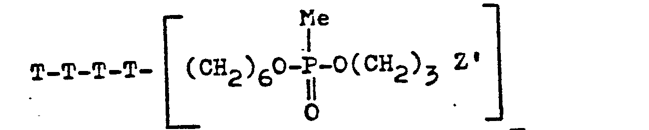 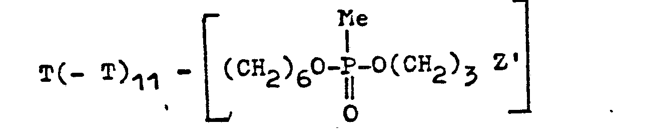
(T―)₄(CH₂)₄Z"
(T―)₈(CH₂)₅Z"
(T―)₈(CH₂)₆Z"
(T―)₁₂(CH₂)₅Z" 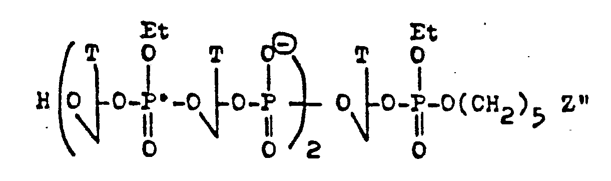 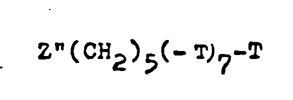 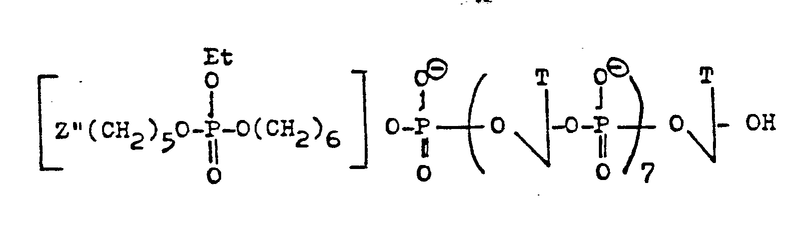 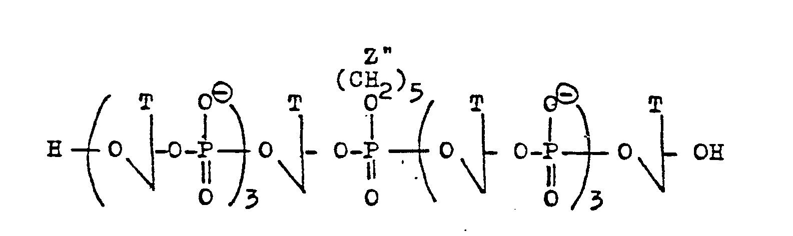 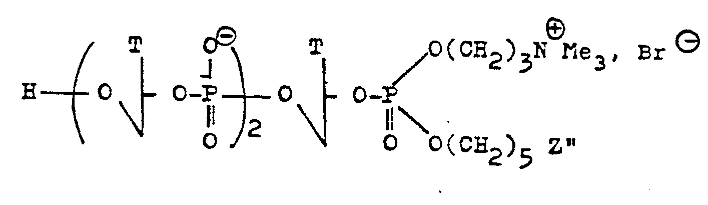 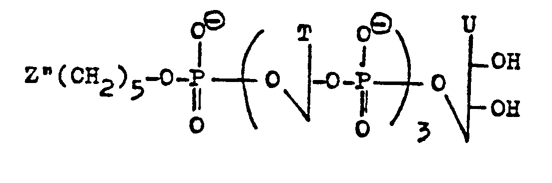 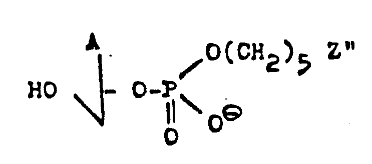 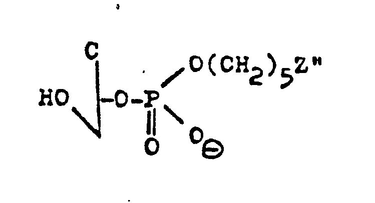 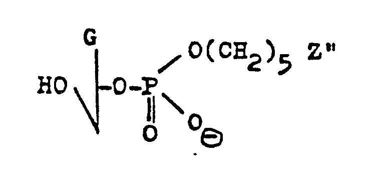
d [A―A―A―A―A―(CH₂)₅Z"]
d [A―T―A―A―A―T―T―C―A―C―(CH₂)₅Z"]
d [A―A―T―G―G―T―A―A―A―A―T―(CH₂)₅Z"]
dans lesquels le symbole (∓) représente la liaison phosphotriester, chaque phosphate étant protégé par le groupe p-chlorophényle.

## Claims (Claims for the following Contracting State(s) : BE, CH, DE, FR, GB, IT, LI, LU, NL, SE)

1. A compound of the formula : 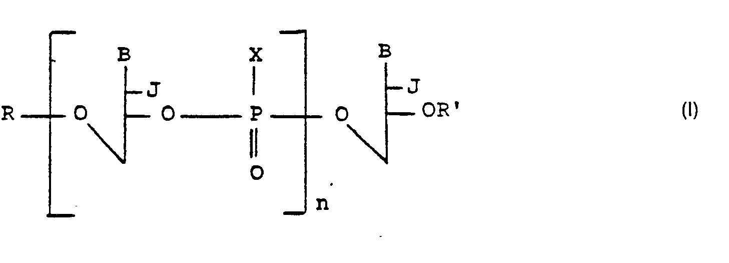 in which :
the radicals B can be identical or different and each represent a base of a natural or modified nucleic acid ;
the radicals X, which can be identical or different, each represent an oxoanion 0^{e}, a thioanion S^{e}, an alkyl group, an alkoxy group, an aminoalkyl group, an aminoalkoxy group, a thioalkyl group or a grouping -Y-Z ;
R and R', which can be identical or different, each represent a hydrogen atom or a grouping -Y-Z ;
Y represents an alkylene radical -alk-, straight-chain or branched, or a radical 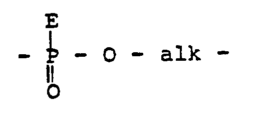 or a radical ―Y"―0―Y'― where Y" or Y' can have the meanings given for Y ;
E can have the same meanings as X ;
J represents a hydrogen atom or a hydroxyl group ;
Z is a radical corresponding to an intercalating agent and one at least of X, R, R' groups or E is ―Y―Z group ;
n is an integer including 0 ; and salts and mixtures of isomers or the separate isomers.

2. A compound as claimed in claim 1, in which the intercalating agent Z is chosen from polycyclic compounds having a flat configuration.

3. A compound as claimed in claim 2, in which Z is chosen from acridine, furocoumarin and ellipticine.

4. A compound as claimed in any one of claims 1 to 3, in which the radical B is a radical corresponding to thymine, adenine, cytosine, guanine, uracil, 5-bromo-uracil, 8-azido-adenine, 2,6-diaminopurine or a photo-activatable derivative of these bases.

5. A compound as claimed in any one of claims 1 to 4, in which the radical Z is chosen from :

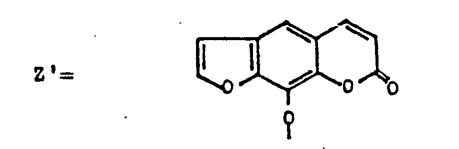

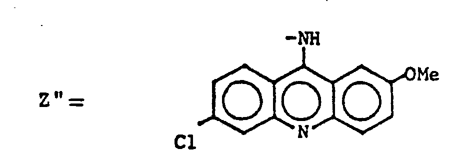

6. A compound as claimed in any one of claims 1 to 5, in which B is chosen T, G, A, C and U ; X is chosen from : 0⁶, methyl, ethyl, propyl, methoxy, ethoxy and propoxy and J = H.

7. A compound as claimed in claim 1, of the formula : 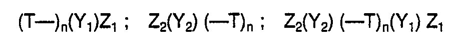 where T is the thymidine radical, n is an integer from 1 to 25 and Y₁ and Y₂ represent the C₁ to C₇- alkylene radical or a radical : 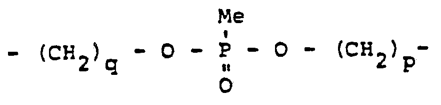 in which p and q are whole numbers from 1 to 10 and Z, and Z₂ have the meanings of Z.

8. A compound as claimed in claim 1, of the formula :

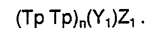

9. A compound as claimed in claim 1, of the formula : 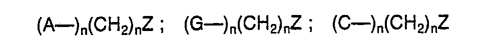 and a sequenced compound, such as one of the oligonucleotides :
d [A―T―A―A―A―T―T―C―A―C―(CH₂)ₙZ] :
d [A―A―T―G―G―T―A―A―A―A―T―(CH₂)ₙZ] .
d [T―T―C―T―C―C―C―C―C―A―C―C―A―(CH₂)ₙZ]
d [T―T―T―A―A―(CH₂)ₙZ]
d [T―T―T―A―A―T―T―T―A―A―(CH₂)ₙZ]
d [T―T―T―A―A―T―T―T―A―A―T―T―T―A―A―(CH₂)ₙZ]
d [T―T―T―T―T―T―T―A―(CH₂)ₙZ]

10. Compounds as claimed in claim 5, of the formula :
Z'(CH₂)₃∓T∓T∓T∓T∓(CH₂)₃Z'
Z"(CH₂)₅∓T∓T∓T∓T∓(CH₂)₅Z"
Z'(CH₂)₃―T―T―T―T―(CH₂)₃Z'
Z"(CH₂)₅―T―T―T―T―(CH₂)₅Z"
Z"(CH₂)₅―(T―)₈(CH₂)₅Z"
T--(CH₂)₃Z
T―(CH₂)₃Z"
T―(CH₂)₅Z"
T―T―T―(CH₂)₅Z"
T―T―T―T―(CH₂)₃Z'
T―T―T―T―(CH₂)₅Z" 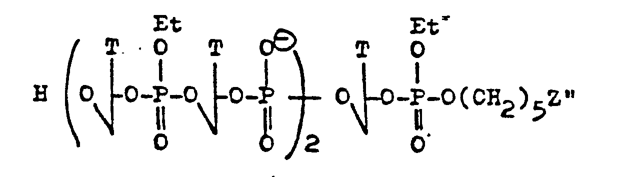 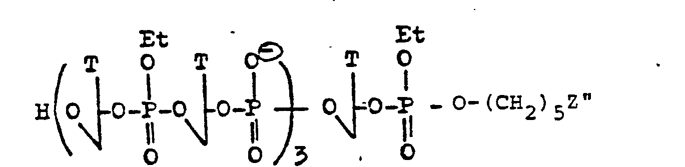 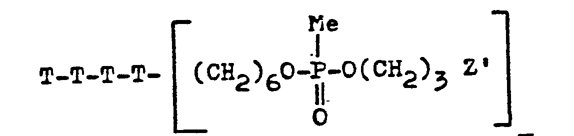 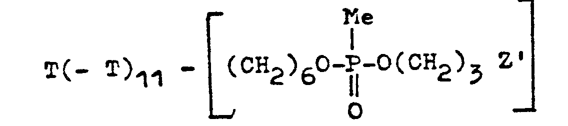
(T―)₄(CH₂)₄Z"
(T―)₈(CH₂)₅Z"
(T―)₈(CH₂)₆Z"
(T―)₁₂(CH₂)₅Z" 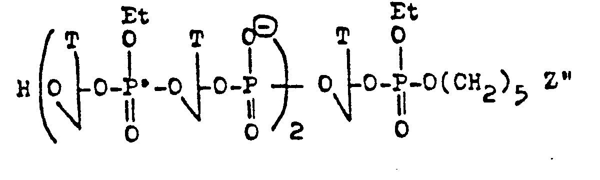 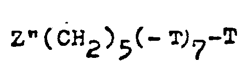 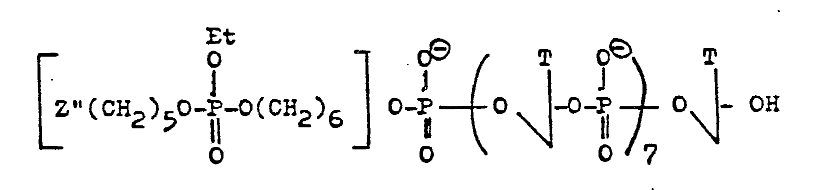 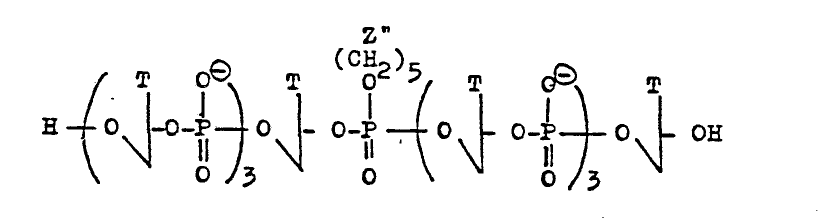 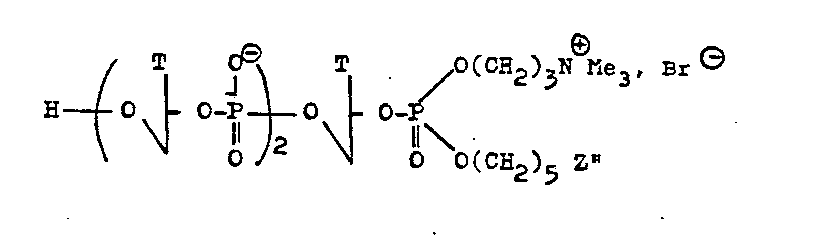 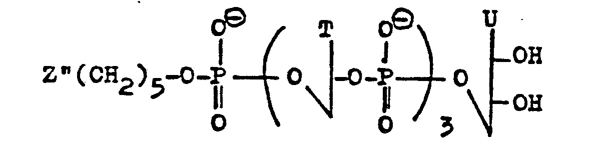 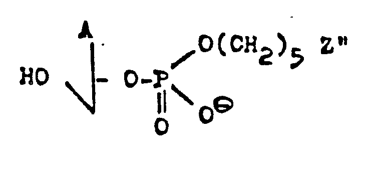 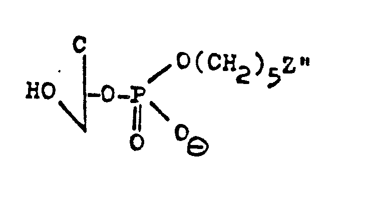 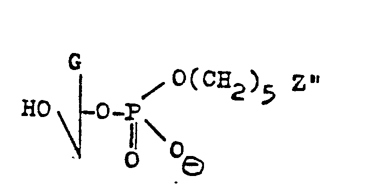
d [A-A-A-A-A-(CH₂)₅Z"l
d [A―T―A―A―A―T―T―C―A―C―(CH₂)₅Z"]
d [A―A―T―G―G―T―A―A―A―A―T―(CH₂)₅Z"]
in which the symbol (∓) represents the phosphotriester bond, each phosphate being protected by the p-chlorophenyl group.

11. A process for the preparation of a compound as claimed in any one of claims 1 to 10, which comprises preparing totally protected derivatives and removing the protective groups.

12. The process as claimed in claim 11, wherein a protected oligonucleotide 3'-phosphodiester is coupled with the hydroxyl of the intercalating agent or with the 5'-hydroxyl of a protected oligonucleotide which already possesses the intercalating. agent.

13. The process as claimed in claim 11, wherein a protected oligonucleotide 5'-phosphodiester is coupled with the hydroxyl of the intercalating agent or a diester carrier of the intercalating group is coupled with the 5'-hydroxyl of a protected oligonucleotide.

14. The process as claimed in claim 11, wherein the hydroxyl of the intercalating agent is coupled with a protected oligonucleotide 3',5'-bis-(phosphodiester).

15. The process as claimed in claim 11, wherein a diester carrier of the intercalating agent is coupled with the 5'-hydroxyl of a protected oligonucleotide which already possesses the intercalating agent.

16. The process as claimed in claim 11, wherein the de-protection of the arylated phosphoester group is carried out in an anhydrous medium with the aid of benzohydroxamic acid and in the presence of a non-nucleophilic tertiary base.

17. The use of a compound as claimed in any one of claims 1 to 10 as a probe for the detection of a definite DNA or RNA sequence.

18. The use of a compound as claimed in any one of claims 1 to 10 for the purification of the definite DNA or RNA sequence.

19. The use of a compound as claimed in any one of claims 1 to 10 for diagnosis of mutations in DNA and RNA.

## Claims (Claims for the following Contracting State(s) : AT)

1. Process for the preparation of a compound of the formula : 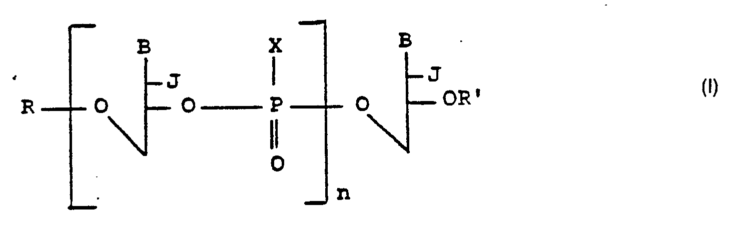 in which :
the radicals B can be identical or different and each represent a base of a natural or modified nucleic acid ;
the radicals X, which can be identical or different, each represent an oxoanion 0^{e}, a thioanion S⁼, an alkyl group, an alkoxy group, an aminoalkyl group, an aminoalkoxy group, a thioalkyl group or a grouping -Y-Z;
R and R', which can be identical or different, each represent an hydrogen atom or a grouping ―Y―Z ;
Y represents an alkylene radical -alk-, straight-chain or branched, or a radical 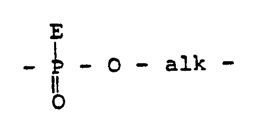 or a radical ―Y"―0―Y'―. where Y" or Y' can have the meanings given for Y ;
E can have the same meanings as X ;
J represents a hydrogen atom or a hydroxyl group ;
Z is a radical corresponding to an intercalating agent and one at least of X, R, R' groups or E is -Y-Z group ;
n is an integer, including 0, and salts and mixtures of isomers or the separate isomers. process which comprises preparing totally protected derivatives and removing the protective groups.

2. The process as claimed in claim 1, wherein a protected oligonucleotide 3'-phosphodiester is coupled with the hydroxyl of the intercalating agent or with the 5'-hydroxyl of a protected oligonucleotide which already possesses the intercalating agent.

3. The process as claimed in claim 1, wherein a protected oligonucleotide 5'-phosphodiester is coupled with the hydroxyl of the intercalating agent or a diester carrier of the intercalating group is coupled with the 5'-hydroxyl of a protected oligonucleotide.

4. The process as claimed in claim 1, wherein the hydroxyl of the intercalating agent is coupled with a protected oligonucleotide 3',5'-bis-(phosphodiester).

5. The process as claimed in claim 1, wherein a diester carrier of the intercalating agent is coupled with the 5'-hydroxyl of a protected oligonucleotide which already possesses the intercalating agent.

6. The process as claimed in claim 1, wherein the de-protection of the arylated phosphoester group is carried out in an anhydrous medium with the aid of benzohydroxamic acid and in the presence of a non-nucleophilic tertiary base.

7. A process as claimed in claims 1 to 6, in which the intercalating agent Z is chosen from polycyclic compounds having a flat configuration.

8. A process as claimed in claim 7, in which Z is chosen from acridine, furocoumarin and ellipticine.

9. A process as claimed in any one of claims 1 to 8, in which the radical B is a radical corresponding to thymine, adenine, cytosine, guanine, uracil, 5-bromo-uracil, 8-azido-adenine, 2,6-diaminopurine or a photo-activatable derivative of these bases.

10. A process as claimed in any one of claims 1 to 9, in which the radical Z is chosen from :

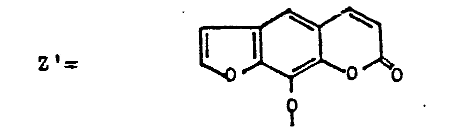

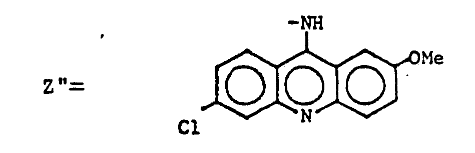

11. A process as claimed in anyone of claims 1 to 10, in which B is chosen from T, G, A, C and U ; X is chosen from : O^{e}, methyl, ethyl, propyl, methoxy, ethoxy and propoxy and J = H.

12. A process as claimed in claims 1 to 11, of the formula : 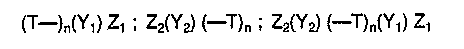 where T is the thymidine radical, n is an integer from 1 to 25 and Y, and Y₂ represent the C₁ to C₇- alkylene radical or a radical : 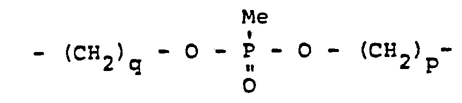 in which p and q are whole numbers from 1 to 10 and Z₁ and Z₂ have the meanings of Z.

13. A process as claimed in claims 1 to 12 of the formula :

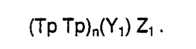

14. A process as claimed in claims 1 to 13 of the formula : 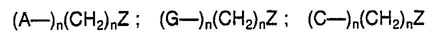 and a sequenced compound, such as one of the oligonucleotides :
d [A―T―A―A―A―T―T―C―A―C―(CH₂)ₙZ] ;
d [A―A―T―G―G―T―A―A―A―A―T―(CH₂)ₙZ].
d [T―T―C―T―C―C―C―C―C―A―C―C―A―(CH₂)nZ]
d [T―T―T―A―A―(CH₂)ₙZ]
d [T―T―T―A―A―T―T―T―A―A―(CH₂)ₙZ]
d [T―T―T―A―A―T―T―T―A―A―T―T―T―A―A―(CH₂)ₙZ]
d [T―T―T―T―T―T―T―A―(CH₂)ₙZ]

15. A process as claimed in claims 1 to 14 of the formula :
Z'(CH₂)₃∓T∓T∓T∓T∓(CH₂)₃Z'
Z"(CH₂)₅∓T∓T∓T∓T∓(CH₂)₅Z"
Z'(CH₂)₃―T―T―T―(CH₂)₃Z' Z"(CH₂)₅―T―T―T―T―(CH₂)₅Z"
Z"(CH₂)₅―(T―)₈(CH₂)₅Z"
T―(CH₂)₃Z^{,}
T―(CH₂)₃Z"
T―(CH₂)₅Z"
T―T―T―(CH₂)₅Z"
T―T―T―T―(CH₂)₃Z'
T―T―T―T―(CH₂)₅Z" 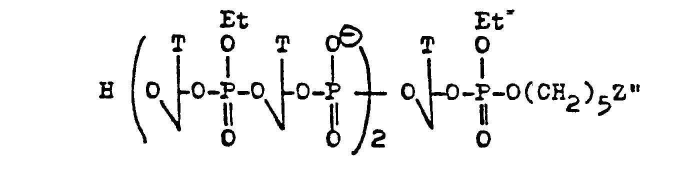 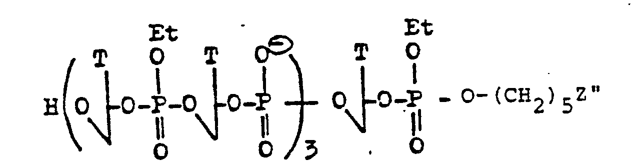 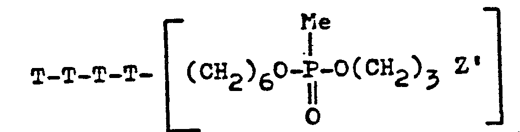 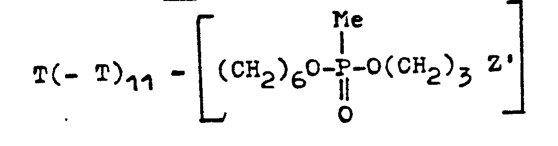
(T―)₄(CH₂)₄Z"
(T―)₈(CH₂)₅Z"
(T―)₈(CH₂)₆Z"
(T―)₁₂(CH₂)₅Z" 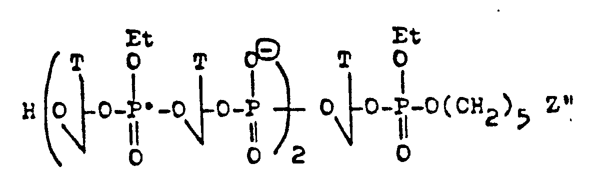 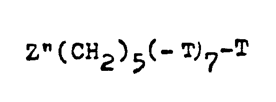 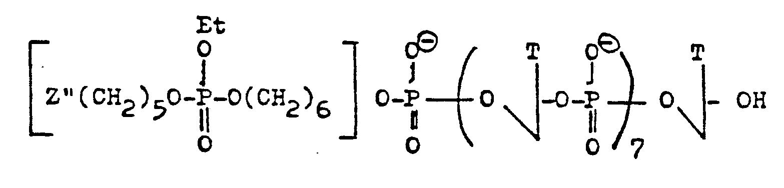 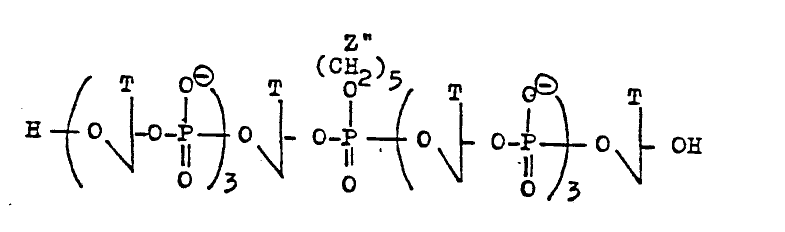 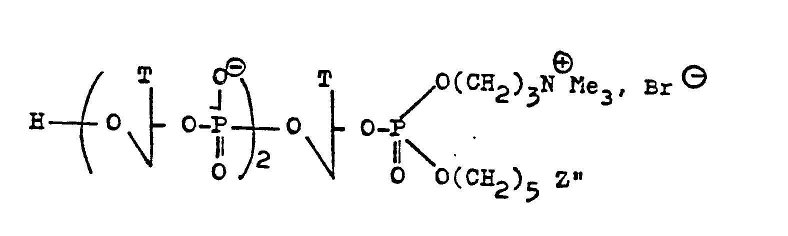 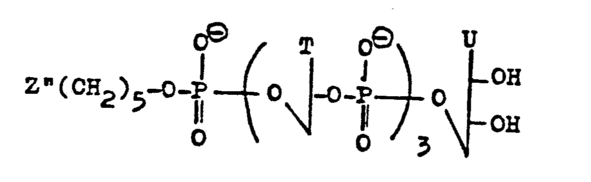 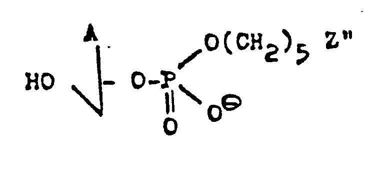 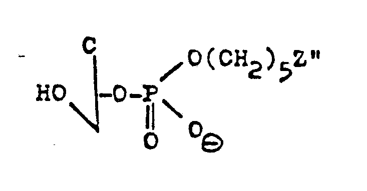 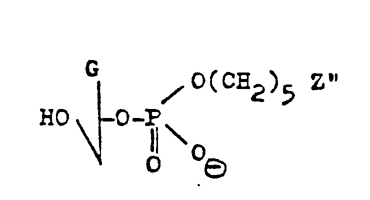
d [A―A―A―A―A―(CH₂)₅Z"]
d [A―T―A―A―A―T―T―C―A―C―(CH₂)₅Z"]
d [A―A―T―G―G―T―A―A―A―A―T―(CH₂)₅Z"]
in which the symbol (∓) represents the phosphotriester bond, each phosphate being protected by the p-chlorophenyl group.

## Patentansprüche (Patentansprüche für folgende(n) Vertragsstaat(en) : BE, CH, DE, FR, GB, IT, LI, LU, NL. SE)

1. Verbindung der Formel : 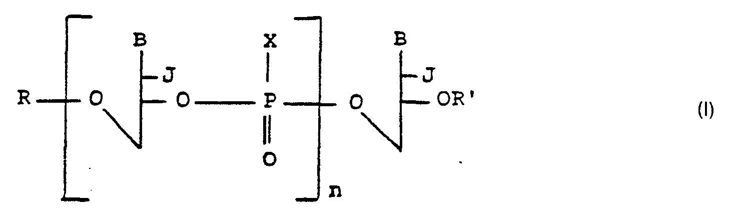 worin bedeuten :
die Reste B, die gleich oder verschieden sein können, jeweils eine Base einer natürlichen oder modifizierten Nukleinsäure ;
die Reste X, die gleich oder verschieden sein können, jeweils ein Sauerstoffanion 0^{e}, ein Schwefelanion S^{e}, eine Alkylgruppe, eine Alkoxygruppe, eine Aminoalkylgruppe, eine Aminoalkoxygruppe, eine Thioalkylgruppe oder eine Gruppe ―Y―Z;
R und R', die gleich oder verschieden sein können, jeweils ein Wasserstoffatom oder eine Gruppe ―Y―Z;
Y einen gerad- oder verzweigtkettigen Alkylenrest « alk » oder einen Rest 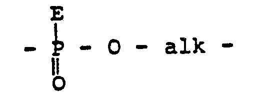 oder auch einen Rest ―Y"―O―Y'― mit Y" oder Y' in der für Y angegebenen Bedeutung ;
E einen Rest entsprechend der Bedeutung von X;
J ein Wasserstoffatom oder eine Hydroxygruppe ;
Z einen Rest entsprechend einer Einlagerungsverbindung, wobei mindestens eine der Gruppen X, R, R' oder E für eine Gruppe -Y-Z steht ;
n eine ganze Zahl einschließlich 0 ; sowie die Salze und Isomerengemische oder die aufgetrennten Isomeren.

2. Verbindung nach Anspruch 1, dadurch gekennzeichnet, daß die Einlagerungsverbindung Z aus polycyclischen Verbindungen mit ebener Konfiguration ausgewählt ist.

3. Verbindung nach Anspruch 2, dadurch gekennzeichnet, daß Z aus Acridin, Furocumarin oder Ellipticin ausgewählt ist.

4. Verbindung nach einem der Ansprüche 1 bis 3, dadurch gekennzeichnet, daß der Rest B aus einem Thymin-, Adenin-, Cytosin-, Guanin-, Uracil-, 5-Bromuracil-, 8-Azidoadenin- oder 2,6-Diaminopurinrest oder einem photoaktivierbaren Basederivat besteht.

5. Verbindung nach einem der Ansprüche 1 bis 4, dadurch gekennzeichnet, daß der Rest Z aus 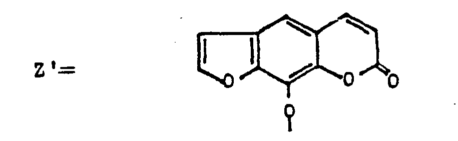 oder 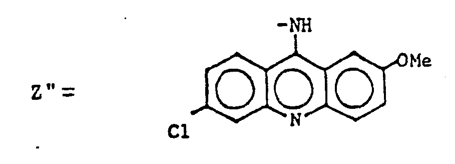 ausgewählt ist.

6. Verbindung nach einem der Ansprüche 1 bis 5, dadurch gekennzeichnet, daß B aus T, G, A, C und U und X aus O^{e}, Methylen, Ethylen, Propylen, Methoxy, Ethoxy und Propoxy ausgewählt sind und J = H.

7. Verbindung nach Anspruch 1 der Formel : 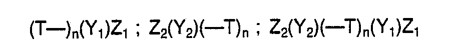 worin bedeuten :
T den Thymidinrest ;
n eine ganze Zahl von 1 bis 25 und
Y₁ und Y₂ einen C₁- bis C₇-Alkylenrest oder einen Rest 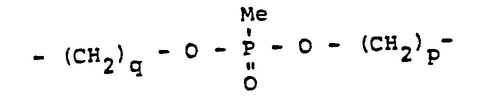 mit p und q gleich ganzen Zahlen von 1 bis 10 und Z₁ und Z₂ in den Bedeutungen entsprechend Z.

8. Verbindungen nach Anspruch 1 der Formel :

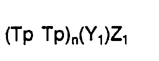

9. Verbindung nach Anspruch 1 der Formel : 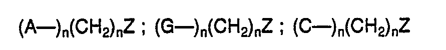 und die Sequenzverbindungen, nämlich die Oligonukleotide :
d [A―T―A―A―A―T―T―C―A―C―(CH₂)ₙZ]
d [A―A―T―G―G―T―A―A―A―A―T―(CH₂)ₙZ]
d [T―T―C―T―C―C―C―C―C―A―C―C―A―(CH₂)ₙZ]
d [T―T―T―A―A―(CH₂)ₙZ]
d [T―T―T―A―A―T―T―T―A―A―(CH₂)ₙZ]
d [T―T―T―A―A―T―T―T―A―A―T―T―T―A―A―(CH₂)ₙZ]
d [T―T―T―T―T―T―T―A―(CH₂)ₙZ]

10. Verbindungen nach Anspruch 5 oder Formel :
Z'(CH₂)₃∓T∓T∓T∓T∓(CH₂)₃Z'
Z"(CH₂)₅∓T∓T∓T∓T∓(CH₂)₅Z"
Z'(CH₂)₃―T―T―T―T―(CH₂)₃Z' Z''(CH₂)₅―T―T―T―T―(CH₂)₅Z''
Z"(CH₂)₅―(T―)₈(CH₂)₅Z"
T―(CH₂)₃Z'
T―(CH₂)₃Z"
T―(CH₂)₅Z"
T―T―T―(CH₂)₅Z"
T―T―T―T―(CH₂)₃Z'
T―T―T―T―(CH₂)₅Z" 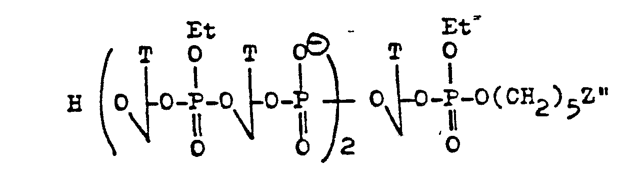 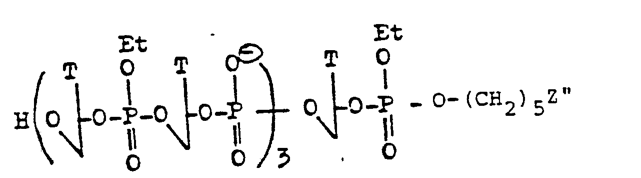 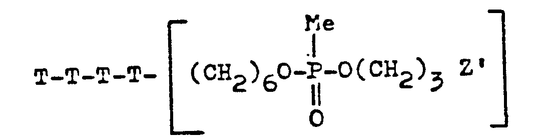 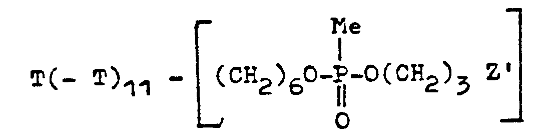
(T―)₄(CH₂)₄Z"
(T―)₈(CH₂)₅Z"
(T―)₈(CH₂)₆Z"
(T―)₁₂(CH₂)₅Z" 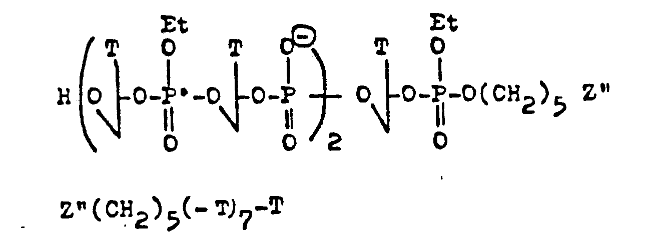 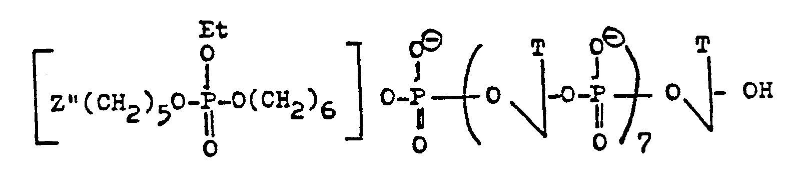 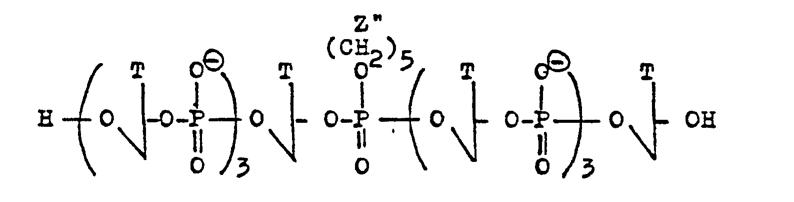 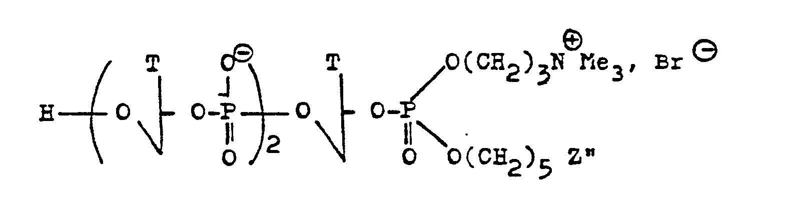 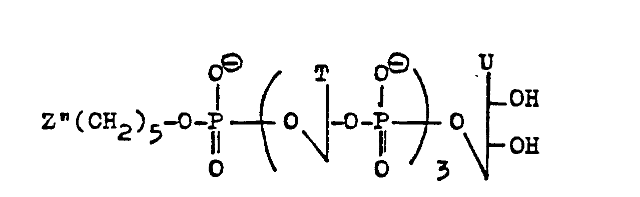 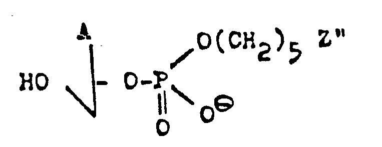 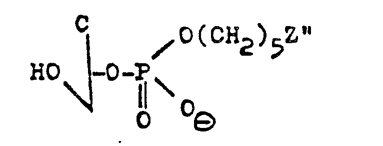 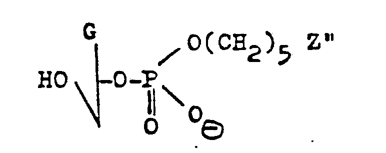
d [A―A―A―A―A―(CH₂)₅Z"]
d [A―T―A―A―A―T―T―C―A―C―(CH₂)₅Z"]
d [A-A-T--G--G-T-A-A-A-A-T-(CH₂)₅Z"]
in denen das Symbol (+) die Phosphotriesterbindung darstellt, wobei jedes Phosphat durch die p-Chlorphenylgruppe geschützt ist.

11. Verfahren zur Herstellung von Verbindungen nach einem der Ansprüche 1 bis 10, dadurch gekennzeichnet, daß man die vollständig geschützten Derivate herstellt und die Schutzgruppen entfernt.

12. Verfahren nach Anspruch 11, dadurch gekennzeichnet, daß man einen geschützten Oligonukleotid-3'-phosphodiester mit der Hydroxylgruppe der Einlagerungsverbindung oder mit der 5'-Hydroxylgruppe eines bereits die Einlagerungsverbindung enthaltenden geschützten Oligonukleotids kuppelt.

13. Verfahren nach Anspruch 11, dadurch gekennzeichnet, daß man einen durch die Hydroxylgruppe der Einlagerungsverbindung geschützten Oligonukleotid-5'-phosphodiester oder einen die Gruppe der Einlagerungsverbindung tragenden Diester mit der 5'-Hydroxylgruppe eines geschützten Oligonukleotids kuppelt.

14. Verfahren nach Anspruch 11, dadurch gekennzeichnet, daß man die Hydroxylgruppe der Einlagerungsverbindung mit einem geschützten Oligonukleotid-3',5'-bis-(phosphodiester) kuppelt.

15. Verfahren nach Anspruch 11, dadurch gekennzeichnet, daß man einen die Einlagerungsverbindung tragenden Diester mit der 5'-Hydroxylgruppe eines bereits die Einlagerungsverbindung enthaltenden geschützten Oligonukleotids kuppelt.

16. Verfahren nach Anspruch 11, dadurch gekennzeichnet, daß man die Entschützung der arylierten Phosphoestergruppen in wasserfreiem Milieu mittels Benzhydroxamsäure in Gegenwart einer nichtnukleophilen tertiären Base durchführt.

17. Verwendung der Verbindungen nach einem der Ansprüche 1 bis 10 als Sonde zum Nachweis einer bestimmten DNA- oder RNA-Sequenz.

18. Verwendung von Verbindungen nach einem der Ansprüche 1 bis 10 zur Reinigung der bestimmten DNA- oder RNA-Sequenz.

19. Verwendung von Verbindungen nach einem der Ansprüche 1 bis 10 zur Diagnose von DNA- und RNA-Mutationen.

## Patentansprüche (Patentansprüche für folgende(n) Vertragsstaat(en) : AT)

1. Verfahren zur Herstellung einer Verbindung der Formel : 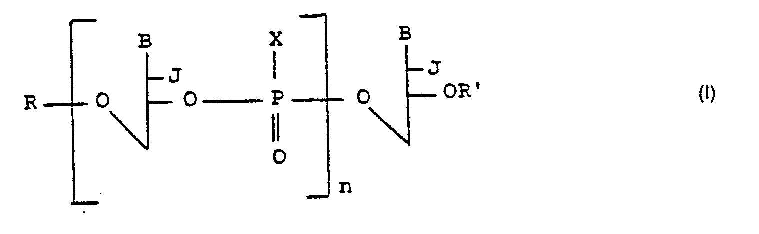 worin bedeuten :
die Reste B, die gleich oder verschieden sein können, jeweils eine Base einer natürlichen oder modifizierten Nukleinsäure ;
die Reste X, die gleich oder verschieden sein können, jeweils ein Sauerstoffanion 0⁻, ein Schwefelanion S^{e}, eine Alkylgruppe, eine Alkoxygruppe, eine Aminoalkylgruppe, eine Aminoalkoxygruppe, eine Thioalkylgruppe oder eine Gruppe ―Y―Z;
R und R', die gleich oder verschieden sein können, jeweils ein Wasserstoffatom oder eine Gruppe -Y-Z ;
Y einen gerad- oder verzweigtkettigen Alkylenrest « alk oder einen Rest 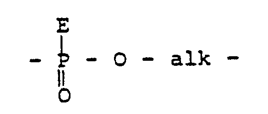 oder auch einen Rest -Y"-0-Y' mit Y" oder Y' in der für Y angegebenen Bedeutung ;
E einen Rest entsprechend der Bedeutung von X ;
J ein Wasserstoffatom oder eine Hydroxygruppe ;
Z einen Rest entsprechend einer Einlagerungsverbindung, wobei mindestens eine der Gruppen X, R, R' oder E für eine Gruppe -Y-Z steht ;
n eine ganze Zahl einschließlich 0 ;
sowie die Salze und Isomerengemische oder die aufgetrennten Isomeren, dadurch gekennzeichnet, daß man die vollständig geschützten Derivate herstellt und die Schutzgruppen eliminiert.

2. Verfahren nach Anspruch 1, dadurch gekennzeichnet, daß man einen geschützten Oligonukleotid-3'-phosphodiester mit der Hydroxylgruppe der Einlagerungsverbindung oder mit der 5'-Hydroxylgruppe eines bereits die Einlagerungsverbindung enthaltenden geschützten Oligonukleotids kuppelt.

3. Verfahren nach Anspruch 1, dadurch gekennzeichnet, daß man einen durch die Hydroxylgruppe der Einlagerungsverbindung geschützten Oligonukleotid-5'-phosphodiester oder einen die Gruppe der Einlagerungsverbindung tragenden Diester mit der 5'-Hydroxylgruppe eines geschützten Oligonukleotids kuppelt.

4. Verfahren nach Anspruch 1, dadurch gekennzeichnet, daß man die Hydroxylgruppe der Einlagerungsverbindung mit einem geschützten Oligonukleotid-3',5'-bis-(phosphodiester) kuppelt.

5. Verfahren nach Anspruch 1, dadurch gekennzeichnet, daß man einen die Einlagerungsverbindung tragenden Diester mit der 5'-Hydroxylgruppe eines bereits die Einlagerungsverbindung enthaltenden geschützten Oligonukleotids kuppelt.

6. Verfahren nach Anspruch 1, dadurch gekennzeichnet, daß man die Entschützung der arylierten Phosphoestergruppen in wasserfreiem Milieu mittels Benzhydroxamsäure in Gegenwart einer nichtnukleophilen tertiären Base durchführt.

7. Verfahren nach einem der Ansprüche 1 bis 6, dadurch gekennzeichnet, daß die Einlagerungsverbindung Z aus polycyclischen Verbindungen mit ebener Konfiguration ausgewählt ist.

8. Verfahren nach Anspruch 7, dadurch gekennzeichnet, daß Z aus Acridin, Furocumarin oder Ellipticin ausgewählt ist.

9. Verfahren nach einem der Ansprüche 1 bis 8, dadurch gekennzeichnet, daß der Rest B aus einem Thymin-, Adenin-, Cytosin-, Guanin-, Uracil-, 5-Bromuracil-, 8-Azidoadenin- oder 2,6-Diaminopurinrest oder einem photoaktivierbaren Basederivat besteht.

10. Verfahren nach einem der Ansprüche 1 bis 9, dadurch gekennzeichnet, daß der Rest Z aus 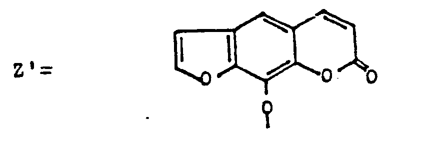 oder 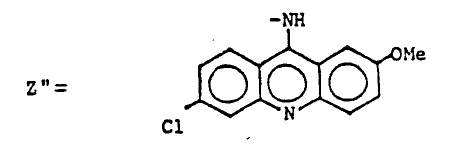 ausgewählt ist.

11. Verfahren nach einem der Ansprüche 1 bis 10, dadurch gekennzeichnet, daß B aus T. G, A, C und U und X aus 0², Methylen, Ethylen, Propylen. Methoxy, Ethoxy und Propoxy ausgewählt sind und J = H.

12. Verfahren zur Herstellung einer Verbindung nach einem der Ansprüche 1 bis 11 der Formel : 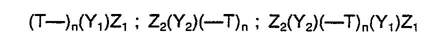 worin bedeuten :
T den Thymidinrest ;
n eine ganze Zahl von 1 bis 25 und
Y₁ und Y₂ einen C₁- bis C₇-Alkylenrest oder einen Rest 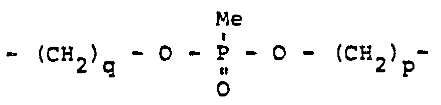 mit p und q gleich ganzen Zahlen von 1 bis 10 und Z₁ und Z₂ in den Bedeutungen entsprechend Z.

13. Verfahren zur Herstellung einer Verbindung nach einem der Ansprüche 1 bis 12 der Formel :

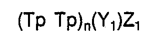

14. Verfahren zur Herstellung von Verbindungen nach einem der Ansprüche 1 bis 13 der Formel : 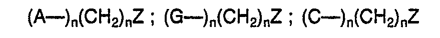 und die Sequenzverbindungen, nämlich die Oligonukleotide :
d [A―T―A―A―A―T―T―C―A―C―(CH₂)ₙZ]
d [A―A―T―G―G―T―A―A―A―A―T―(CH₂)ₙZ]
d [T―T―C―T―C―C―C―C―C―A―C―C―A―(CH₂)ₙZ]
d [T―T―T―A―A―(CH₂)ₙZ]
d [T―T―T―A―A―T―T―T―A―A―(CH₂)ₙZ]
d [―T―T―A―A―T―T―T―A―A―T―T―T―A―A―(CH₂)ₙZ]
d [T-T-T-T-T-T-T-A-(CH₂)ₙZ]

15. Verfahren zur Herstellung von Verbindungen nach einem der Ansprüche 1 bis 14 der Formeln
Z'(CH₂)₃∓T∓T∓T∓T∓(CH₂)₃Z'
Z"(CH₂)₅∓T∓T∓T∓T∓(CH₂)₅Z"
Z'(CH₂)₃―T―T―T―T―(CH₂)₃Z'
Z"(CH₂)₅―T―T―T―T―(CH₂)₅Z'
Z"(CH₂)₅―(T―)₈(CH₂)₅Z"
T―(CH₂)₃Z'
T―(CH₂)₃Z"
T―(CH₂)₅Z"
T―T―T―(CH₂)₅Z"
T―T―T―T―(CH₂)₃Z'
T―T―T―T―(CH₂)₅Z" 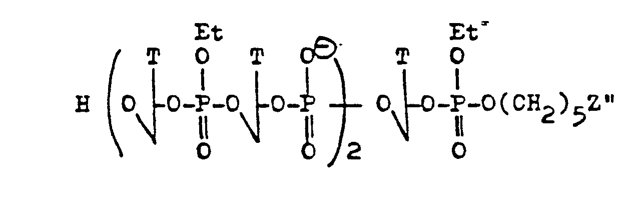 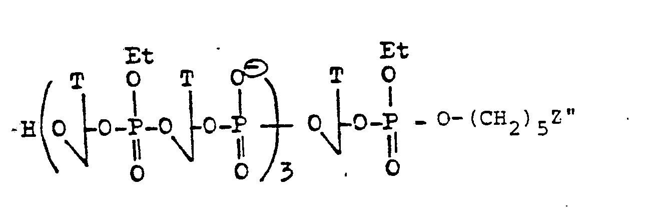 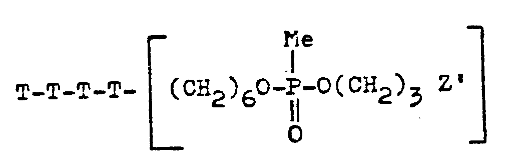 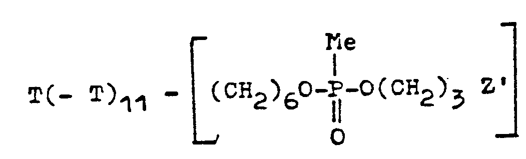
(T―)₄(CH₂)₄Z"
(T―)₈(CH₂)₅Z"
(T―)₈(CH₂)₆Z"
(T―)₁₂(CH₂)₅Z" 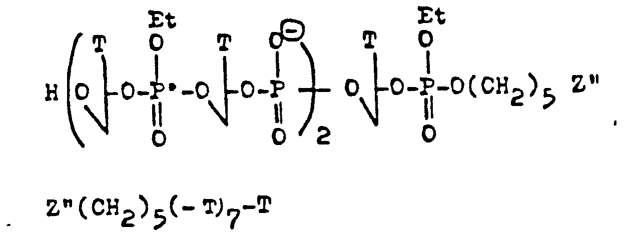 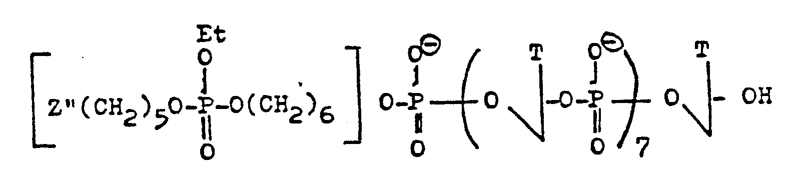 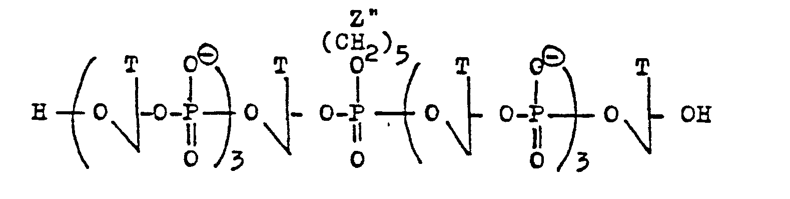 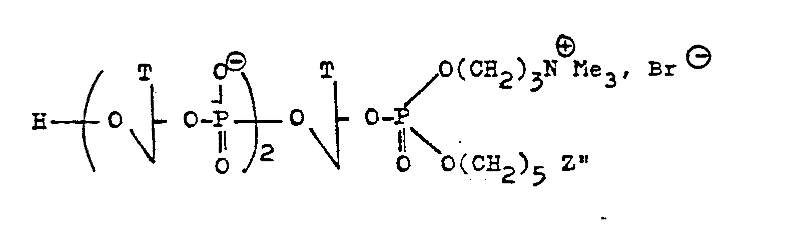 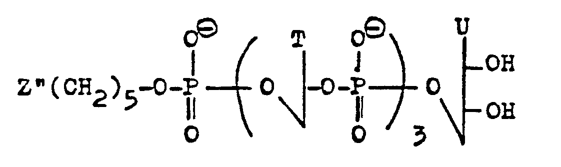 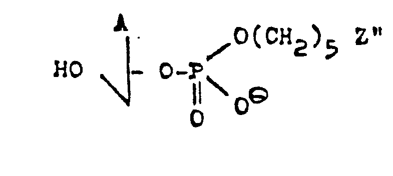 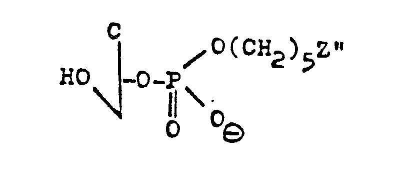 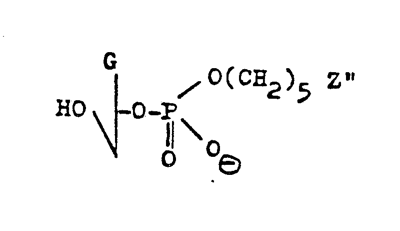
d [A―A―A―A―A―(CH₂)₅Z"]
d [A―T―A―A―A―T―T―C―A―C―(CH₂)₅Z"]
d [A―A―T―G―G―T―A―A―A―A―T―(CH₂)₅Z"]
in denen das Symbol (∓) die Phosphotriesterbindung darstellt, wobei jedes Phosphat durch die p-Chlorphenylgruppe geschützt ist.
